# EUROPEAN PATENT APPLICATION

(11) **EP 2 017 289 A1**
(43) Date of publication of application: **21.01.2009**
(21) Application number: 07742002.4
(22) Date of filing: 20.04.2007
(51) Int. Cl.: C08B 11/08, A61K 31/717, A61P 25/28, C08B 11/02

(54) **PHARMACEUTICAL COMPOSITION FOR CONFORMATIONAL DISEASE**

(30) Priority: 20.04.2006 JP 2006117294
(71) Applicant: Itoham Foods Inc., Kobe-shi, Hyogo 657-0037 (JP); National University Corporation Tohoku Unversity, Sendai-shi, Miyagi 9808577 (JP)
(72) Inventor: DOHURA, Katsumi, Sendai-shi, Miyagi 980-5577 (JP)
(74) Representative: Golding, Louise Ann
(86) International application number: PCT/JP2007/058566
(87) International publication number: WO 2007/123187

(57) **Abstract**

A pharmaceutical composition is provided which is prophylactically and/or therapeutically effective for a conformational disease typified by Alzheimer's disease, prion disease, and amyloidosis. The pharmaceutical composition of the present invention includes as an active ingredient one or more cellulose ethers selected from a cellulose ether having a structural unit represented by the general formula (1): 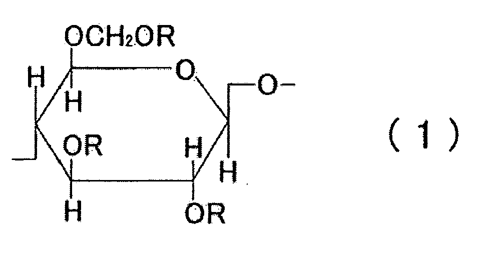 in which at least one R is selected from CH₃, C₂H₅, CH₂CH₂OH, CH₂CH(OH)CH₃, and CH₂COOM; while other R is H; and M is selected from H, Na, K and Ca,
an alkali metal salt of the cellulose ether, an aliphatic acid ester of the cellulose ether, and a mixture thereof.

## Description

### Technical Field to which the Invention Belongs

The present invention relates to a pharmaceutical composition for a conformational disease, and particularly relates to a pharmaceutical composition effective in prophylaxis and therapy of conformational diseases.

### Background Art

Conformational diseases are a group of diseases in which a certain host protein has its secondary structure (conformation) changed, thereby turning into an insoluble form, which is accumulated at cells and tissues, and thus a diseased state is caused. A variety of cases of the diseases have been reported (see, Nonpatent Document 1, Nonpatent Document 2, and Nonpatent Document 3). Examples of the conformational disease include intractable neurological diseases typified by Alzheimer's disease, prion disease, polyglutamine disease, Parkinson's disease, and amyotrophic lateral sclerosis.

In the conformational disease, a certain protein is changed to take a conformation, which is rich in structure components, referred to as a ß sheet structure that is different from the native conformation, thereby turning into an indegradable form. Among the conformational diseases, a group of diseases in which a protein having such an abnormal conformation accumulates at tissues extracellularly to form insoluble matters (referred to as "amyloid" ) is referred to as amyloidosis. In the meanwhile, there also exists a group of diseases in which such a protein having an abnormal conformation accumulates intracellularly to form insoluble matters (referred to as "inclusion body" in some cases) . The former includes a variety of disease of amyloidosis, whilst the latter includes polyglutamine disease, Parkinson's disease, amyotrophic lateral sclerosis, diffuse Lewy body disease, Huntington's disease, Pick's disease, progressive supranuclear palsy, frontotemporal dementia and the like. Also, both extracellular and intracellular accumulation of the protein having an abnormal conformation are found in Alzheimer's disease, prion disease and the like. The responsible proteins for these conformational diseases are varied and depend on the disease, with no common feature of the protein primary structure, but involvement of a common factor has been suggested with respect to the mechanism for abnormalizing the conformation as well as the mechanism for promoting deposition of the abnormalized protein. Accordingly, development of a therapeutic compound which can be commonly employed for any conformational diseases or for a plurality of conformational diseases would be possible (see, Nonpatent Document 4, Nonpatent Document 5). Actually, developments of medicaments expected to exhibit common medicinal virtues for Alzheimer's disease, any amyloidosis, and prion disease have been abundantly reported hitherto (for example, see, Nonpatent Document 6, Nonpatent Document 7, Nonpatent Document 8, Nonpatent Document 9).

Although pathogenesis and action mechanisms have been gradually revealed for these conformational diseases, any absolutely effective medical drug for the prophylaxis and therapy has not been found so far.
Heretofore, prophylactically and therapeutically effective medical drugs such as, for example: an artificially designed peptide having six contiguous amino acid residues derived from an amino acid sequence that constitutes a laminin binding site (see, Patent Document 1), a compound having a nitrogen-containing heterocyclic group such as a quinoline ring, a quinuclidine ring or a pyridine ring, or a nitrogen-containing side chain such as aromatic amine or hydrazone attached to a nitrogen-containing heterocycle such as a quinoline ring or a naphthyridine ring (see, Patent Document 2) , and the like targeting for prion disease; for example, a specific plant such as a plant of Picrorhiza kurrooa, a plant of genus Apocynum, Catharanthus roseus, a plant of genus Iris, and the extract of the same (see, Patent Document 3), polyphenols such as myricetin, morin, catechin, and epicatechin (see, Patent Document 4) and the like targeting for Alzheimer's disease; as well as saccharides including an anion substituent such as glucose pentasulfate (see, Patent Document 5) targeting for amyloidosis including Alzheimer's disease; geranylgeraniol that induces a heat shock protein (see, Patent Document 6) targeting for diseases including amyloidosis; for example, a protein having a specified amino acid sequence (see, Patent Document 7), neuropeptides having a neuroprotective action (see, Patent Document 8), bioactive peptides having a blood flow acceleration and/or blood pressure lowering action, such as a pituitary adenylate cyclase activating peptide or a vasoactive intestinal peptide (see, Patent Document 9) targeting for a conformational disease, and the like were proposed.

Patent Document 1: Japanese Patent Unexamined Publication No. 2005-192415
Patent Document 2: Japanese Patent Unexamined Publication No. 2004-099553
Patent Document 3: Japanese Patent Unexamined Publication No. 2005-350391
Patent Document 4: Japanese Patent Unexamined Publication No. 2005-104850
Patent Document 5: Japanese Patent Unexamined Publication No. 2001-513569 (a published Japanese translation of a PCT application)
Patent Document 6: Japanese Patent Unexamined Publication No. 2001-172171
Patent Document 7: Japanese Patent Unexamined Publication No. 2001-275687
Patent Document 8: pamphlet of International Publication WO 2003/051387
Patent Document 9: pamphlet of International Publication WO 2004/048401
Nonpatent Document 1: Robin W Carrell, David A Lomas, The Lancet, 1997, No. 350, pp. 134-138
Nonpatent Document 2: Tokuhiro Ishihara (Editorial supervisor), Syuichi Ikeda (Editor), "Basic Study and Clinical Application of Amyloidosis" March 2005, KANEHARA & Co., LTD. (Publisher), pp. 8-13
Nonpatent Document 3: Takeshi Iwatsubo, Cell Technology, 2001, Vol. 20, No. 11, pp. 1474-1477
Nonpatent Document 4: Jarrett JT, Lansbury PT Jr. , Cell, 1993, Vol. 73, pp. 1055-1058
Nonpatent Document 5: Gervais F, Morissette C, Kong X, Curr. Med. Chem. Immun. , Endoc. & Metab. Agents, 2003, Vol. 3, pp. 361-370
Nonpatent Document 6: Watson JD, Lander DA, Selkoe JD, The Journal of Biological Chemistry, 1997, Vol. 50, pp. 31617-31624
Nonpatent Document 7: Kisilevsky R, Nature Medicine, 1995, Vol. 1, pp. 143-148
Nonpatent Document 8: Zhu H, Yu J, Kindy MS, Molecular Medicine, 2001, Vo. 7, pp. 517-522
Nonpatent Document 9: Katsumi Doura, Pharmacia, 2002, Vol. 7, pp. 635-639

### Disclosure of the Invention

In view of the foregoing circumstances, an object of the present invention is to provide a medical drug that is prophylactically and/or therapeutically effective for a conformational disease typified by Alzheimer's disease, prion disease, and amyloidosis.

In order to accomplish the object described above, the pharmaceutical composition for a conformational disease of the present invention is characterized by including as an active ingredient one or more cellulose ethers selected from a cellulose ether having a structural unit represented by the general formula (1):

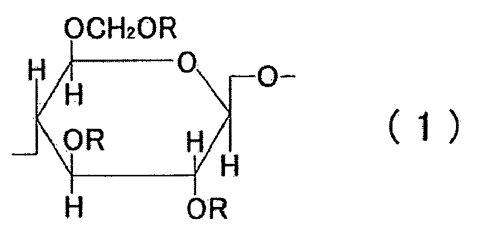

wherein, at least one R is selected from CH₃, C₂H₅. CH₂CH₂OH, CH₂CH(OH)CH₃, and CH₂COOM; while other R is H; and M is selected from H, Na, K and Ca,
an alkali metal salt of the cellulose ether, an aliphatic acid ester of the cellulose ether, and a mixture thereof.

In such a pharmaceutical composition for a conformational disease, the cellulose ether is preferably one or more selected from hydroxypropyl methylcellulose (HPMC), hydroxypropyl cellulose and methyl cellulose. The pharmaceutical composition is preferably formulated to be administrable to a mammal including human, and particularly preferably, the pharmaceutical composition is formulated in a dosage form selected from injections, tablets, granules, powdered formulations, capsules, pills, lozenges, liquid formulations, suspensions, emulsions, syrups, spirits, elixirs, lemonades, ointments, plasters, cataplasms, tinctures, lotions, liniments, aerosols, and suppositories. Such a pharmaceutical composition is suitable as a prophylactic drug for a conformational disease, or a therapeutic drug for a conformational disease. Also, the conformational disease is preferably a prion disease of any one of Creutzfeldt-Jakob disease (CJD) in human,
Gerstmann-Straussler-Scheinker syndrome in human, fatal familial insomnia in human, bovine spongiform encephalopathy (BSE) in cattle, scrapie in sheep, and chronic wasting disease in deer, more preferably Alzheimer's disease, and still more preferably amyloidosis.

The cellulose ether according to the present invention has prominent therapeutic and/or prophylactic effects with sustained-release performance in typical conformational diseases such as prion disease, Alzheimer's disease and amyloidosis. In addition, these compounds have been industrially used broadly, and also used routinely as ingredients in additives for foods as well as in medical drugs, with high level of safety.

### Brief Description of the Drawings

Fig. 1 shows a photograph presented as a drawing for illustrating an electrophoretic image on Western blotting that shows results of evaluation *in vitro* on the HPMC effects of suppressing formation of prion that is a responsible protein of prion disease (Example 1).
Fig. 2 shows a graph illustrating results of investigation in *vivo* on relationship between the administration timing of HPMC, and a therapeutic effect for the prion disease (Example 2).
Fig. 3 shows a graph illustrating results of investigation *in vivo* on relationship between the administration frequency of HPMC, and the therapeutic effect for prion disease (Example 3).
Fig. 4 shows a graph illustrating results of investigation *in vivo* on relationship between the amount of administration of HPMC, and the therapeutic effect for prion disease (Example 4).
Fig. 5 shows a graph illustrating one example of results of investigation *in vivo* on the therapeutic and/or prophylactic effect for the prion disease by single administration of HPMC before the infection (Example 5).
Fig. 6 shows a graph illustrating results of investigation *in vivo* on the therapeutic and/or prophylactic effect for prion disease by single administration of a variety of cellulose ethers before the infection (Example 6).
Fig. 7 shows a graph illustrating results of investigation *in vivo* as to whether or not a synergistic effect is exhibited on the therapeutic and/or prophylactic effect for prion disease by multiple-times administrations of HPMC before the infection (Example 7).
Fig. 8 shows a graph illustrating results of comparative investigation *in vivo* on the therapeutic effect for prion disease with respect to a variety of cellulose ethers (Example 8).
Fig. 9 shows a graph illustrating results of investigation on the therapeutic effect for prion disease by peripheral intravenous administration of HPMC (Example 9).
Fig. 10 shows a graph illustrating results of investigation on the dose-dependent therapeutic effect for prion disease by direct intracerebroventricular administration of HPMC (Example 10).
Fig. 11 shows a graph illustrating results of investigation on the therapeutic effect for prion disease by direct intracerebroventricular administration of HPMCs, in comparison with pentosan polysulfate (PPS) (Example 11).
Fig. 12 shows a graph illustrating results of investigation on the therapeutic effect for prion disease by combined administration of HPMC, i.e., in combination of subcutaneous administration and interperitoneal administration (Example 12).
Fig. 13 shows a graph illustrating results of investigation on the therapeutic effect for prion disease by combined administration of HPMC, i.e., in combination of subcutaneous administration and intracerebroventricular administration (Example 13).
Fig. 14 shows a graph illustrating one example of results of investigation on the prophylactic effect for prion disease by HPMC with respect to prion infection from the periphery (Example 14).
Fig. 15 shows a graph illustrating results of investigation on relationship between the administration dose of HPMC to a syrian hamster, and the therapeutic effect for prion disease (Example 15).
Fig. 16 shows a graph illustrating results of investigation on relationship between the administration timing of HPMC to a syrian hamster, and the therapeutic effect for prion disease (Example 15).
Fig. 17 shows a graph illustrating results of investigation *in vivo* on the therapeutic effect for prion disease by cellulose ethers on RML strain (prion strain of scrapie passaged through mice) (Example 16).
Fig. 18 shows a graph illustrating results of investigation on the therapeutic effect for prion disease by cellulose ethers on Fukuoka 1 strain (prion strain of a human prion disease passaged through mice) (Example 17).
Fig. 19 shows a graph illustrating results of investigation on the therapeutic effect for prion disease by cellulose ethers on 22L strain, another prion strain of scrapie passaged through mice (Example 18).
Fig. 20 shows a graph illustrating results of investigation on the therapeutic effect for prion disease with respect to the content of methoxyl groups and hydroxypropoxyl groups in the cellulose ethers, and molecular weight of the cellulose ethers (Example 19).
Fig. 21 shows a graph illustrating results of investigation on the therapeutic effect for prion disease with respect to the molecular weight of the cellulose ethers (Example 20).
Fig. 22 shows a graph illustrating results examined on the therapeutic effect for prion disease by hexasaccharide cellulose ethers having a low molecular weight (Example 21).
Fig. 23 shows a graph illustrating results of investigation on the therapeutic effect for prion disease by HPMC having a molecular weight lowered by way of hydrolysis (Example 22).
Fig. 24 shows a graph illustrating results of investigation on the therapeutic effect for prion disease by other compound that is similar to the cellulose ethers (Example 23).
Fig. 25 shows a cross-sectional view demonstrating around the hippocampus in mouse brain (Example 24).
Fig. 26 shows a low magnification photomicrography illustrating accumulation of abnormal prion protein around the hippocampus (Example 24).
Fig. 27 shows a high magnification photomicrography illustrating white matter region positioned between the hippocampus and the cerebral cortex in Fig. 26 (Example 24).
Fig. 28 shows a photomicrography of immunostaining with GFAP at the same site as that observed in Fig. 26 (Example 24).
Fig. 29 shows a high magnification photomicrography of a field including the pyramidal cell layer of hippocampus in Fig. 28 (Example 24).
Fig. 30 shows a cross-section view demonstrating the inferior colliculus in mouse brain (Example 24).
Fig. 31 shows a low magnification photomicrography illustrating accumulation of abnormal prion protein at the inferior colliculus (Example 24).
Fig. 32 shows a high magnification photomicrography illustrating accumulation of abnormal prion protein at the inferior colliculus (Example 24).
Fig. 33 shows a high magnification photomicrography of immunostaining with GFAP at the same site as that observed in Fig. 31 (Example 24).
Fig. 34 shows a photograph presented as a drawing for illustrating an electrophoretic image on Western blotting that shows one example of results of measurement of anti-prion activity in the blood (serum) of hamsters given subcutaneous administion of HPMC (Example 25).
Fig. 35 shows a photograph presented as a drawing for illustrating an electrophoretic image on Western blotting that shows results of investigation on the sensitivity to the temperature with respect to the anti-prion activity (albumin not removed) in the hamster serum (Example 26).
Fig. 36 shows a photograph presented as a drawing for illustrating an electrophoretic image on Western blotting that shows results of investigation on the sensitivity to the temperature with respect to the anti-prion activity (albumin removed) in the hamster serum (Example 26).
Fig. 37 shows a photograph presented as a drawing for illustrating an electrophoretic image on Western blotting that shows one example of results of estimation of the molecule size of the anti-prion activity in the hamster serum using an ultrafiltration membrane (Example 27).
Fig. 38 shows a photograph presented as a drawing for illustrating an electrophoretic image on Western blotting that shows one example of results of investigation on the sensitivity to treatments with degradative enzymes with respect to the anti-prion activity in the hamster serum (Example 28).
Fig. 39 shows a graph illustrating time dependent alteration of the radioactivity density in each tissue following single administration of labeled HPMC into the mouse tail vein (Example 29).
Fig. 40 shows a graph illustrating time dependent alteration of the radioactivity density in each tissue following single administration of labeled HPMC into the mouse tail vein (Example 29).
Fig. 41 shows a graph illustrating time dependent alteration of the radioactivity density in each tissue following single administration of labeled HPMC into the mouse tail vein (Example 29).
Fig. 42 shows a graph illustrating time dependent alteration of the radioactivity density in each tissue following single administration of labeled HPMC into the mouse tail vein (Example 29).
Fig. 43 shows a graph illustrating time dependent alteration of the radioactivity density in each tissue following single administration of labeled HPMC into the mouse tail vein (Example 29).
Fig. 44 shows a graph illustrating time dependent alteration of the radioactivity density in each tissue following single administration of labeled HPMC into the mouse tail vein (Example 29).
Fig. 45 shows a graph illustrating time dependent alteration of the radioactivity density in each tissue following single subcutaneous administration of labeled HPMC into the mouse dorsal region (Example 30).
Fig. 46 shows a graph illustrating time dependent alteration of the radioactivity density in each tissue following single subcutaneous administration of labeled HPMC into the mouse dorsal region (Example 30).
Fig. 47 shows a graph illustrating time dependent alteration of the radioactivity density in each tissue following single subcutaneous administration of labeled HPMC into the mouse dorsal region (Example 30).
Fig. 48 shows a graph illustrating time dependent alteration of the radioactivity density in each tissue following single subcutaneous administration of labeled HPMC into the mouse dorsal region (Example 30).
Fig. 49 shows a graph illustrating time dependent alteration of the radioactivity density in each tissue following single subcutaneous administration of labeled HPMC into the mouse dorsal region (Example 30).
Fig. 50 shows a graph illustrating time dependent alteration of the radioactivity density in each tissue following single subcutaneous administration of labeled HPMC into the mouse dorsal region (Example 30).
Fig. 51 shows a right axis aspect of a freeze-dried section following single administration of labeled HPMC into the mouse tail vein (upper panel), and a radioluminogram illustrating the radioactivity density in each tissue (lower panel) (Example 31).
Fig. 52 shows a central axis aspect of a freeze-dried section following single administration of labeled HPMC into the mouse tail vein (upper panel), and a radioluminogram illustrating the radioactivity density in each tissue (lower panel) (Example 31).
Fig. 53 shows a left axis aspect of a freeze-dried section following single administration of labeled HPMC into the mouse tail vein (upper panel), and a radioluminogram illustrating the radioactivity density in each tissue (lower panel) (Example 31).
Fig. 54 shows a graph illustrating results of investigation on an AB protein aggregation-inhibitory activity by a cellulose ether (HPCL) (Example 32).
Fig. 55 shows a graph illustrating results of investigation on the AB protein aggregation-inhibitory activity by a cellulose ether (MC) (Example 32).
Fig. 56 shows a graph illustrating results of investigation on the AB protein aggregation-inhibitory activity by a cellulose ether (HPMC) (Example 32).
Fig. 57 shows a graph illustrating one example of results of investigation on the AB protein aggregation-inhibitory activity by a cellulose ether (HPMC hydrolyzed with 0.3% hydrochloric acid) (Example 32).
Fig. 58 shows a graph illustrating results of investigation on the AB protein aggregation-inhibitory activity by a cellulose ether (HPMC hydrolyzed with 40% hydrochloric acid) (Example 32).
Fig. 59 shows a graph illustrating results of investigation on protective effect on the nerve cells against aggregated AB by a cellulose ether (HPCL) (Example 33).
Fig. 60 shows a graph illustrating results of investigation on protective effect on the nerve cells against aggregated AB by a cellulose ether (MC (SM-4)) (Example 33).
Fig. 61 shows a graph illustrating results of investigation on protective effect on the nerve cells against aggregated AB by a cellulose ether (HPMC (TC-5RW)) (Example 33).
Fig. 62 shows a graph illustrating results of investigation on protective effect on the nerve cells against aggregated AB by a cellulose ether (HPMC hydrolyzed with 0.3% hydrochloric acid) (Example 33).
Fig. 63 shows a graph illustrating results of investigation on protective effect on the nerve cells against aggregated AB by a cellulose ether (HPMC hydrolyzed with 40% hydrochloric acid) (Example 33).
Fig. 64 shows results of burrowing test for investigating the therapeutic effect in Alzheimer's disease model mice by cellulose ethers (Example 34).
Fig. 65 shows results of glucose liquid consumption test for investigating the therapeutic effect in Alzheimer's disease model mice by the cellulose ethers (Example 34).
Fig. 66 shows a photograph presented as a drawing for illustrating a HE-stained specimen of liver, revealing results of investigation on the effect of suppressing onset of amyloidosis by HPMC, using an animal disease model in which AA amyloid is accumulated at various peripheral organs (Example 35).
Fig. 67 shows a photograph presented as a drawing for illustrating a Congo red-stained specimen of liver, revealing results of investigation on the effect of suppressing onset of amyloidosis by HPMC, using the same animal disease model as in Fig. 66 (Example 35).
Fig. 68 shows a photograph presented as a drawing for illustrating an immunostained specimen of liver with an anti-SAA antibody, revealing results of investigation on the effect of suppressing onset of amyloidosis by HPMC, using the same animal disease model as in Fig. 66 (Example 35).
Fig. 69 shows a photograph presented as a drawing for illustrating a HE-stained specimen of spleen, revealing results of investigation on the effect of suppressing onset of amyloidosis by HPMC, using the same animal disease model as in Fig. 66 (Example 35).
Fig. 70 shows a photograph presented as a drawing for illustrating a Congo red-stained specimen of spleen, revealing results of investigation on the effect of suppressing onset of amyloidosis by HPMC, using the same animal disease model as in Fig. 66 (Example 35).
Fig. 71 shows a photograph presented as a drawing for illustrating an immunostained specimen of spleen with an anti-SAA antibody, revealing results of investigation on the effect of suppressing onset of amyloidosis by HPMC, using the same animal disease model as in Fig. 66 (Example 35).

### Best Mode for Carrying Out the Invention

In the present invention, the conformational diseases comprehensively include any of the group of amyloidosis diseases typified by prion disease and Alzheimer's disease as well as AA amyloidosis caused by change of the conformation (secondary structure) of a certain host protein, followed by accumulation at cells and tissues. More particularly, the prion disease is a group of diseases believed to result from a prion protein, and Creutzfeldt-Jakob disease (CJD), bovine spongiform encephalopathy (BSE), Gerstmann-Straussler-Scheinker Syndrome (GSS), fatal familial insomnia, Kuru, sheep scrapie, transmissible mink encephalopathy (TME), deer chronic wasting disease (CWD) and the like are included. Mammals which are susceptible to suffer from such prion diseases other than human include, for example, cattle, goat, sheep, deer, elk, mink, pig, monkey, mouse, rat, hamster, cat, and the like.

The amyloidosis is a disease believed to cause dysfunction by extracellular deposition of amyloid fibril at various organs, and disease types such as systemic amyloidosis (immunoglobulinic amyloidosis, reactive AA amyloidosis, familial amyloidosis, dialytic (AB2M) amyloidosis, senile amyloidosis and the like), localized amyloidosis (cerebral amyloidosis, endocrine amyloidosis, cutaneous amyloidosis, corneal amyloidosis, localized nodular amyloidosis and the like) are included. In addition, there exists a group of diseases believed to result from intracellular abnormal structures (inclusion body) composed of aggregated fibrillary protein similar to amyloid, and Alzheimer's disease, Parkinson's disease, diffuse Lewy body disease, Huntington's disease, frontotemporal dementia, amyotrophic lateral sclerosis, and the like are included (Nonpatent Documents 2 and 3).

The therapeutic drug for a conformational disease of the present invention includes as an active ingredient a cellulose ether having a structural unit represented by the formula (1), an alkali metal salt of the cellulose ether, an aliphatic acid ester of the cellulose ether, and a mixture of the same. In the formula 1, at least one R is selected from CH₃, C₂H₅, CH₂CH₂OH, CH₂CH(OH)CH₃, and CH₂COOM, while other R is H. Moreover, M in CH₂COOM is selected from H, Na, K, and Ca.

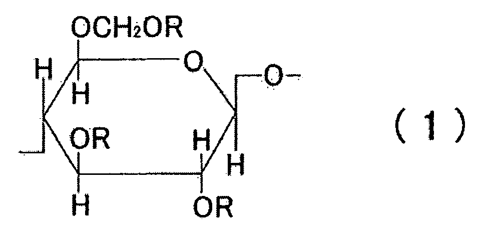

Specific examples of the cellulose ether having a structural unit represented by the formula (1) include e.g., methyl cellulose, ethyl cellulose, methylethyl cellulose, hydroxypropyl cellulose, hydroxyethyl cellulose, hydroxypropyl methylcellulose, hydroxyethyl methylcellulose, carboxymethyl cellulose, and carboxymethyl ethylcellulose. Among these, hydroxypropyl methylcellulose, methyl cellulose, and hydroxypropyl cellulose are preferably selected since they exhibit particularly prominent effects.

The alkali metal salt of the cellulose ether is formed at the hydroxyl group (OH group) in the cellulose ether to give its alkali metal salt, and specific examples include a sodium salt, a potassium salt, and a lithium salt. The alkali metal may be one kind of the salt alone, or a mixture of two or more thereof.

The aliphatic acid ester of the cellulose ether is formed by esterification of the hydroxyl group (OH group) in the cellulose ether with an aliphatic acid. The aliphatic acid is preferably a fatty acid having no more than 18 carbon atoms, and specific examples include acetic acid, propionic acid, succinic acid, oleic acid, stearic acid, citric acid, lactic acid, and malic acid. One kind of the fatty acid may be used alone, or two or more thereof may be also used as a mixture.

Furthermore, the cellulose ether may be a degradation product by a mineral acid, an alkali metal hydroxide, or an enzyme. The degradation product herein may be of any form such as: the intact form subjected to the degradative treatment in water; the degradative treatment liquid further subjected to pH adjustment; the liquid obtained by a fractionation treatment of the degradative treatment liquid; the solid content isolated from the degradative treatment liquid or the fractionation treatment liquid, or the like. Herein, the mineral acid is typically hydrochloric acid, or sulfuric acid; the alkali metal hydroxide is typically sodium hydroxide, or potassium hydroxide; and the enzyme is typically endo or exo cellulase.

For preparing the pharmaceutical composition of the present invention, the cellulose ether is included as an active ingredient. One kind of the cellulose ether may be used alone, or two or more thereof may be used as a mixture.

The pharmaceutical composition includes the cellulose ether represented by the formula (1) as an active ingredient, but any component other than the active ingredient may be blended, or coadministered in the range not to impair the effect of the present invention. Moreover, the pharmaceutical composition of the present invention can be administered via any pharmacologically acceptable administration route after formulating in any pharmaceutically acceptable dosage form. The dosage form to be formulated may be, for example, injection, tablet, granule, powdered formulation, capsule, pill, lozenge, liquid formulation, suspension, emulsion, syrups, spirit, elixir, lemonade, ointment, plaster, cataplasm, tincture, lotion, liniment, aerosol, suppository, or the like. Depending on the type of the cellulose ether used as the active ingredient, the absorption ratio via gastrointestinal tract may be inferior. In such a case, it is preferred to administer via an administration route other than oral route after formulating in a parenteral formulation other than an oral formulation, such as tablet, powdered formulation, granule, liquid formulation for internal use, syrup, lemonade, elixir, suspension, emulsion, capsule filled with any of the same, and the like. Also, the pharmaceutical composition of the present invention often exhibits the absorption ratio not so high via cutaneous, oral, pharyngeal, nasal and pulmonary mucosa as well as gastrointestinal tract. In such a case, it is preferred to administer via an administration route other than transdermal and transmucosal routes, such as those with dosage forms for external use by embrocation, e.g., liquid formulations, tinctures, lotions, ointments and plasters, as well as liniments, cataplasms, aerosols, suppositories, and the like. As described above, when the cellulose ether included as an active ingredient is hardly absorbed via gastrointestinal tract, skin or mucosa, the pharmaceutical composition of the present invention for a conformational disease may be formulated into an injection having the form of a solution, suspension or emulsion in an aqueous solvent or nonaqueous solvent, or formulated in an injection to be dissolved before use, and it may be preferably administered by intravenous, subcutaneous, intramuscular, interperitoneal, intrathecal, intraspinal, intracerebroventricular or intracutaneous injection, or preferably administered by intravenous drip infusion in the form of a transfusion or together with a transfusion. Alternatively, it can be also administered continuously by injection using an osmotic pump or the like, or subcutaneously administered after formulating into an implantable sustained release form. To the pharmaceutical composition of the present invention for a conformational disease may be added in the range not to deteriorate the effect of the present invention, depending on the dosage form to be formulated, additives such as a stabilizing agent, a buffer, a preservative, an excipient and the like. For example, in the case of injections, as a stabilizing agent, a pH-adjusting buffer such as a citric acid salt, acetic acid salt or the like, and an anti-oxidizing agent such as sodium pyrosulfite, acetone or ascorbic acid may be added through selecting ad libitum among these. In the case of subcutaneous or intramuscular administration, as a soothing agent, benzyl alcohol, phenol, procaine hydrochloride, glucose or the like may be added through selecting ad libitum among these. When the formulation is to be dissolved before use, a solubilization agent and/or an excipient such as ethanol or propylene glycol may be also added.

The amount of the cellulose ether administered into a body is not limited uniformly but may vary depending on the type of the conformational disease, animal species to be administered, the administration route, the intended use whether prophylactic or therapeutic, symptoms and the like, and thus, it can be predetermined ad libitum depending on the type of the cellulose ether used, frequency of administration (interval), and each tolerance dose. The pharmaceutical composition of the present invention can prohibit the onset of the conformational disease when administered as a prophylactic drug prior to exposure to the cause of the conformational disease, or prior to appearance of the sign. Further, in the case in which the administration is initiated following the exposure to the cause of the conformational disease or following the onset of the disease, the onset of the conformational disease can be delayed or prohibited, or the progression following the onset can be retarded.

### Example 1

In prion disease that is one of typical conformational diseases, the effect of hydroxypropyl methylcellulose (HPMC) on prion (abnormal prion protein) that is a responsible protein for the disease was evaluated *in vitro.*

### Cellulose Ether Used for Evaluation

Hydroxypropyl methylcellulose (HPMC-TCSRW); manufactured by Shin-Etsu Chemical Co., "TC-5RW" (trade name) (methoxyl group content: 29.2% by weight, hydroxypropoxyl group content: 8.9% by weight, viscosity of 2% by weight aqueous solution at 20°C: 5.81 mm²/s, pH of 1% by weight aqueous solution: 6.8).

### Evaluation Method

According to assay evaluation of Ishikawa, Doh-ura et al., in which cells persistently infected with prion are used (K. Ishikawa, K. Doh-ura, et al., Journal of General Virology, 2004, 85, pp. 1785-1790), *in vitro* evaluation of anti-prion effect was made. Herein, ScN2a cells prepared by persistent infection of neuroblastoma cells N2a with a scrapie prion, RML strain, were used. The cells were seeded into a 6-well dish for culture at a cell number corresponding to 10% of confluence, and an HPMC aqueous solution was added thereto such that the HPMC concentration in the culture medium became 0.1, or 1 mg/mL. As a control (0 mg/mL), sterile distilled water was added to the culture liquid. With respect to the cell culture conditions, Opti-MEM (manufactured by GibcoBRL) to which fetal bovine serum was added at a final concentration of 10% was used as a culture medium. The culture was carried out by allowing the dish for culture to stand still in a carbon dioxide gas incubator regulated to provide 5% CO₂ atmosphere, and adjusted at 37°C for three days. After the confluent cells were washed with phosphate buffered physiological saline (PBS), the cells were lysed in a cell lysis buffer (PBS to which 0.5% polyoxyethylene-p-octylphenol (Wako Pure Chemical Industries, Ltd., "NP-40" (trade name) was used) and 0.5% sodium deoxycholate were added), and the lysate was briefly centrifuged (5,000 x g, 5 min) to obtain a supernatant. Thereto was added proteinase K (PK) (manufactured by Merck & Co., Inc.) to give a final concentration of 10 µg/mL, and the reaction (protein degradation) was allowed at 37°C for 30 min. Thereafter, phenylmethanesulfonyl fluoride (PMSF) was added to give a final concentration of 1 mM, whereby the reaction was terminated. Thereafter, the abnormal prion protein was recovered as a sediment by ultracentrifugation (100,000 x g, 4°C, 30 min) , and the prion protein was analyzed by a Western blotting method. With respect to the Western blotting conditions, the protein electrophoresed on 15% tris glycine SDS-PAGE gel was blotted on a nylon membrane (manufactured by Millipore Corporation, PVDF membrane), and then the prion protein was detected using a mouse monoclonal antibody against prion protein (manufactured by SPI-BIO, SAF83 (5,000 x dilution)), an alkaliphosphatase-conjugate goat anti-mouse immunoglobulin antibody (manufactured by Promega Corporation (20,000 x dilution), and a chemiluminescence detection reagent (manufactured by Amersham plc, CDP-Star detection reagent).

### Results

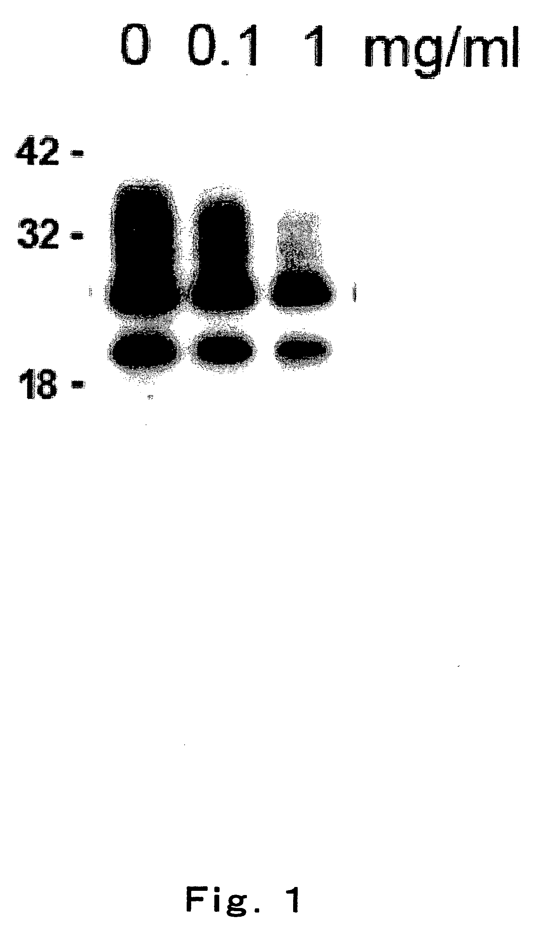

The results are shown in Fig. 1. These results revealed that HPMC (TC-5RW) inhibited, the formation of the abnormal prion protein (i.e., prion) in persistently prion-infected cells in a concentration dependent manner.

### Example 2

Relationship between the administration timing and therapeutic effect for prion disease was studied *in vivo.*

### Evaluation Method

Using HPMC (TC-5RW) , *in vivo* evaluation of anti-prion effect was made according to assay evaluation of Doh-ura et a1. (Katsumi Doh-ura et al. , Journal of Virology, 2004, Vol. 78, pp. 4999-5006), in which Tg7 mouse (genetically modified mouse in which not mouse prion protein but hamster prion protein is overexpressed) and 263K (prion strain of scrapie passaged through hamster) brain homogenate are employed. In this method, infection with prion was performed by intracerebral inoculation to mouse with 20 µL of the 263K brain homogenate adjusted to a concentration of 1% (W/V) with physiological saline. On day 14, day 21, day 28 and day 35 following inoculation, respectively, 75 mg of HPMC (TC-5RW) was subcutaneously administered to the mouse dorsal region. The administration was carried out by a process in which powdery HPMC was wrapped in a wafer sheet (manufactured by Kyokko Corporation, triangle bag (trade name; "Sankaku-gata fukuro")) to give one cartridge, which was subcutaneously embedded.
HPMC (TC-5RW) in an amount of 75 mg was subcutaneously administered: once per week and four times in total in the group received the administration 14 days later; once per week and three times in total in the group received the administration 21 days later; and once per week and twice in total in the group received the administration 28 days later. The control was an HPMC-nonadministered group (administered with wafer sheet alone). Each three male and female eight-weeks or older mice (body weight: 20 to 25 g) were used in every group, and the incubation period from intracerebral infection to disease terminal stage was determined. Thus obtained data were analyzed by one-way analysis of variance (one-way ANOVA) and a multiple comparison test according to a Turkey-Kramer method.

### Results

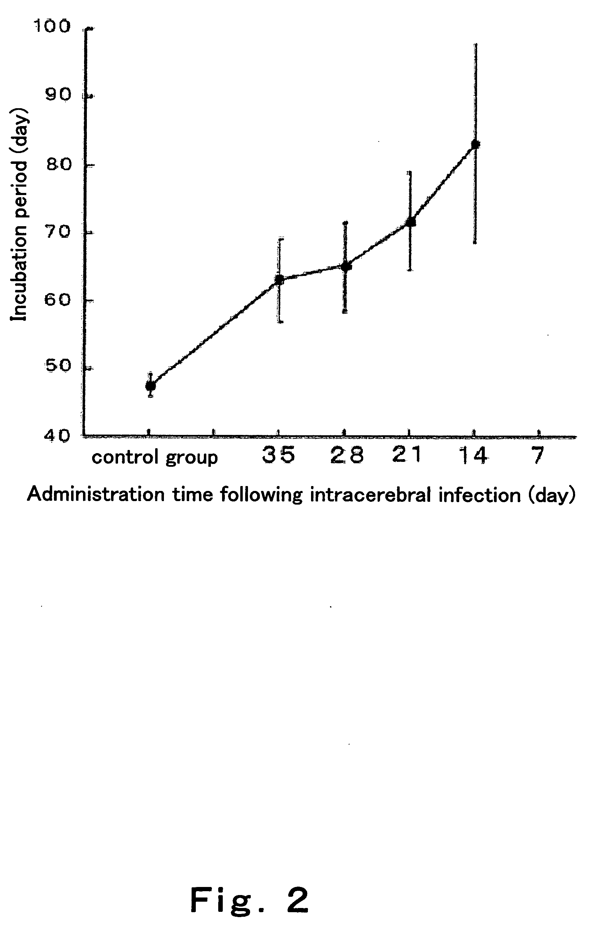

The results are shown in Fig. 2.
The incubation period of the mouse in the group that received the administration of HPMC (TC-5RW) on day 14 following the intracerebral infection with prion was 83.3 ± 14.5 days; that in the group received the administration on day 21 was 72.0 ± 7.0 days; that in the group received the administration on day 28 was 65.3 ± 6.3 days; and that in the group received the administration on day 35 was 63.2 ± 6.2 days. Accordingly, significant extension (p < 0.01 in any case) of the incubation period by the administration of HPMC was observed as compared with the control group i.e., 47.6 ± 2.7 days. In addition, the incubation period of the groups in which the administration was initiated on day 14 was significantly longer than those of the group in which the administration was initiated on day 28 and on day 35 (both p < 0.05), and thus the therapeutic effect for the prion disease was more excellent as the HPMC was administered earlier following the intracerebral infection.

### Example 3

Relationship between the administration frequency and therapeutic effect for prion disease was studied *in vivo.*

### Evaluation Method

Single subcutaneous administration of HPMC (TC-5RW) was compared with multiple subcutaneous administration.
A similar experiment to Example 2 was carried out in which a group (single) that received single subcutaneous administration of 100 mg of HPMC (TC-5RW) one week following the intracerebral infection was compared with a group that received the administration three times, i.e. , first week, second week and third week following the intracerebral infection. Similarly, a group (single) that received single subcutaneous administration of 100 mg of HPMC (TC-5RW) three weeks following the intracerebral infection was compared also with a group that received the administration three times, i.e., third week, fourth week and fifth week following the intracerebral infection. As a control group, an HPMC-nonadministered group (administered with wafer sheet alone) was provided. Each three male and female eight-weeks or older mice (body weight: 20 to 25 g) were used in every group, and the incubation period from intracerebral infection to disease terminal stage was determined. Thus obtained data were analyzed in a similar manner to Example 2.

### Results

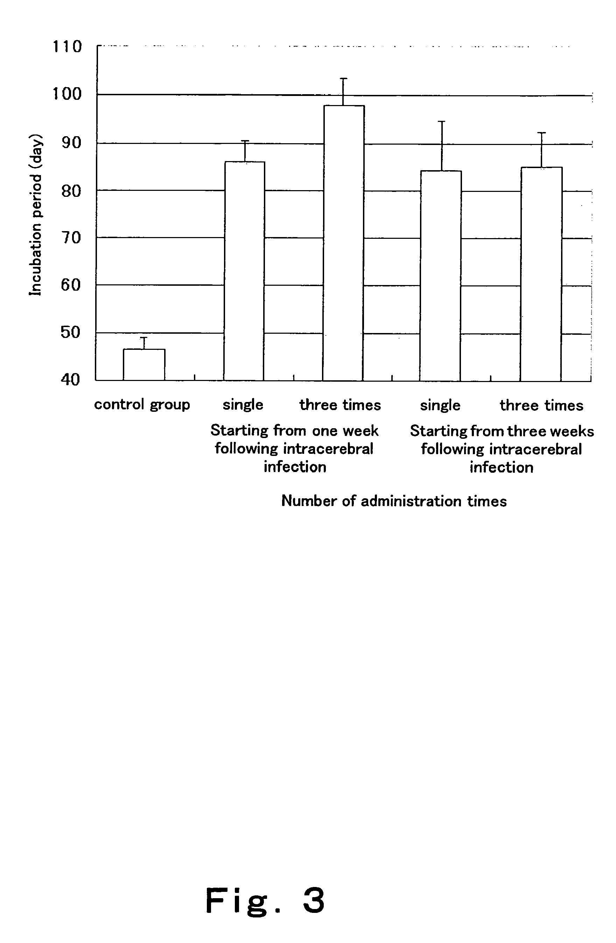

The results are shown in Fig. 3.
The incubation period of the mouse in the group that received the single administration of HPMC on the first week following the intracerebral infection was 86.0 ± 4.4 days; while the incubation period of the mouse in the group that received the administration every week three times starting from the first week following the intracerebral infection was 97.8 ± 5.6 days. Accordingly, the administration of multiple times lead to significant extension (p < 0.01) of the incubation period, with the difference of those periods being about 12 days. However, no significant difference was found between the incubation period of the group received the single administration of HPMC on the third week following the intracerebral infection, 84.2 ± 10.4 days, and the incubation period of the group received the administration every week three times starting from the third week following the intracerebral infection, 85.2 ± 6.9 days. Therefore, the administration of multiple times was effective when it was started from an early stage following the infection, but the extent of the increase in the effect was not so great.

### Example 4

Relationship between the administration dose and therapeutic effect for prion disease was studied in *vivo.*

### Evaluation Method

A similar experiment to Example 2 was carried out in which 15, 25, 50, 100, 150, and 200 mg of HPMC (TC-5RW), respectively, were subcutaneously administered once to the dorsal region on day 5 following the intracerebral infection. As a control group, an HPMC-nonadministered group (administered with wafer sheet alone) was provided. Each three male and female eight-weeks or older mice (body weight: 20 to 25 g) were used in every group, and the incubation period from intracerebral infection to disease terminal stage was determined. Thus obtained data were analyzed in a similar manner to Example 2.

### Results

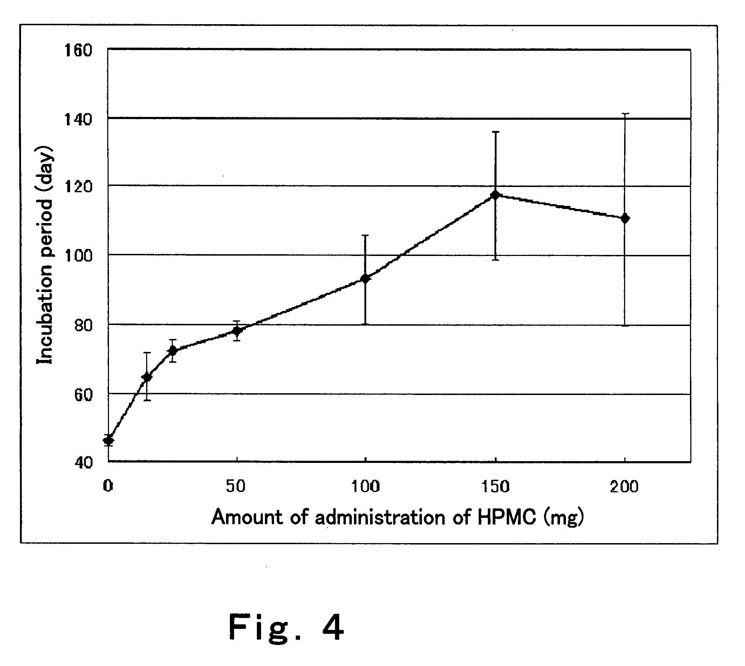

The results are shown in Fig. 4.
HPMC (TC-5RW) lead to extension of the incubation period in a dose dependent manner up to 100 mg. The administration of 100 mg resulted in the incubation period of 93.0 ± 12.5 days; the administration of 150 mg resulted in 117 ± 18.6 days; and the administration of 200 mg resulted in 110.7 ± 30.9 days. Accordingly, the effect reached to almost saturation when the administration dose was 100 mg. In the group received the administration in an amount of 200 mg, two mice died two days following the administration, indicating that the mice could not endure the administration of HPMC in such an amount.

### Example 5

From the experiment in Example 3, it was presumed that HPMC (TC-5RW) has a therapeutic effect for the prion disease for a long period of time even in the case of single administration.
Therefore, *in vivo* study was performed as to whether or not it has the prophylactic effect on the onset even in the case of single administration prior to infection.

### Evaluation Method

A similar experiment to Example 2 was carried out in which 75 mg of HPMC (TC-5RW) was subcutaneously administered once to the dorsal region of each mouse of the groups of, day 74, day 18 and day 9 before the intracerebral infection, and day 22 following the intracerebral infection. As a control group, an HPMC-nonadministered group was provided. The mice were eight-weeks or older, and each three male and female mice (body weight: 20 to 25 g) were used in every group. Accordingly, the incubation period from intracerebral infection to disease terminal stage was determined. Thus obtained data were analyzed in a similar manner to Example 2.

### Results

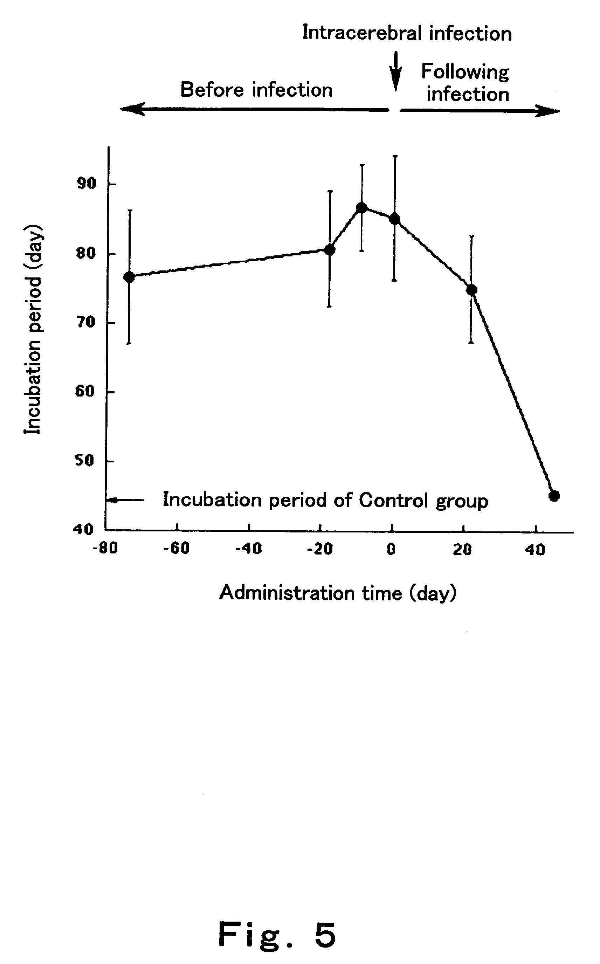

The results are shown in Fig. 5.
In the control group that received no administration of HPMC, the incubation period was 45.3 ± 1.9 days, and 76.7 ± 9.7 days of the incubation period in the group received the administration of HPMC (TC-5RW) on day 74 before the intracerebral infection; 80.8 ± 8.3 days in the group received the administration on day 18 before; 86.8 ± 6.2 days in the group received the administration on day 9 before; 85.3 ± 8.9 days in the group received the administration concurrent to the intracerebral infection; and 75.1 ± 7.8 days in the group received the administration on day 22 following the intracerebral infection were observed. Accordingly, significant extension (p < 0.01 in any case) of the incubation period than that of the control group was revealed. As noteworthy data, no significant differences of the therapeutic and/or prophylactic effects for the prion disease were found among the groups administered on day 74 before, day 18 before, and day 9 before the intracerebral infection, and concurrent to the intracerebral infection, respectively. Because the therapeutic effects for prion disease by single HPMC administration prior to the intracerebral infection was equivalent to the effects exhibited when administered concurrently to the intracerebral infection (even though the administration was conducted two months beforehand in the experiment of Example 5), it was revealed that HPMC (TC-5RW) exhibited an extremely excellent prophylactic effect.

### Example 6

Since Example 5 ascertained the effect of retarding the onset even in the case of single administration of HPMC (TC-5RW) on day 74 before the infection, the effect of single and still earlier administration of agents including other various cellulose ethers were studied *in vivo.*
Cellulose Ethers Used in Evaluation
- Hydroxypropyl methylcellulose (HPMC (TC-5RW)); described in Example 1.
- Hydroxypropyl methylcellulose (HPMC (60SH-50)); manufactured by Shin-Etsu Chemical Co., "60SH-50" (trade name) (methoxyl group content: 28.8%, hydroxypropoxyl group content: 8.9%, viscosity of 2% by weight aqueous solution at 20°C: 50.4 mm²/s (molecular weight: about 9 times larger than HPMC (TC-5RW)), pH of 1% by weight aqueous solution: 6.3).
- Hydroxypropyl methylcellulose (HPMC (60SH-4K)); manufactured by Shin-Etsu Chemical Co., "60SH-4000" (trade name) (methoxyl group content: 29.4%, hydroxypropoxyl group content: 8.9%, viscosity of 2% by weight aqueous solution at 20°C: 3860 mm²/s (molecular weight: about 700 times larger than HPMC (TC-5RW)), pH of 1% by weight aqueous solution: 6.7).

### Evaluation Method

A similar experiment to Example 2 was carried out in which 100 mg of each of various cellulose ethers was subcutaneously administered once to the dorsal region of mouse 6.5 months before the intracerebral infection. As a control group, an HPMC-nonadministered group was provided. The mice were eight-weeks or older, and each seven male and female mice (body weight: 20 to 25 g) were used in every group. Accordingly, the incubation period from intracerebral infection to disease terminal stage was determined. Thus obtained data were analyzed in a similar manner to Example 2.

### Results

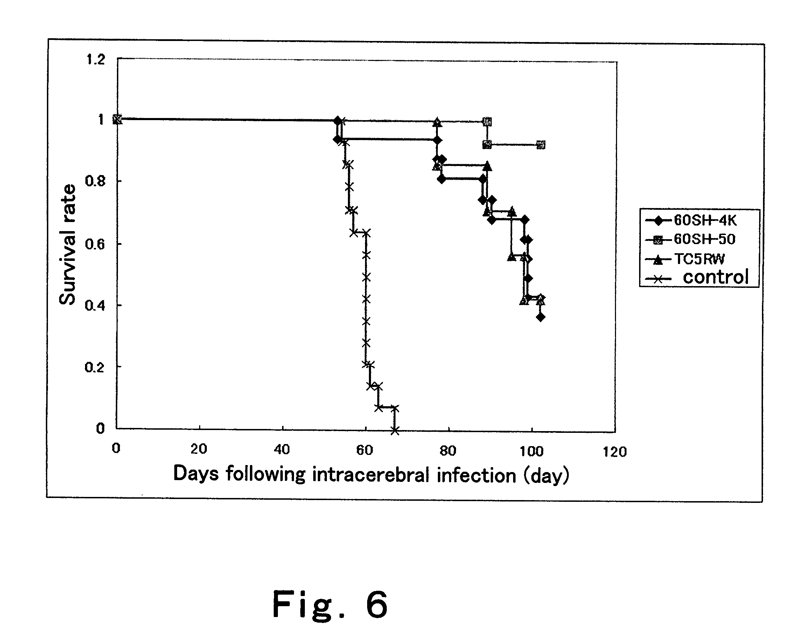

The results are shown in Fig. 6.
In the HPMC-nonadministered group as a control, all mice developed the disease and reached the disease terminal stage within 67 days following the intracerebral infection. To the contrary, in the HPMC (TC-5RW)-administered group and HPMC (60SH-4K)-administered group, about 40% animals survived 102 days following the intracerebral infection, and in the HPMC (60SH-50)-administered group, 90% or more avoided the disease following the intracerebral infection. In the cellulose ethers-administered group, significant extension (p < 0.01 in all cases) of the incubation period was observed as compared with the control group. Particularly, the effect of retarding the onset was significantly greater (P < 0.01) when HPMC (60SH-50) was administered in comparison with the case of HPMC (TC-5RW) administration and HPMC (60SH-4K) administration. From the results described above, it was proven that HPMC, particularly HPMC (60SH-50), exerts extremely excellent prophylactic effect even though it was administered half a year ago or earlier.

### Example 7

Since Example 5 and Example 6 revealed the prophylactic effect of the cellulose ethers for prion disease by single administration before the infection, *in vivo* study as to whether or not a synergistic effect by administration of multiple times before the infection was performed.

### Evaluation Method

A similar experiment to Example 2 was carried out in which a 25 mg/mL solution of HPMC (TC-5RW) in PBS was intraperitoneally administered to a mouse in a volume of 0.6 mL or 1.2 mL one week before, five weeks before, or one week and five weeks before the intracerebral infection. As a control group, 1.2 mL of PBS was intraperitoneally administered one week before the intracerebral infection. Each three male and female eight-weeks or older mice (body weight: 20 to 25 g) were used in every group, and the incubation period from intracerebral infection to disease terminal stage was determined. Thus obtained data were analyzed in a similar manner to Example 2.

### Results

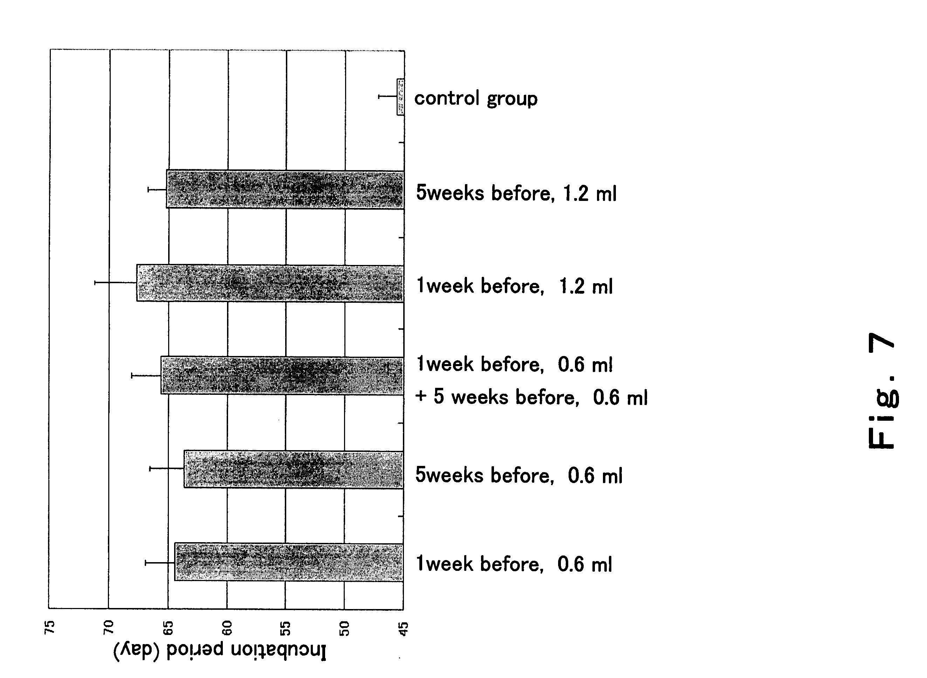

The results are shown in Fig. 7.
Even though the administration was conducted twice, i.e., one week and five weeks before the intracerebral infection, there was no difference in the effects of retarding the onset as compared with the case of single administration one week or five weeks before the infection in an amount of twice. Accordingly, synergistic effect by multiple-times administrations was not observed. This suggests that it is unlikely that a biological defense mechanism such as acquired immunity involving specific antibody production participates in the action mechanism.

### Example 8

Therapeutic effect of various cellulose ethers for prion disease was studied *in vivo* (intraperitoneal administration) by comparison.
Cellulose Ethers Used in Evaluation
- Hydroxypropyl methylcellulose (HPMC (TC-5RW)); described in Example 1.
- Hydroxypropyl methylcellulose (HPMC (TC-5EW)); manufactured by Shin-Etsu Chemical Co., "TC-5EW" (trade name) (methoxyl group content: 28.9%, hydroxypropoxyl group content: 9.0%, viscosity of 2% by weight aqueous solution at 20°C: 2.9 mm²/ s (molecular weight: about half of TC-5RW), pH of 1% by weight aqueous solution: 6.8).
- Methyl cellulose (MC (SM-4)); manufactured by Shin-Etsu Chemical Co., "M-4" (trade name) (methoxyl group content: 29.5%, viscosity of 2% by weight aqueous solution at 20°C: 3.93 mm²/s, pH of 1% by weight aqueous solution: 6.9).
- Hydroxypropyl cellulose (HPCL) ; manufactured by Nippon Soda Co., Ltd., "HPC-SSL" (trade name) (hydroxypropoxyl group content: 62.4%, viscosity of 2% by weight aqueous solution at 20°C: 2.7 mm²/s, pH of 1% by weight aqueous solution: 5.5).

### Evaluation Method

A similar experiment to Example 2 was carried out in which single intraperitoneal administration of 1 mL of a 25 mg/mL solution of each of HPMC (TC-5RW), HPMC (TC-5EW), MC and HPCL prepared in PBS was conducted to mouse on day 5 following the intracerebral infection. As a control group, single intraperitoneal administration was similarly conducted with 1 mL of PBS. Each three male and female eight-weeks or older mice (body weight: 20 to 25 g) were used in every group, and the incubation period from intracerebral infection to disease terminal stage was determined. Thus obtained data were analyzed in a similar manner to Example 2.

### Results

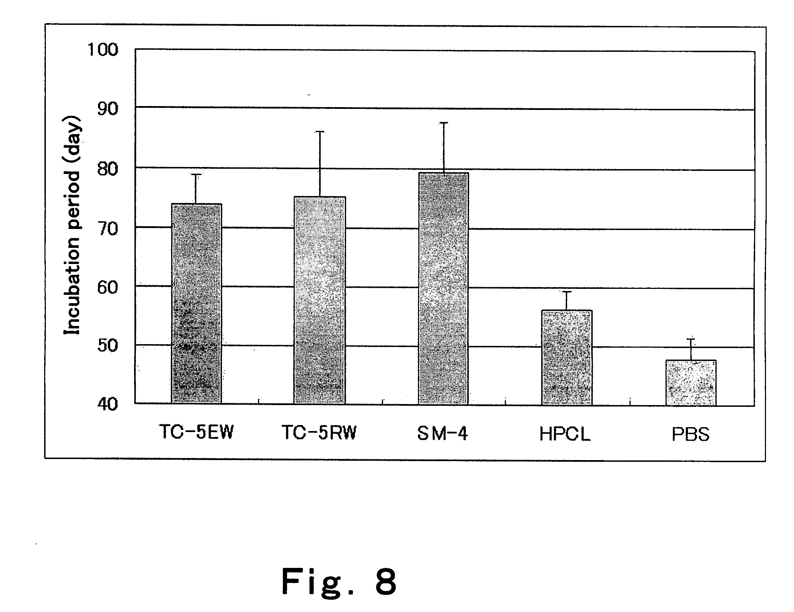

The results are shown in Fig. 8. In the figure, "TC-5EW", "TC-5RW", "SM-4", "HPCL", and "PBS" indicate HPMC (TC-5EW), HPMC (TC-5RW), MC (SM-4), HPCL, and PBS, respectively.
The incubation period was 75.4 ± 10.6 days in the case of HPMC (TC-5RW); 74.0 ± 5.0 days in the case of HPMC (TC-5EW); and 79.3 ± 8.5 days in the case of MC (SM-4), revealing no significant difference among the therapeutic effects of these groups for prion disease. To the contrary, the incubation period in the case of HPCL was 56.3 ± 3.2 days, suggesting significantly inferior effect (p < 0.01) as compared with the foregoing three groups. However, the HPCL group exhibited a significant difference (p < 0.01) from that in the case of PBS as a control group, with the incubation period of 48.0 ± 3.5 days, although the extended incubation period was only about 8 days.

The investigation was not made by the same experiment, but in the case of HPMC (TC-5RW), when the same amount (25 mg) was administered, the incubation period upon intraperitoneal administration was 75.4 ± 10.6 days (in the control group, 48.0 ± 3.5 days), and the incubation period upon subcutaneous administration was 72.4 ± 3.1 days (in the control group, 46.0 ± 1.7 days, see data shown in Fig. 4), demonstrating nearly equal level of the therapeutic effect. In addition, since there was no difference between the therapeutic effects of HPMC (TC-5EW) and HPMC (TC-5RW), it was revealed that the molecular weight of HPMC administered intraperitoneally did not affect the therapeutic effect for prion disease even though the molecular weight was lowered to approximately a half. Furthermore, methyl cellulose had almost the same level of the effect as HPMC, demonstrating a greater effect as compared to hydroxypropyl cellulose.

### Example 9

Therapeutic effect for the prion disease by administration into peripheral vein was studied.

### Evaluation Method

The experiment was carried out in a similar manner to Example 8. However, 300 µL of a 25 mg/mL solution of HPMC (TC-5RW) in PBS was administered once into the tail vein 6 hrs before the intracerebral infection. In the control group, 300 µL of PBS was similarly administered once into the tail vein. Each six male eight-weeks or older mice (body weight: about 25 g) were used in every group, and the incubation period from intracerebral infection to disease terminal stage was determined.

### Results

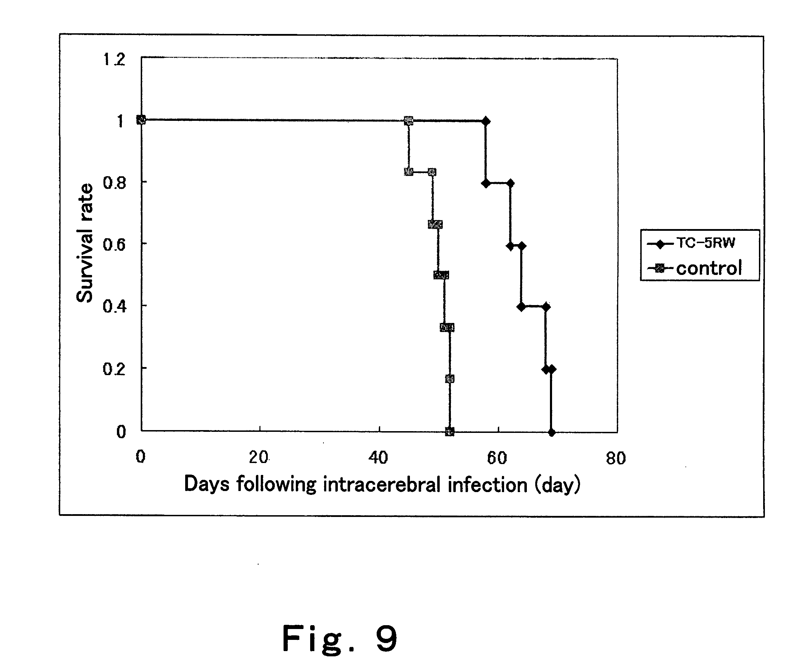

The results are shown in Fig. 9. The incubation period was 49.8 ± 2.6 days in the control group, but it was 64.2 ± 4.5 days in the HPMC-administered group, demonstrating a significant effect (P < 0.01) of retarding the onset in the HPMC-administered group. This result indicates that HPMC is effective also by single administration into peripheral vein.

### Example 10

HPMC was directly administered into brain that is a main target organ of prion, and its therapeutic effect was studied.

### Evaluation Method

The experiment was carried out in a similar manner to Example 8. However, an Alzet osmotic pump (manufactured by DURECT Corporation, "Model 2004") was used which enables continuous injection for 4 weeks, while connecting the pump to a cannula, and the tip of the cannula was inserted into the third ventricle of mouse cerebrum, whereby continuous intracerebroventricular injection into mouse was carried out for 4 weeks. The osmotic pump was subcutaneously indwelled into the mouse dorsal region, where each solution of HPMC (TC-5RW) having a concentration of 0.25 mg/mL, 2.5 mg/mL and 25 mg/mL in PBS, and PBS alone (control group) was filled. Then, each solution started to be fed from the pump into the cerebral ventricle of the mouse on day 8 following the intracerebral infection, thereby administering 1.5 µg, 15 µg, 150 µg, and 0 pg (control group) of HPMC (TC-5RW) per day. Each six male eight-weeks or older mice (body weight: about 25 g) were used in every group, and the incubation period from intracerebral infection to disease terminal stage was determined. Thus obtained data were analyzed similarly to Example 2.

### Results

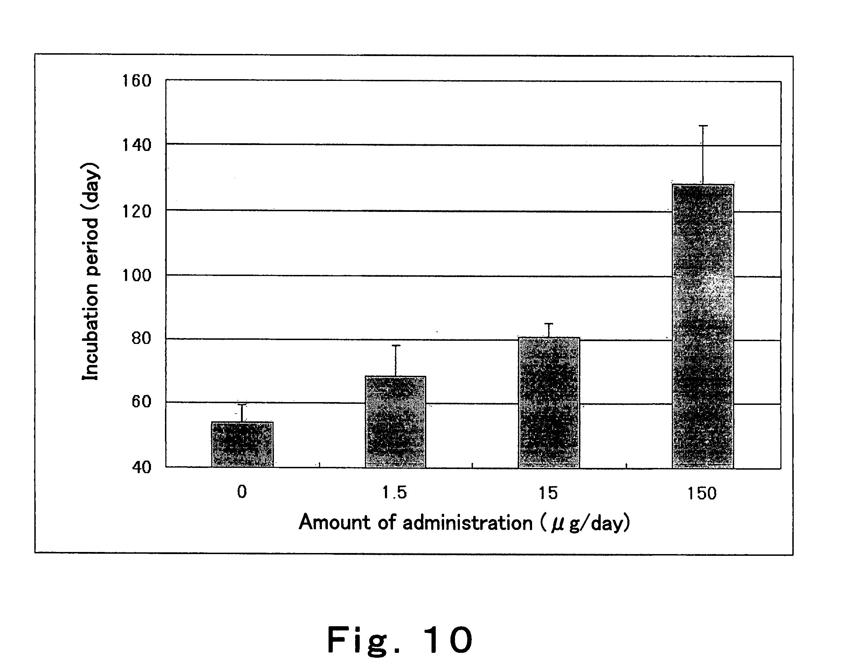

The results are shown in Fig. 10.
The incubation period was 54.0 ± 5.7 days in the control group, but in contrast, in the HPMC-administered group, the incubation period was 68.6 ± 9.7 days in administration of 1.5 µg/day, 80.8 ± 4.4 days in administration of 15 µg/day, and 128.8 ± 17.0 days in administration of 150 µg/day. Accordingly, significant (p < 0.05, p < 0.01, and p < 0.01, respectively) therapeutic effect was exhibited, and the effect was enhanced in a concentration dependent manner of the administration. In the studied range of the administration concentration, no noxious effects occurred in the mice, and saturation of the effect was not observed. However, the therapeutic effect in the 150 µg/day-administered group seems to be a similar level to the saturated effect exhibited upon the subcutaneous administration of HPMC (100 mg (incubation period: 93.0 ± 12.5 days), and 200 mg (incubation period: 110.7 ± 30.9 days in Fig. 4)).

### Example 11

In view of the results in Example 10, comparison of HPMC (TC-5RW) with other HPMC, and comparison with pentosan polysulfate (PPS), which had been reported to have a marked therapeutic effect heretofore, were performed by intracerebroventricular administration.
Cellulose Ethers and Comparative Pharmaceutical Preparation Used in Evaluation
- Hydroxypropyl methylcellulose (HPMC (TC-5RW)); described in Example 1.
- Hydroxypropyl methylcellulose HPMC (60SH-400); manufactured by Shin-Etsu Chemical Co., "60SH-400" (trade name) (methoxyl group content: 29.1% by weight, hydroxypropoxyl group content: 9.0% by weight, viscosity of 2% by weight aqueous solution at 20°C: 450 mm²/s (molecular weight: about 80 times larger than TC-5RW), pH of 1% by weight aqueous solution: pH 6.8).
- Hydroxypropyl methylcellulose HPMC (HPMC602); manufactured by Shin-Etsu Chemical Co., "HPMC602" (trade name) (methoxyl group content: 29.1%, hydroxypropoxyl group content: 9%, viscosity of 2% by weight aqueous solution at 20°C: 1.68 mm²/s (molecular weight: about 1/3.5 of TC-5RW), pH of 1% by weight aqueous solution: 6.8).
- Pentosan polysulfate (PPS); sodium pentosan polysulfate (manufactured by Biopharm Australia Pty Ltd., "Cartrophen Vet" (trade name)).

### Evaluation Method

The experiment was carried out in a similar manner to Example 10. The HPMCs used were adjusted with a PBS solution to have a concentration of 25 mg/mL, and PPS was prepared by evaporating a sodium PPS injectable solution to dryness to remove the alcoholic antiseptic agent, followed by dissolving in a PBS solution to have a concentration of 1.25 mg/mL. The solutions were filled in the pump, respectively. After 20 µL of the 263K brain homogenate having a concentration of 0.1% (W/V) was inoculated into mouse brain, intracerebroventricular administration of the sample solution was started on day 3. Each seven male eight-weeks or older mice (body weight: about 25 g) were used in every group, and the incubation period from intracerebral infection to disease terminal stage was determined. Thus obtained data were analyzed similarly to Example 2.

### Results

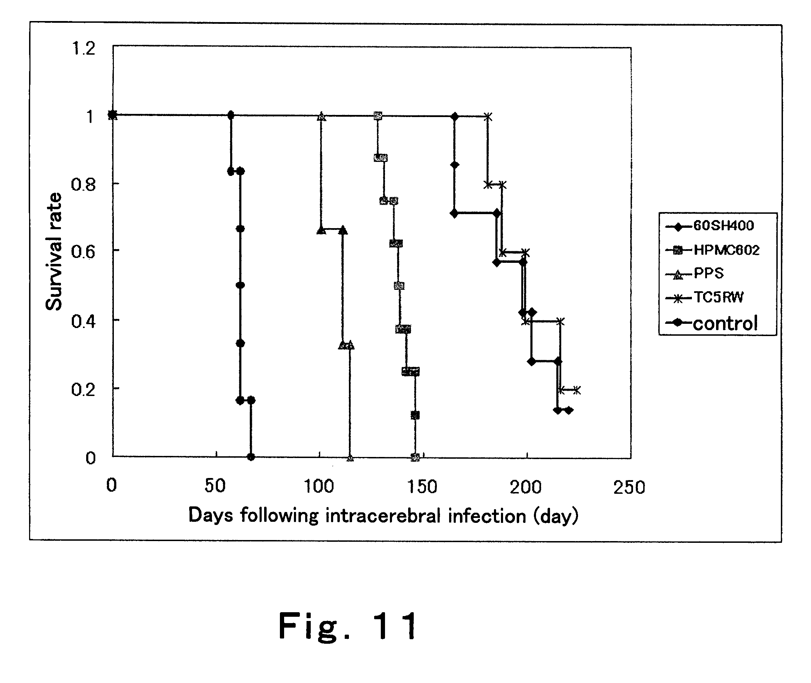

The results are shown in Fig. 11.
The incubation period was 62.0 ± 3.2 days in the control group, and the incubation period was 109.0 ± 7.2 days in the PPS-administered group. To the contrary, the incubation period was 138.3 ± 6.5 days in the HPMC (HPMC602)-administered group, and longer in incubation period than that was observed in the HPMC (60SH-400)-administered group and HPMC (TC-5RW)-administered group. In these groups, 14%, and 20% of the mice avoided the disease onset, respectively even after a lapse of 224 days. Thus, HPMCs are more effective than PPS significantly (p < 0.01, respectively) . The PPS product used, and the preparation procedure are the same as in a reported literature (Katsumi Doh-ura, et al., Journal of Virology, 2004, Vol. 78, pp. 4999-5006). Hence, this Example demonstrates the results of comparative study carried out at a concentration of PPS approximately close to the reported concentration at which the greatest therapeutic effect was exhibited, and indicates that HPMCs are extremely safer and exhibit much more excellent therapeutic effects.

### Example 12

The foregoing Examples revealed that cellulose ethers exhibited the therapeutic effect for prion disease via any of the administration routes of subcutaneous, interperitoneal, intravenous, and intracerebroventricular routes. Therefore, the therapeutic effect upon concomitant administration via multiple administration routes was studied.

### Cellulose Ethers Used in Evaluation

- Methyl cellulose (MC (SM-4)); described in Example 8.

### Evaluation Method

The experiment was carried out in a similar manner to Example 2 for the subcutaneous administration, and in a similar manner to Example 8 for the intraperitoneal administration. After 20 µL of the 263K brain homogenate having a concentration of 1% (W/V) was inoculated into mouse brain, 0.5 mL or 1.0 mL of a 50 mg/mL aqueous solution of MC (SM-4) was intraperitoneally administered, or 25 mg or 50 mg was subcutaneously administered. In the duplicate administration group, 0.5 mL was intraperitoneally administered, and 25 mg was subcutaneously administered. In the control group, 0.5 mL of distilled water was intraperitoneally administered, and the wafer sheet alone was subcutaneously administered. Each four male eight-weeks or older mice (body weight: about 25 g) were used in every group, and the incubation period from intracerebral infection to disease terminal stage was determined. Thus obtained data were analyzed similarly to Example 2.

### Results

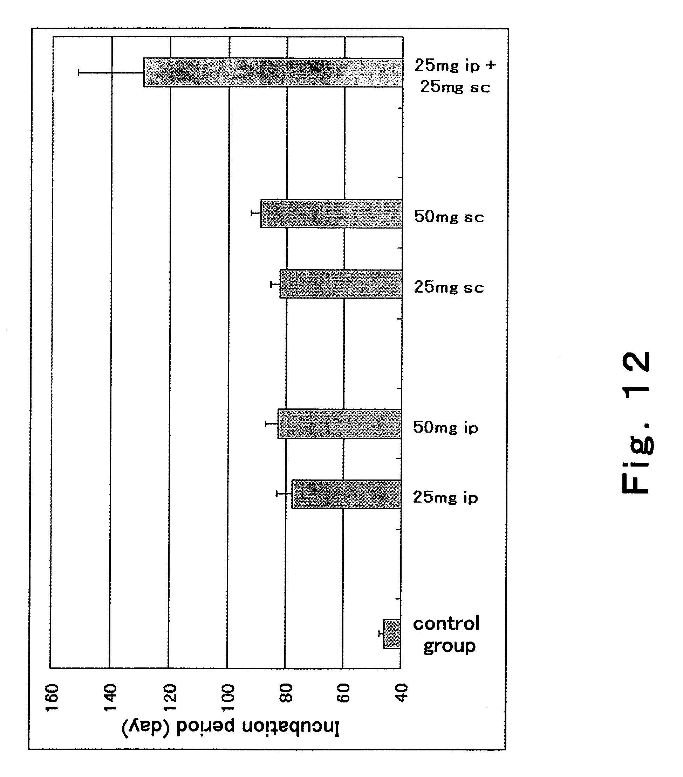

The results are shown in Fig. 12. In the figure, "ip" represents intraperitoneal administration, and "sc" represents subcutaneous administration. The incubation periods of the 25 mg-intraperitoneally administered group, 50 mg-intraperitoneally administered group, 25 mg-subcutaneously administered group, and 50 mg-subcutaneously administered group were 77.5 ± 5.8 days, 83.0 ± 3.9 days, 82.4 ± 3.1 days, and 89.0 ± 3.2 days, respectively, while the incubation period of the duplicate administration group, i.e., 25 mg intraperitoneal administration + 25 mg subcutaneous administration, was 129.0 ± 22.6 days. This suggests that duplicate administration via different administration routes can prominently enhance the therapeutic effect for prion disease.

### Example 13

The therapeutic effect by duplicate administration via different administration routes shown in Example 12 was further studied by a combination of subcutaneous administration and intracerebroventricular administration.
Cellulose Ethers Used in Evaluation
- Hydroxypropyl methylcellulose (HPMC (TC-5RW)); described in Example 1.

### Evaluation Method

The subcutaneous administration was conducted similarly to Example 2, and the intracerebroventricular administration was conducted similarly to Example 10. After 20 µL of the 263K brain homogenate having a concentration of 0.1% (W/V) was inoculated into mouse brain, subcutaneous administration of 50 mg of HPMC (TC-5RW), or intracerebroventricular administration of a solution of HPMC (TC-5RW) in PBS having a concentration of 25 mg/mL filled in the pump was initiated on day 3. In the duplicate administration group, 50 mg was subcutaneously administered, and concomitantly the solution having a concentration of 25 mg/mL was filled in the pump to conduct the intracerebroventricular administration. In the control group, the wafer sheet alone was subcutaneously administered, or the PBS solution alone was filled in the pump to conduct the intracerebroventricular administration. Each seven male eight-weeks or older mice (body weight: about 25 g) were used in every group, and the incubation period from intracerebral infection to disease terminal stage was determined. Thus obtained data were analyzed similarly to Example 2.

### Results

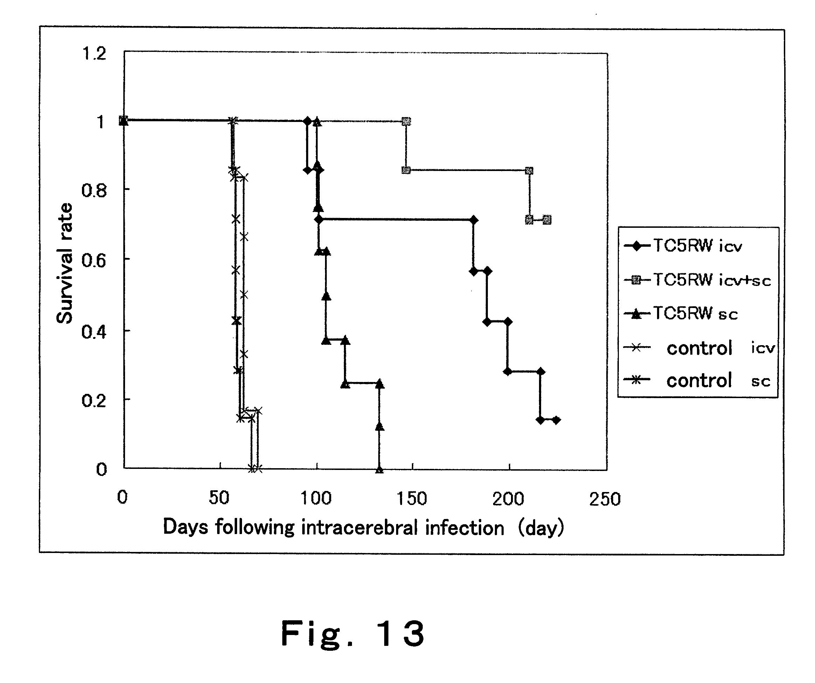

The results are shown in Fig. 13. In the figure, "icv" represents intracerebroventricular administration, and "sc" represents subcutaneous administration.
The incubation period was 111.5 ± 14.1 days in the 50 mg-subcutaneously administered group. In the 25 mg/mL-intracerebroventricularly administered group, or in the duplicate administration group, i.e., 25 mg/mL intracerebroventricular administration + 50 mg subcutaneous administration, 14%, and 71% mice avoided the disease onset, respectively, even after a lapse of 224 days following the intracerebral infection. Therefore, it was ascertained that the duplicate administration of subcutaneous and intracerebroventricular administration exhibited evidently more excellent effect of retarding the onset than the subcutaneous or intracerebroventricular administration via single route, and that the duplicate administration prominently enhances the therapeutic effect even in the case of the combination of the subcutaneous administration and intracerebroventricular administration.

### Example 14

It is known that infection of bovine spongiform encephalopathy (BSE), scrapie, deer chronic wasting disease in animals, as well as human variant Creutzfeldt-Jakob disease and the like among prion diseases occurs by intake of a substance contaminated with prion from periphery, particularly by oral intake. Therefore, the therapeutic and prophylactic effect by cellulose ethers on the prion infection from periphery was studied.

### Cellulose Ethers Used in Evaluation

- Hydroxypropyl methylcellulose (HPMC (TC-5RW)); described in Example 1.

### Evaluation Method

Using the Tg7 mouse and 263K strain prion similarly to Example 2, peripheral infection with prion was allowed by intraperitoneal inoculation. The 263K brain homogenate was prepared to have a concentration of 1% (W/V) with physiological saline, 100 µL of this homogenate was inoculated into mouse peritoneal cavity, and the prion was allowed to infect. In the test, 100 mg of HPMC (TC-5RW) had been subcutaneously administered in the mouse dorsal region just before the intraperitoneal infection, while HPMC was not administered (i.e., wafer sheet alone) in the control group. Each three male and female eight-weeks or older mice (body weight: 20 to 25 g) were used in every group, and the incubation period from intraperitoneal infection to disease terminal stage was determined.

### Results

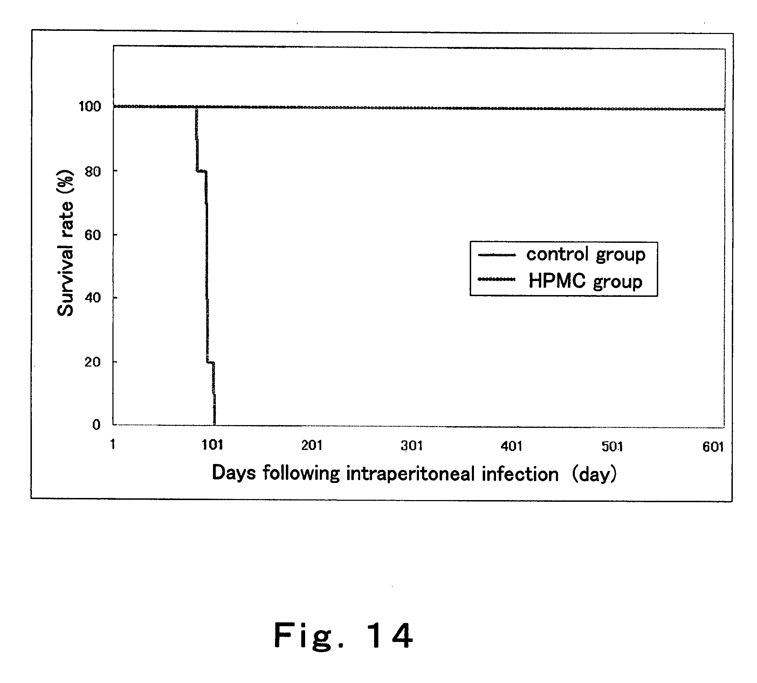

The results are shown in Fig. 14.
The incubation period was 93.0 ± 7.4 days in the HPMC-nonadministered group (control group), to the contrary, development of the prion disease was not observed at all in the HPMC-administered group even after a lapse of 610 days. Also in the case of direct infection into brain that is the target organ of prion, HPMC exhibited an excellent effect of retarding the onset upon peripheral administration (i.e., subcutaneous administration, peripheral intravascular administration, and intraperitoneal administration), but the effect was even more prominent in the case of peripheral infection with prion.

### Example 15

Therapeutic effect for prion disease by cellulose ethers was studied by changing the animal species to syrian hamster.

### Evaluation Method

The scrapie prion strain, 263K causes the prion disease also in hamsters by infection. Five to six-weeks old female syrian hamsters (3 animals/group) (available from Japan SLC, Inc., body weight: approximately 90 g/animal) were infected with prion by inoculating into brain 40 µL of the 263K brain homogenate adjusted to have a concentration of 1% (W/V) concentration with physiological saline. After a lapse of eight days following the intracerebral infection, 150 mg, 300 mg, 450 mg, and 600 mg of HPMC (TC-5RW) were subcutaneously administered, respectively, into the dorsal region of the syrian hamster. Moreover, after a lapse of 8 days, 16 days, 56 days (time at which manifestation of evident neurologic symptom (ataxia) due to the disease was already found) following the intracerebral infection, 300 mg of HPMC (TC-5RW) was subcutaneously administered into the dorsal region of the syrian hamster (administration was carried out by wrapping in a wafer sheet and embedding under the skin, in a similar manner to Example 2). As the control group, a HPMC-nonadministered group (wafer sheet alone) was provided. The incubation period from intracerebral infection to disease terminal stage was determined, and thus obtained data were analyzed similarly to Example 2.

### Results

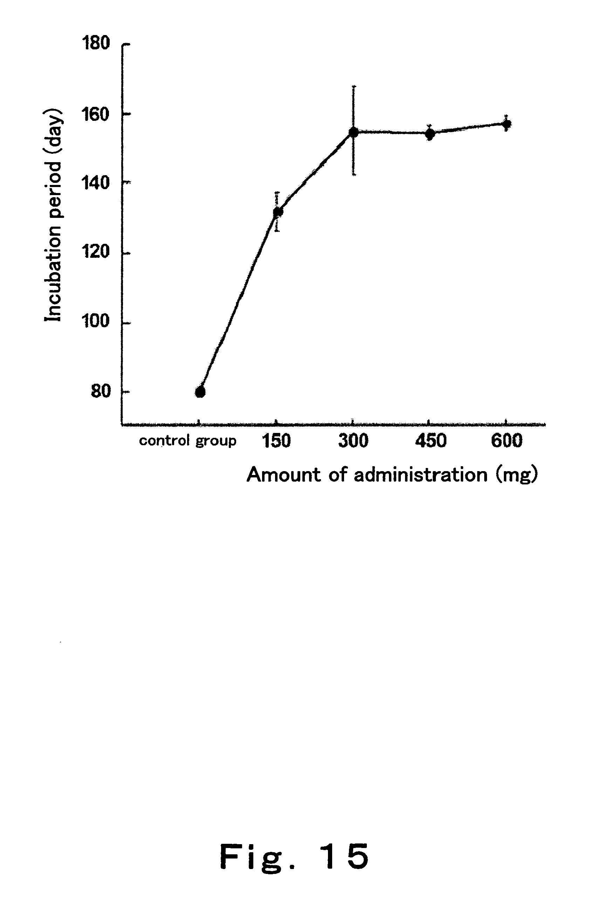

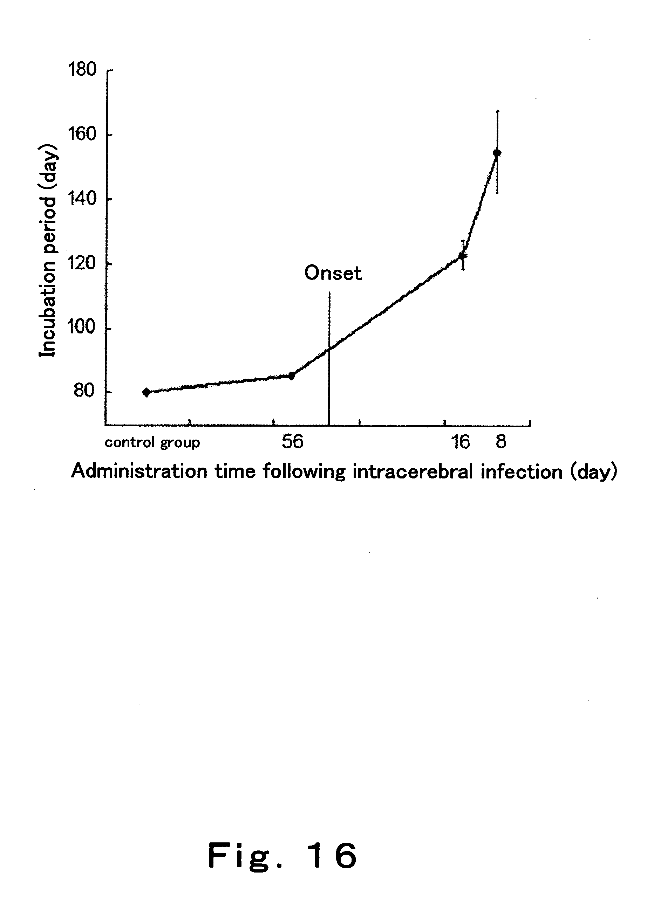

Fig. 15 shows the results of study on the amount of administration, and Fig. 16 shows the results of study on the administration timing.
With respect to the relationship with the amount of administration, the incubation period was 80.0 ± 2.1 days in the HPMC-nonadministered group (control group), but in the HPMC-administered group, the incubation period was 132.0 ± 5.6 days in the 150 mg-administered group, 155.0 ± 12.7 days in the 300 mg-administered group, 154.5 ± 2.1 days in the 450 mg-administered group, and 157.5 ± 2.1 days in the 600 mg-administered group, whereby marked extension (p < 0.01 in any case) of the incubation period was observed in all cases. When the amount was equal to or greater than 300 mg, the effect was saturated, and the effect exhibited with 150 mg was somewhat inferior compared to the effect exhibited with 300 mg (p < 0.01).
With respect to the relationship with the administration timing, the incubation period was 80.0 ± 2.1 days in the HPMC-nonadministered group (control group), but in the HPMC-administered group, the incubation period was 155.0 ± 12.7 days upon administration after a lapse of 8 days following the intracerebral infection, 123.2 ± 4.3 days upon administration after a lapse of 16 days, and 85.0 ± 0.3 days upon administration after a lapse of 56 days. Thus, the incubation period was further prolonged as the timing of the administration following the infection is earlier (p < 0.01 in any case). Also in the case of single administration following the disease onset (day 56 following the intracerebral infection), it is noteworthy that a significant amelioration effect on survival period was observed although the exhibited effect was not great.
The foregoing results demonstrate that the therapeutic and prophylactic effects for prion disease by cellulose ethers are observed also in animal species other than mouse.

### Example 16

Therapeutic effect of cellulose ethers for the prion disease was studied *in vivo* with respect to RML strain that is a prion strain of scrapie passaged through mice.
Cellulose Ethers Used in Evaluation
- Hydroxypropyl methylcellulose (HPMC (TC-5RW)); described in Example 1.
- Hydroxypropyl methylcellulose (HPMC (TC-5EW)); described in Example 8.
- Hydroxypropyl methylcellulose (HPMC (60SH-50)); described in Example 6.
- Hydroxypropyl methylcellulose (HPMC (60SH-4K)); described in Example 6.
- Methyl cellulose (MC (SM-4)); described in Example 8.
- Hydroxypropyl cellulose (HPCL); described in Example 8.
- Carmellose calcium (carboxymethyl cellulose calcium)[CMC (ECG-505)); manufactured by Gotoku Chemical Co., Ltd., "ECG-505" (trade name).
- Hydroxypropyl methylcellulose acetate succinate (HPMCAS (AS-MG)); manufactured by Shin-Etsu Chemical Co., Shin-Etsu AQOAT "AS-MG" (trade name) (methoxyl group content: 23.3%, hydroxypropoxyl group content: 7.3%, acetyl group content: 8.8%, succinoyl group content: 10.9%, viscosity of 2% by weight aqueous solution at 20°C_{:} 2.74 mm²/ₛ).

### Evaluation Method

A brain homogenate of RML strain was prepared by adjusting to give a concentration of 1% (W/V) with physiological saline. After intracerebral infection of the C57BL/6 mouse (available from Japan SLC, Inc.) was performed using 20 µL of this homogenate, each 100 mg of HPMC (TC-5EW), HPMC (TC-5RW), HPMC (60SH-50), HPMC (60SH-4K), MC (SM-4), HPCL, CMC (ECG-505), or HPMCAS (AS-MG) was subcutaneously administered once to the mouse dorsal region immediately before the intracerebral infection (administration was carried out by wrapping in a wafer sheet, and embedding subcutaneously in a similar manner to Example 2). As the control group, a compound-nonadministered group (wafer sheet alone) was provided. Five male eight-weeks or older mice (body weight: about 20 g) were used in every group, and the incubation period from intracerebral infection to disease terminal stage was determined. Thus obtained data were analyzed similarly to Example 2.

### Results

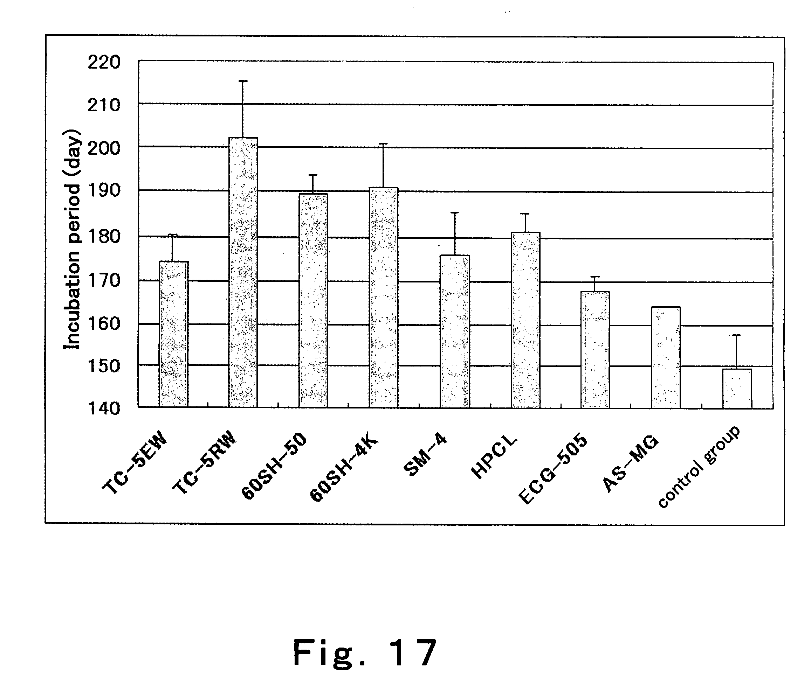

The results are shown in Fig. 17. In the figure, "TC-5EW", "TC-5RW", "60SH-50", "60SH-4K", "SM-4", "HPCL", "ECG-505", and "AS-MG" indicate HPMC (TC-5EW), HPMC (TC-5RW), HPMC (60SH-50), HPMC (60SH-4K), MC (SM-4), HPCL, CMC (ECG-505), and HPMCAS (AS-MG), respectively.
The incubation period was 149.3 ± 8.5 days in the control group, while with the cellulose ethers used herein, the incubation period was 174.3 ± 5.9 days with HPMC (TC-5EW), 202.5 ± 13.4 days with HPMC (TC-5RW), 189.6 ± 4.2 days with HPMC (60SH-50), 191.0 ± 9.9 days with HPMC (60SH-4K), 175.7 ± 9.8 days with MC (SM-4), 181.0 ± 4.2 days with HPCL, 167.5 ± 3.5 days with CMC (ECG-505), and 164 ± 0 days with HPMCAS (AS-MG). Accordingly, the administration prolonged the incubation period, suggesting the therapeutic effect for prion disease (p < 0.01 in any case). However, in the case of HPMCAS (AS-MG), three animals died within two days following the administration since they could not endure the administration in such an amount of 100 mg.

When compared among the HPMCs, significant difference of the therapeutic effect was not found among HPMC (TC-5RW), HPMC (60SH-50), and HPMC (60SH-4K), but HPMC (TC-5EW) exhibited a somewhat less effect compared to these HPMCs (p < 0.01). In the case of MC (SM-4) and HPCL, no significant difference was found, and almost similar effect to that of HPMC (TC-5EW) was found. In the case of CMC (ECG-505) and HPMCAS (AS-MG), the effect is likely to be somewhat inferior to HPCL, and a significant difference (p < 0.01) was found, but the incubation period was prolonged by 18 days or 15 days as compared with the control group, thereby suggesting a significant (p < 0.01) effect.
In this experiment, it was demonstrated that the effect of HPMC was reduced when the molecular weight was lowered to approximately a half (HPMC (TC-5EW)) of HPMC (TC-5RW). In addition, the results were different from the results obtained when the 263K prion strain was used, in that methyl cellulose (SM-4) exhibited a similar effect to HPCL, while it exhibited less effect than HPMC (TC-5RW).

### Example 17

Fukuoka 1 strain that is a prion strain of a human prion disease which was passaged through mice was studied.

### Evaluation Method

The experiment was carried out in a similar manner to Example 16 with the C57BL/6 mouse infected with prion by intracerebral inoculation of 20 µL of a brain homogenate of Fukuoka 1 strain adjusted to have a concentration of 1% (W/V) with physiological saline.

### Results

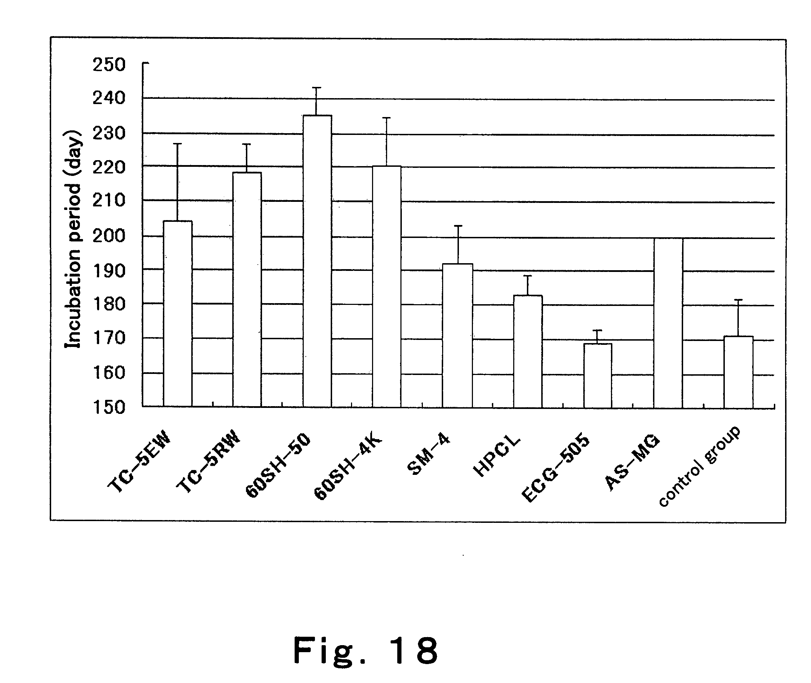

The results are shown in Fig. 18.
There was no significant difference between the incubation period of 171.3 ± 10.7 days in the control group, and the incubation period of 183.0 ± 5.7 days in the HPCL group and the incubation period of 169.0 ± 3.6 days in the CMC (ECG-505) group, but with other cellulose ethers, the incubation period was 204.3 ± 22.5 days in the HPMC (TC-5EW) group, 218.0 ± 8.4 days in the HPMC (TC-5RW) group, 235.5 ± 7.8 days in the HPMC (60SH-50) group, 220.5 ± 14.1 days in the HPMC (60SH-4K) group, 191.7 ± 11.0 days in the MC (SM-4) group, and 200 ± 0 days in the HPMCAS (AS-MG) group, exhibiting significant therapeutic effect (p < 0.05 with HPMC (TC-5EW), MC (SM-4), and p < 0.01 with others). However, in the case of HPMCAS (AS-MG), three animals died within two days following the administration since they could not endure the administration in such an amount of 100 mg.

HPMC (60SH-50) exhibited the highest therapeutic effect, and also HPMC (60SH-4K) exhibited a similar effect. In addition, HPMC (TC-5RW) (p < 0.01) and HPMC (TC-5EW) (p< 0.05) exhibited a somewhat inferior effect to HPMC (60SH-50). There was no significant difference between MC (SM-4) and HPMCAS (AS-MG), and these exhibited a lower effect than HPMCs (p < 0.01 with HPMC (TC-5RW), HPMC (60SH-50), HPMC (60SH-4K), and no significant difference with HPMC (TC-5EW)).
The foregoing results demonstrate that HPMCs are the most effective cellulose ethers also on the Fukuoka 1 strain prion similarly to the RML prion.

### Example 18

Therapeutic effect of cellulose ethers for prion disease was studied with respect to 22L strain that is another prion strain of scrapie passaged through mice.

### Cellulose Ethers Used in Evaluation

- Hydroxypropyl methylcellulose (HPMC (TC-5RW)); described in Example 1.

### Evaluation Method

The experiment was carried out in a similar manner to Example 16 with the C57BL/6 mouse infected with prion by intracerebral inoculation of 20 µL of a brain homogenate of 22L strain adjusted to have a concentration of 1% (W/V) with physiological saline.

### Results

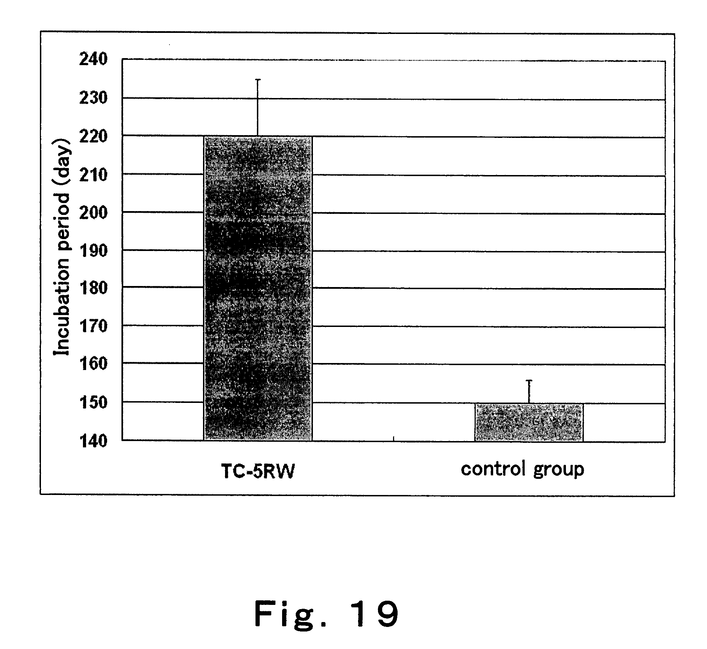

The results are shown in Fig. 19.
Contrary to the incubation period of 150.3 ± 6.2 days in the control group, the incubation period was 220.3 ± 15.1 days in the HPMC (TC-5RW)-administered group, revealing a significant therapeutic effect (p < 0.01 . From the foregoing Examples, it was proven that the cellulose ethers exhibited effects on a wide variety of prion strain without limitation to particular prion strains.

### Example 19

Therapeutic effects exerted on prion disease were studied in concern with the content of the methoxyl group and the hydroxypropoxyl group in the cellulose ethers, and molecular weight of the cellulose ethers.

### Cellulose Ethers Used in Evaluation

- Hydroxypropyl methylcellulose (HPMC (60SH-4K)); described in Example 6. (methoxyl group content: 29.4%, hydroxypropoxyl group content: 8.9%, viscosity of 2% by weight aqueous solution at 20°C: 3860 mm²/s, pH of 1% by weight aqueous solution: 6.7)
- Hydroxypropyl methylcellulose (HPMC (90SH-4K)); manufactured by Shin-Etsu Chemical Co., "90SH-4000" (trade name) (methoxyl group content: 23.1%, hydroxypropoxyl group content: 9.3%, viscosity of 2% by weight aqueous solution at 20ºC: 4450 mm²/s (molecular weight: approximate to 60SH-4K), pH of 1% by weight aqueous solution: 6.8).
- Hydroxypropyl methylcellulose (HPMC (HPMC602)); described in Example 11. (methoxyl group content: 29.1%, hydroxypropoxyl group content: 9%, viscosity of 2% by weight aqueous solution at 20°C: 1.68 mm²/s, pH of 1% by weight aqueous solution: 6.8.)
- Hydroxypropyl methylcellulose (HPMC (HPMC902)); manufactured by Shin-Etsu Chemical Co., "HPMC902" (trade name) (methoxyl group content: 22.6%, hydroxypropoxyl group content: 6.7%, viscosity of 2% by weight aqueous solution at 20ºC: 1.87 mm²/s (molecular weight: approximate to HPMC602), pH of 1% by weight aqueous solution: 6.8.

### Evaluation Method

To the Tg7 mouse infected with prion by intracerebral inoculation of 20 µL of a brain homogenate of 263K strain having a concentration of 1% (W/V), 50 mg of each of the cellulose ethers was subcutaneously administered 3 days before the infection. Each three male and female eight-weeks or older mice (body weight: 20 to 25 g) were used in every group, and the incubation period from intracerebral infection to disease terminal stage was determined. Thus obtained data were analyzed similarly to Example 2.

### Results

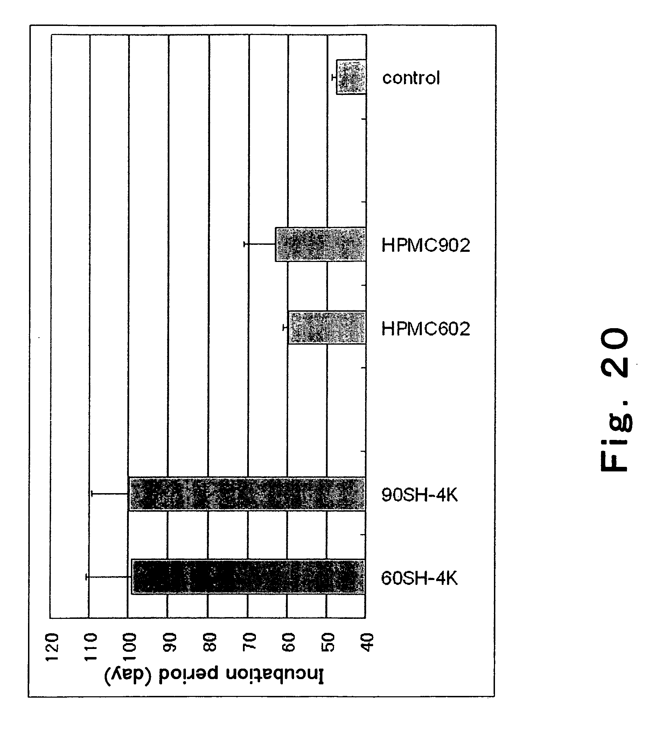

The results are shown in Fig. 20.
With respect to the group having a high viscosity of the aqueous solution, the incubation period was 99.2 ± 11.5 days in the HPMC (60SH-4K)-administered group, and the incubation period was 99.7 ± 9.5 days in the HPMC (90SH-4K)-administered group. Accordingly, no significant difference in the effects was found even though the contents of the methoxyl group and the hydroxypropoxyl group vary. In the group having a low viscosity of the aqueous solution, the incubation period was 59.8 ± 1.6 days in the HPMC (HPMC602) -administered group, and the incubation period was 63.3 ± 8.1 days in the HPMC (HPMC902) -administered group. Thus, no significant difference in the therapeutic effect within the range of the content of each of the methoxyl group and the hydroxypropoxyl group. Meanwhile, a significant difference in the incubation period was found between the group having a high viscosity of the aqueous solution, and the group having a low viscosity of the aqueous solution. Therefore it is reveled that the molecular weight of the cellulose ethers rather affect to a greater extent on the therapeutic effect for prion disease.

### Example 20

In view of the results of Example 19, the influences on the therapeutic effect for prion disease by the molecular weight of the cellulose ethers were studied in more detail.

### Cellulose Ethers Used in Evaluation

In this Example, various HPMCs having a varying viscosity were used.
- Hydroxypropyl methylcellulose (HPMC (HPMC602)); described in Example 11. (methoxyl group content: 29.1%, hydroxypropoxyl group content: 9%, viscosity of 2% by weight aqueous solution at 20ºC: 1.68 mm²/s)
- Hydroxypropyl methylcellulose (HPMC (TC-5EW)); described in Example 8. (methoxyl group content: 28.9%, hydroxypropoxyl group content: 9.0%, viscosity of 2% by weight aqueous solution at 20°C : 2.9 mm²/s)
- Hydroxypropyl methylcellulose (HPMC (TC-5RW)); described in Example 1.
   (methoxyl group content: 29.2% by weight, hydroxypropoxyl group content: 8.9% by weight, viscosity of 2% by weight aqueous solution at 20°-C: 5.81 mm²/s)
- Hydroxypropyl methylcellulose (HPMC (60SH-50)); described in Example 6.
   (methoxyl group content: 28.8%, hydroxypropoxyl group content: 8.9%, viscosity of 2% by weight aqueous solution at 20°C : 50.4 mm²/s)
- Hydroxypropyl methylcellulose (HPMC (HPMC1657)); manufactured by Shin-Etsu Chemical Co., "HPMC1657" (trade name). (methoxyl group content: 32.2%, hydroxypropoxyl group content: 8.5%, viscosity of 2% by weight aqueous solution at 20°C: 210 mm²/s)
- Hydroxypropyl methylcellulose (HPMC (60SH-4K)); described in Example 6.
   (methoxyl group content: 29.4%, hydroxypropoxyl group content: 8.9%, viscosity of 2% by weight aqueous solution at 20°C : 3860 mm²/s)
- Hydroxypropyl methylcellulose (HPMC (60SH-10K)); manufactured by Shin-Etsu Chemical Co., "60SH-10000" (trade name). (methoxyl group content: 29.3%, hydroxypropoxyl group content: 9%, viscosity of 2% by weight aqueous solution at 209C: 9730 mm²/s)

### Evaluation Method

To the Tg7 mouse infected with prion by intracerebral inoculation of 20 µL of a brain homogenate of 263K strain having a concentration of 1% (W/V), 50 mg of each of the cellulose ethers was subcutaneously administered 3 days before the infection. Each three male and female eight-weeks or older mice (body weight: 20 to 25 g) were used in every group, and the incubation period from intracerebral infection to disease terminal stage was determined. Thus obtained data were analyzed similarly to Example 2.

### Results

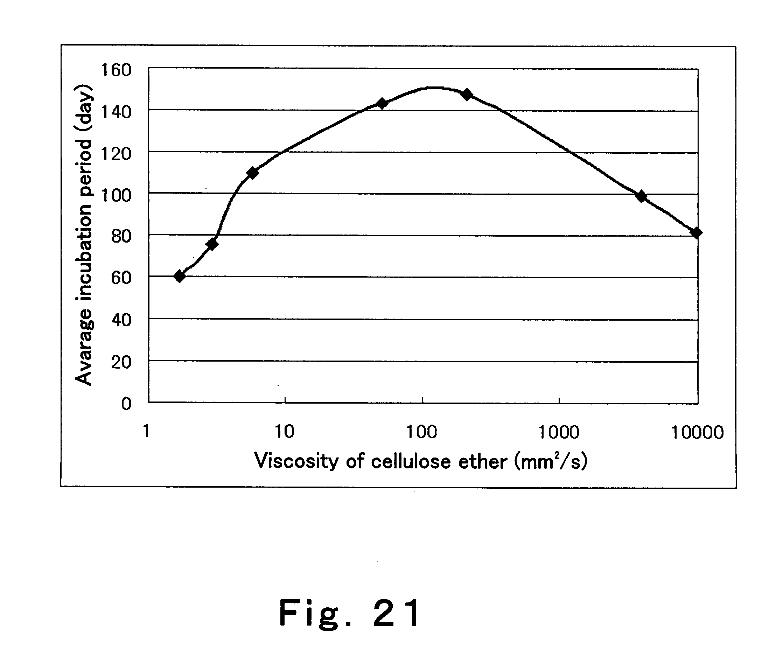

The results are shown in Fig. 21.
The relationship between the viscosity of the cellulose ethers and the therapeutic effect is depicted by a bell-shape curve, accompanied by observation of: elevated therapeutic effect as the viscosity was increased; maximum effect at approximately 100 mm²/s, and reduced therapeutic effect to the contrary as the viscosity was further increased. According to the information provided by the suppliers of the cellulose ethers used as the test samples, the viscosity of 2% by weight aqueous solution at 20°C is 100 mm²/s, and the molecule size (molecular weight) is about 90 kDa.

### Example 21

Taking into account the results of Example 20, hexasaccharide cellulose ethers having a low molecular weight were examined on their therapeutic effects.

### Cellulose Ethers Used in Evaluation

- Methylhexaose (M-6C); methylated hexasaccharide cellulose ether (methoxyl group content: 30%) prepared by adding methyl groups to a hexasaccharide cellulose ether "cellohexaose" (Cat. No., 400406) manufactured by Seikagaku Corporation.
- Hydroxypropyl methylhexaose (HPM-6C); hydroxypropyl methylated hexasaccharide cellulose ether (methoxyl group content: 30%, hydroxypropoxyl group content: 13%) prepared by adding methyl groups and then hydroxypropyl groups to hexasaccharide cellulose ether "cellohexaose" (Cat. No., 400406) manufactured by Seikagaku Corporation.

### Evaluation Method

The experiment for deciding the therapeutic effect for prion disease was carried out in a similar manner to Example 10 by intracerebroventricular administration. Each of the hexasaccharide cellulose ethers was adjusted to have a concentration of 25 mg/mL with a PBS solution, which was filled in a pump for administration into the third ventricle of mouse cerebrum. In the control group, the PBS solution alone was filled into the pump, and similarly administered into the cerebral ventricle. In this method, intracerebral inoculation of 20 µL of the 263K brain homogenate having a concentration of 0.1% (W/V) concentration into the Tg7 mouse was followed by initiation of the intracerebroventricular administration on day 2. Each eight male eight-weeks or older mice (body weight: about 25 g) were used in every group, and the incubation period from intracerebral infection to disease terminal stage was determined. Thus obtained data were analyzed similarly to Example 2.

### Results

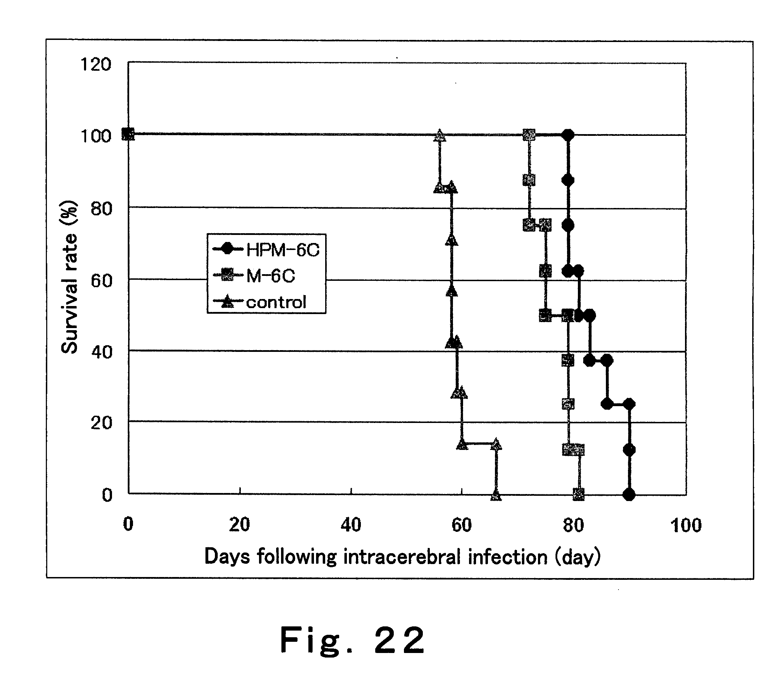

The results are shown in Fig. 22.
The incubation period in the control group, the M-6C-administered group, and the HPM-6C-administered group were 58.2 ± 1.3 days, 76.5 ± 3.5 days, and 83.4 ± 4.7 days, respectively. The HPM-6C group exhibited significantly higher therapeutic effect than the M-6C group (p < 0.05). Also, the M-6C group exhibited significant therapeutic effect, compared to the control group (p < 0.01). These results indicate that the cellulose ethers could exert the therapeutic effect for prion disease even if they have such a small molecule size as hexasaccharide.

### Example 22

Since it is presumed that when HPMC was peripherally administered, it would be gradually absorbed and exhibit the effect of suppressing the replication of prion. Therefore, it is possible that lowering of the molecular weight of HPMC can improve the therapeutic effect by elevating its uptake from the administered site into the blood vessel, and from the blood vessel to each tissue. Therefore, the molecular weight of HPMC was lowered by hydrolysis, and the influences on the therapeutic effect by HPMC were studied *in vivo.*

### Cellulose Ethers Used in Evaluation

- To a 25 mg/mL aqueous solution of hydroxypropyl methylcellulose (HPMC (TC-5RW)) (described in Example 1), hydrochloric acid in a concentration of 0.3%, 4% , or 40% by weight based on the weight of HPMC (TC-5RW) was added. The components were allowed to react at 90°C for 6 hrs while stirring. After the mixture was neutralized with sodium bicarbonate, HPMC deposited by heating to no lower than 80°C was recovered by filtrating through a 3MM filter paper (manufactured by Whatman plc.). HPMC left on the filter paper was washed three times with sterile water heated to no lower than 80°C, and thereafter subjected to freeze-drying.

### Evaluation Method

In a similar manner to Example 2, 15 mg of hydrolyzed HPMC was subcutaneously administered once after a lapse of 10 days following the intracerebral infection. For comparison, 15 mg of nonhydrolyzed HPMC (TC-5RW)-administered group, and compound-nonadministered group (wafer sheet alone) were provided. Each three male and female eight-weeks or older mice (body weight: 20 to 25 g) were used in every group, and the incubation period from intracerebral infection to disease terminal stage was determined. Thus obtained data were analyzed similarly to Example 2.

### Results

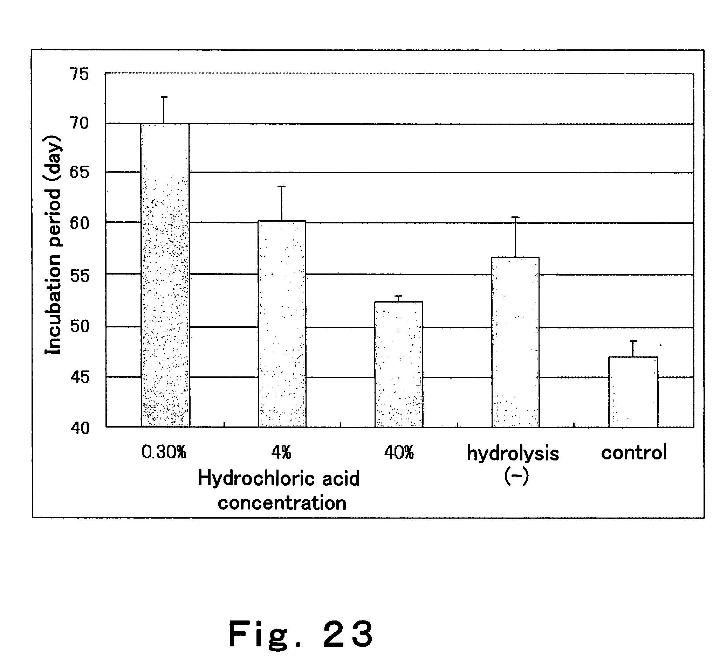

The results are shown in Fig. 23. Contrary to the incubation period being 56.7 ± 4.0 days in the nonhydrolyzed HPMC (TC-5RW)-administered group, the incubation period was 70.0 ± 2.6 days in the hydrolyzed HPMC-administered group prepared with 0.3% hydrochloric acid, suggesting significant improvement (p < 0.01) of the therapeutic effect. However, the incubation period was 60.3 ± 3.4 days in the case of hydrolysis with 4% hydrochloric acid, and 52.3 ± 0.6 days with 40% hydrochloric acid, whereby no significant difference was found. The hydrolysate prepared with 0.3% hydrochloric acid was the most effective, and the effect was evidently reduced as hydrochloric acid concentration was elevated (p < 0.01 in any case), but the therapeutic effect could be apparently observed (p < 0.01 in any case) as compared with the incubation period of 47.0 ± 1.6 days in the nonadministered group (control). This result suggests that the lowering of the molecular weight by hydrolysis improved the therapeutic effect for prion disease, but it is possible that excessive hydrolysis can impair the structure of HPMC responsible for the anti-prion activity.

### Example 23

Compounds that are very similar to the cellulose ethers used in the foregoing Examples were studied as to whether they have similar therapeutic effects for prion disease.
Compounds Used in Evaluation
- Disaccharides; D(+)-cellobiose (manufactured by Nacalai Tesque,Inc.,Cat.No.07511-42), lactose monohydrate(manufactured by Nacalai Tesque, Inc., Cat. No. 20014-52), maltose monohydrate (manufactured by Nacalai Tesque, Inc., Cat. No. 21117-82), sucrose (manufactured by Wako Pure Chemical Industries, Ltd., Cat. No. 198-13525), D (+)-trehalose dihydrate (manufactured by Wako Pure Chemical Industries, Ltd., Cat. No. 203-02252), methylcellobiose (manufactured by Yaizu Suisankagaku Industry Co., Ltd.), respectively.
- Hexasaccharides; cellohexaose (manufactured by Seikagaku Corporation, Cat. No. 400406), isomaltohexaose (manufactured by Seikagaku Corporation, Cat. No. 400481), maltohexaose (manufactured by Seikagaku Corporation, Cat. No. 400516), laminarihexaose (manufactured by Seikagaku Corporation, Cat. No. 400493), hexa-N-acetylchitohexaose (manufactured by Seikagaku Corporation, Cat. No. 400427), chitosanhexamer (manufactured by Seikagaku Corporation, Cat. No. 400436), respectively.
- Cyclodextrins; a-cyclodextrin (manufactured by JUNSEI CHEMICAL CO., LTD., Cat. No. 33730-0410), ß-cyclodextrin (manufactured by JUNSEI CHEMICAL CO., LTD., Cat. No. 33735-0410), y-cyclodextrin (manufactured by JUNSEI CHEMICAL CO., LTD., Cat. No. 33740-0410), monoacetyl ß-cyclodextrin (manufactured by JUNSEI CHEMICAL CO., LTD., Cat. No. 77016-1610), heptakis (2,3,6-tri-O-benzoyl) ß-cyclodextrin (manufactured by Sigma Corporation, Cat. No. T3196), heptakis (2,3,6-tri-acetyl) β-cyclodextrin (manufactured by Sigma Corporation, Cat. No. T3446), respectively.
- Cyclodextrins having a methyl group or a hydroxypropyl group; methyl ß-cyclodextrin (manufactured by ALDRICH Co. (Cat. No. 332615)), 2-hydroxypropyl a-cyclodextrin (manufactured by ALDRICH Co., Cat. No. 390690), 2-hydroxypropyl ß-cyclodextrin (manufactured by ALDRICH Co., Cat. No. 389145, degree of substitution: 1.0 mol), 2-hydroxypropyl ß-cyclodextrin (manufactured by ALDRICH Co., Cat. No. 332607, degree of substitution: 0.8 mol), 2-hydroxypropyl β-cyclodextrin (manufactured by ALDRICH Co., Cat. No. 332593, degree of substitution: 0.6 mol), 2-hydroxypropyl y-cyclodextrin (manufactured by Sigma Corporation, Cat. No. H125), heptakis(2,3,6-tri-O-methyl) ß-cyclodextrin (manufactured by Sigma Corporation(Cat. No. H4645)), heptakis(2,6-di-O-methyl) β-cyclodextrin (manufactured by Sigma Corporation(Cat. No. H0513)), respectively.
- Chitin or Chitosans; chitosan-10 (Wako Pure Chemical Industries, Ltd., Cat. No. 039-16122), chitosan-100 (Wako Pure Chemical Industries, Ltd., Cat. No. 032-16092), chitosan-1000 (Wako Pure Chemical Industries, Ltd., Cat. No. 039-14422), chitin (Wako Pure Chemical Industries, Ltd., Cat. No. 034-13632, respectively.
- Cellulose; cellulose (manufactured by Sigma Corporation, Cat. No. S3504).
- Chitin or Chitosan having a hydroxypropyl group; hydroxypropyl chitosan-100 (manufactured by Yaizu Suisankagaku Industry Co., Ltd.), hydroxypropyl chitin (manufactured by Yaizu Suisankagaku Industry Co., Ltd.), respectively.
- Long chain alkyl group-containing celluloses having a methyl group and a hydroxypropyl group; long chain alkyl group-containing hydroxypropyl methylcellulose (SG-60M) (Daido Chemical Corporation manufactured by , "SANGELOSE(R)-60M" (trade name)), long chain alkyl group-containing hydroxypropyl methylcellulose (SG-60L) (Daido Chemical Corporation manufactured by , "SANGELOSE(R)-60L" (trade name)), respectively.
- Cellulose having a methyl group; methyl cellulose described in Example 8 (MC (SM-4)).
- Cellulose having a hydroxypropyl group; insoluble hydroxypropyl cellulose having a low degree of substitution (L-HPC) (manufactured by Shin-Etsu Chemical Co., "L-HPC (LH-31)" (trade name), hydroxypropoxyl group content: 11%).
- Celluloses having a carboxyl group and a methyl group; carboxymethyl cellulose (CMC) (manufactured by Sigma Corporation, Cat. No. C5678), carboxymethyl cellulose (CMC (NS300))) (manufactured by Gotoku Chemical Co., Ltd., "NS-300" (trade name)), carboxymethyl cellulose sodium (CMC-Na) (manufactured by Gotoku Chemical Co., Ltd., "T. P. T" (trade name)), respectively.
- Cellulose having a methyl group and a hydroxyethyl group; hydroxyethyl methylcellulose (MHEC) (manufactured by ALDRICH Co., Cat. No. 435015).
- Polysaccharides having a hydroxyethyl group; hydroxyethyl cellulose (HEC) (manufactured by FLUKA, Cat. No. 54290), hydroxyethyl starch (manufactured by Sigma Corporation, Cat. No. H6382), fluid infusion formulation containing hydroxyethyl starch 70000 (manufactured by Kyorin Pharmaceutical. Co, , "Hespander" (trade name)), respectively.
- Medical polysaccharides having a immunostimulating action; lentinan (trade name) (manufactured by Ajinomoto Co., Inc.), Picibanil (trade name) (manufactured by Chugai Pharmaceutical Co. , Ltd.), Bestatin (trade name) (manufactured by Nippon Kayaku Co., Ltd.), Sonifilan (trade name) (manufactured by Kaken Pharmaceutical Co., Ltd.), respectively.
   Evaluation Method
   To the Tg7 mouse infected with prion by intracerebral inoculation of 20 µL of a brain homogenate of 263K strain having a concentration of 1% (W/V), each compound in an amount of no more than 100 mg and not accompanied by toxicity was subcutaneously or interperitoneally administered within 5 days following the infection. The subcutaneous administration was carried out in the form of a powder, while the intraperitoneal administration was carried out in the form of an aqueous solution. Each three or more male and female eight-weeks or older mice (body weight: 20 to 25 g) were used in every group, and the incubation period from intracerebral infection to disease terminal stage was determined. Thus obtained data were analyzed similarly to Example 2.

### Results

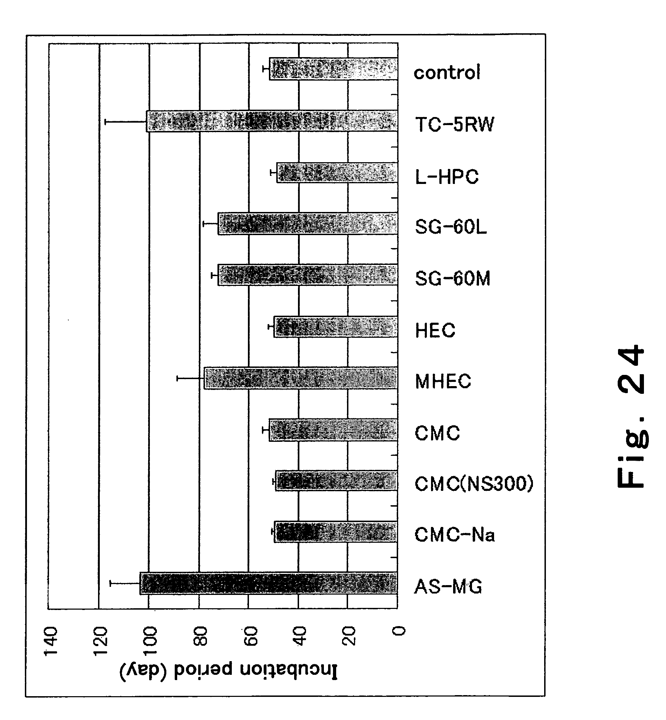

An example of the results obtained by subcutaneous administration of 50 mg of each compound into the mouse dorsal region on day 4 following the intracerebral infection are shown in Fig. 24. In the figure, the HPMC (AS-MG) group and the HPMC (TC-5RW) group are shown as positive controls, while the wafer sheet alone-administered group is shown as a negative control. HPMCs (SG-60M, SG-60L) having a long chain alkyl group that are modified forms of hydroxypropyl methylcellulose, and hydroxyethyl methylcellulose (MHEC) were effective, but carboxymethyl celluloses (CMC-Na, CMC (NS300), CMC), hydroxyethyl cellulose (HEC), and insoluble hydroxypropyl cellulose (L-HPC) having a low degree of substitution were not effective.
Each of the various compounds was tested by subcutaneous administration or intraperitoneal administration in mice intracranially infected with 263K prion, and the therapeutic effect for prion disease was evaluated by rating on a three-point scale, i.e., C; not effective, B; effective, and A; extremely effective, and the results are summarized in Table 1.

**Table 1**

| **Compound group** | **Therapeutic effect for prion disease** |
|---|---|
| Disaccharides | C |
| Hexasaccharides | C |
| Cyclodextrins | C |
| Cyclodextrins having a methyl group or a hydroxypropyl group | C |
| Chitin or Chitosans | C |
| Chitin or Chitosan having a hydroxypropyl group | C |
| Starch | C |
| Starch having a hydroxyethyl group | C |
| Celluloses | C |
| Long chain alkyl group celluloses having a methyl group and a hydroxypropyl group | A |
| Celluloses having a methyl group | A |
| Celluloses having a hydroxypropyl group | B |
| Celluloses having a carboxyl group and a methyl group | C |
| Celluloses having a methyl group and a hydroxyethyl group | A |
| Polysaccharides having a hydroxyethyl group | C |
| Medical polysaccharides having a immunostimulating action | C |

The cellulose having a methyl group, the cellulose having a methyl group and a hydroxypropyl group, the cellulose having a methyl group and a hydroxyethyl group, and the cellulose having a hydroxypropyl group exhibited the therapeutic effect for prion disease.

### Example 24

In the foregoing Examples, it was proven that cellulose ethers exhibit therapeutic effects in individuals suffering from prion diseases to prevent the onset or to retard the onset. Therefore, histopathological study was performed to determine as to whether the therapeutic effect reflects the findings in the brain that is the main site of the lesion in prion diseases.

### Cellulose Ethers Used in Evaluation

- Hydroxypropyl methylcellulose (HPMC (TC-5RW)); described in Example 1.

### Evaluation Method

Evaluation was made in a similar manner to Example 15. Female 5- to 6-weeks old syrian hamsters (6 animals, available from Japan SLC, Inc., body weight: approximately 90 g/animal) were infected with prion by inoculating 40 µL of a 1% (W/V) 263K brain homogenate in the brain. After a lapse of 8 days following the intracerebral infection, 150 mg of HPMC (TC-5RW) was subcutaneously administered to the dorsal region of the syrian hamster. As a control group (4 animals), HPMC-nonadministered group (wafer sheet alone) was provided. On day 64 and day 80 (in the control group, disease terminal stage) following the intracerebral infection, two hamsters in the HPMC-administered group and in the control group were euthanized, respectively. Also, on day 136 following the intracerebral infection (in the HPMC-administered group, disease terminal stage), the remaining two hamsters in the HPMC-administered group were euthanized. Brain was removed from each hamster, and cut into a sagittal section along the midline. The section was immersed in a buffered formalin for 1 month or longer to permit fixation. The fixed brain was immersed in 98% formic acid solution for one hour at room temperature, whereby infectivity was reduced, followed by embedding in paraffin, and slicing to produce a 0.4 µm tissue section. The tissue section was subjected to immunostaining with the antibody against prion protein for detecting accumulation of abnormal prion protein. Subsequently, serial tissue sections were subjected to immunostaining with the antibody against glial fibrillary acidic protein (GFAP) for detecting astroglia that proliferates at neurodegeneration sites. The immunostaining was carried out as follows according to the method of Doh-ura *et al*., (Katsumi Doh-ura et al., Journal of Virology, 2004, Vol. 78, pp. 4999-5006).
The tissue section subjected to a treatment for blocking endogenous peroxidase was immersed in 1 mM hydrochloric acid, and the treatment was allowed at 121°C for 10 min. This treatment is omitted for the GFAP immunostaining. After the tissue section was washed with distilled water three times, with 50 mM Tris hydrochloric acid (pH 7.5), 0.1% Triton X-100 twice, and with 50 mM Tris hydrochloric acid (pH 7.5) once, an anti-prion protein antibody (manufactured by Immuno-Biological Laboratories Co., Ltd., 18631 (item number), x 200 diluted in 5% normal goat serum-added PBS) or an anti-GFAP antibody (manufactured by DakoCytomation A/S, M0761 (item number), x 5,000 diluted in 0.1% bovine serum albumin-added PBS) was allowed to react at 4°C overnight. After washing with 50 mM Tris hydrochloric acid (pH 7.5), and 0.1% Triton X-100 twice, and with 50 mM Tris hydrochloric acid (pH 7.5) once, EnVision + System-HRP Labelled Polymer solution (manufactured by DakoCytomation A/S, K4002 or K4006 (item number)) was added dropwise on the tissue section, and allowed to react at room temperature for 30 min. After washing with 50 mM Tris hydrochloric acid (pH 7.5), and 0.1% Triton X-100 twice, and with 50 mM Tris hydrochloric acid (pH 7.5) once, the antigen-antibody complex was visualized by way of a peroxidase reaction using diaminobenzidine (DAB).

### Results

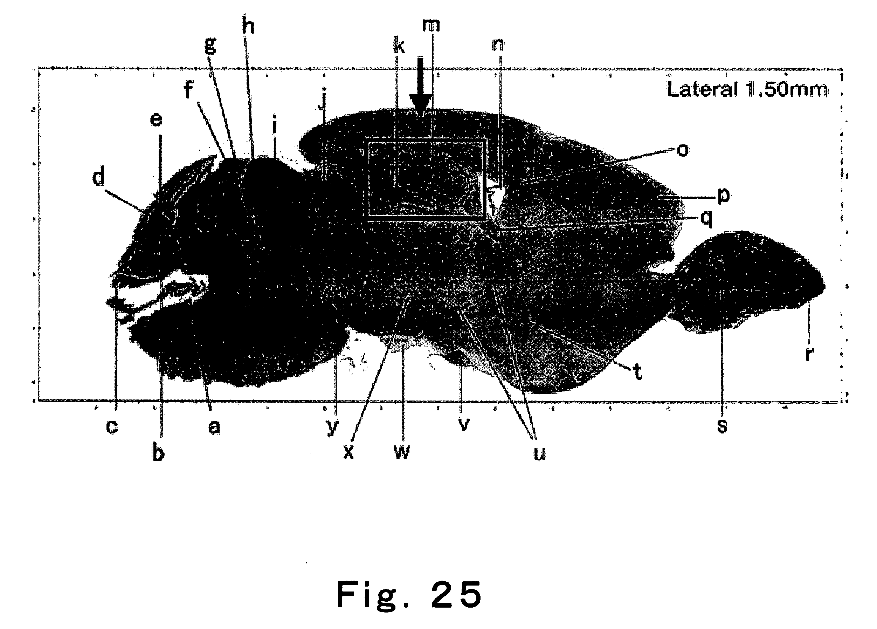

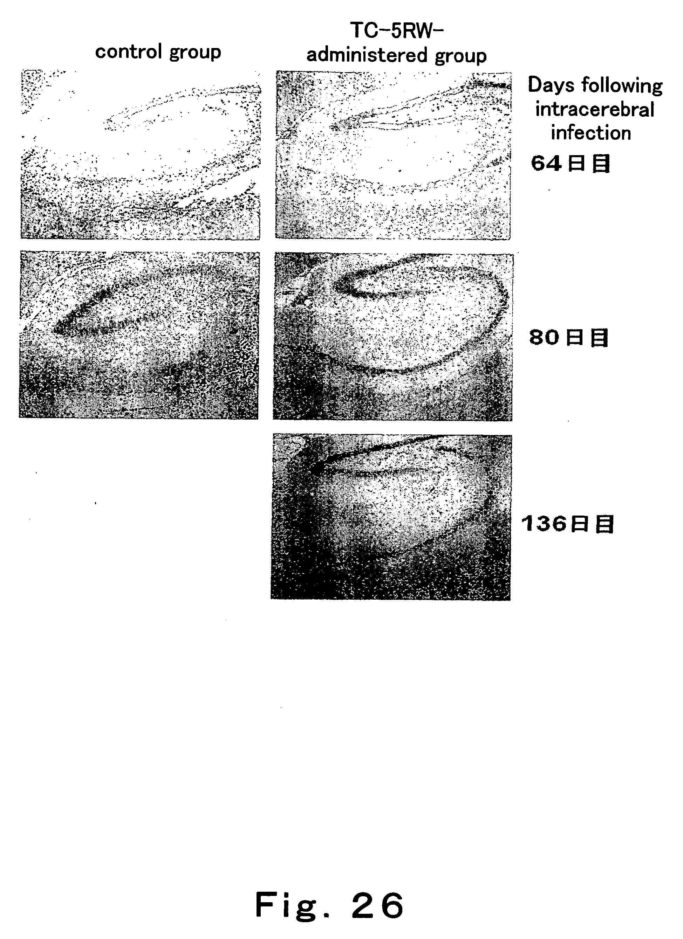

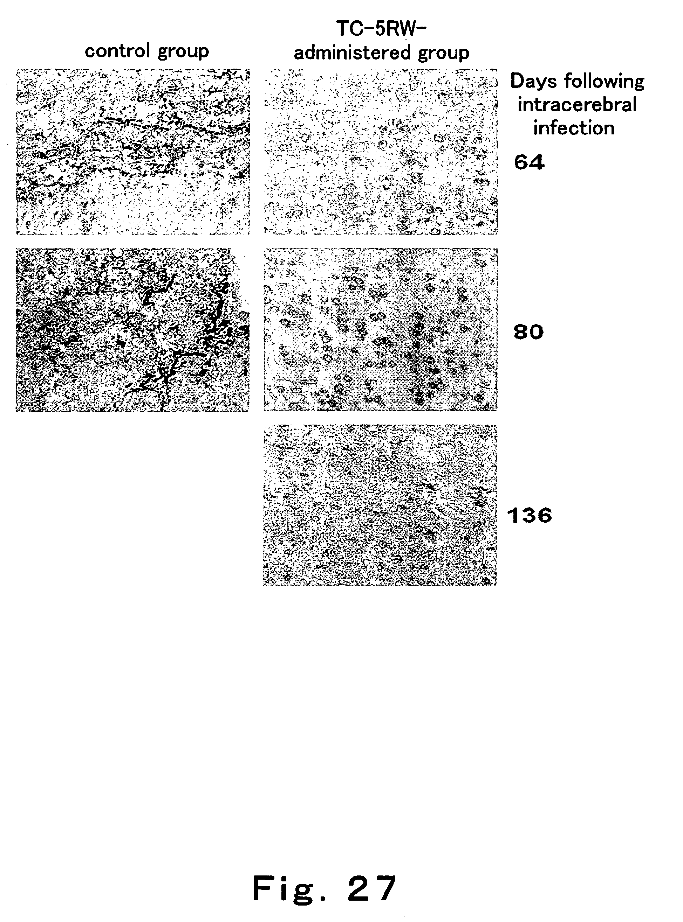

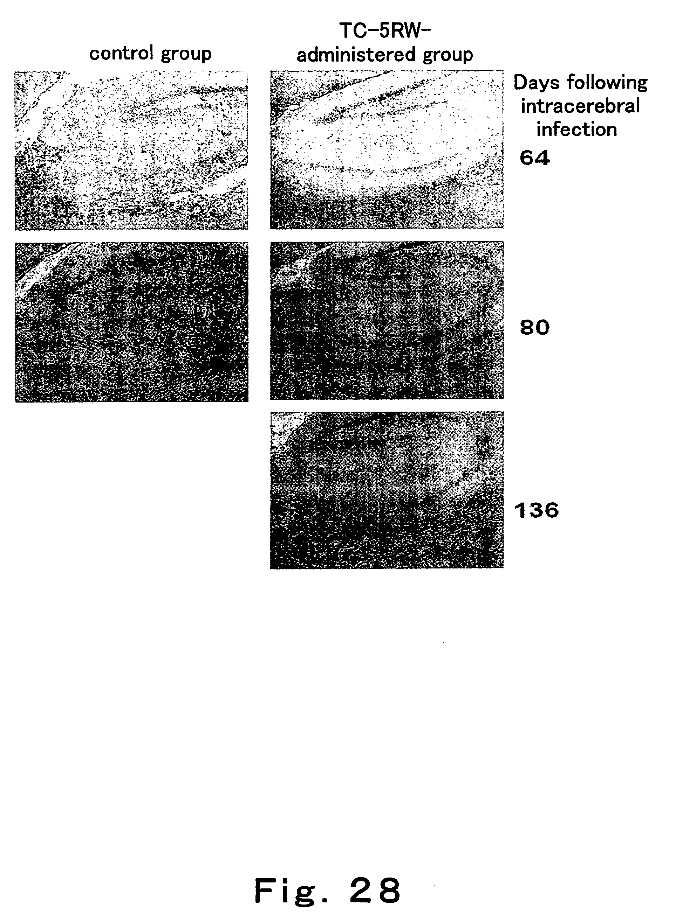

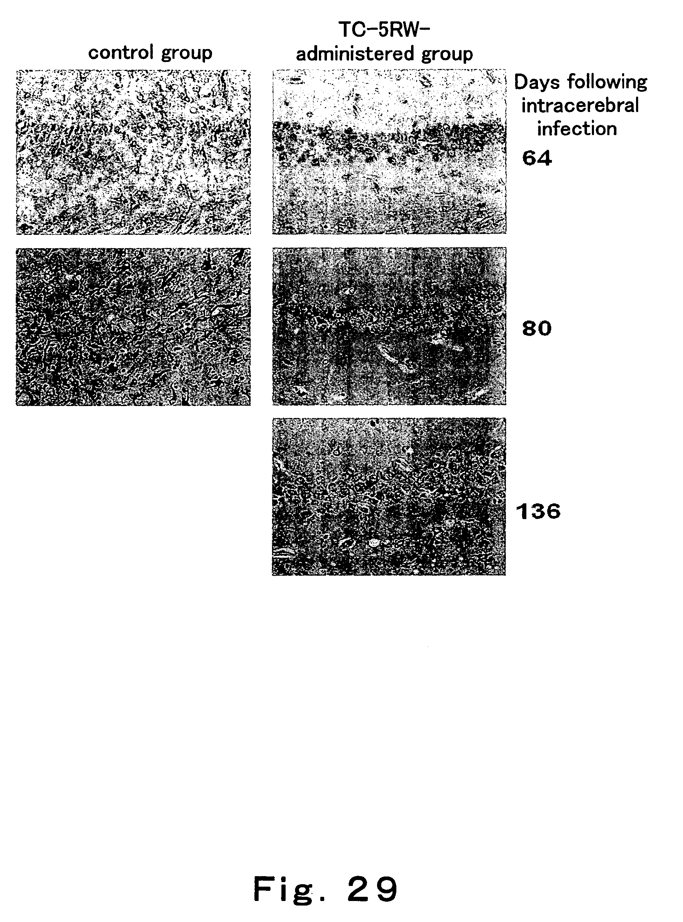

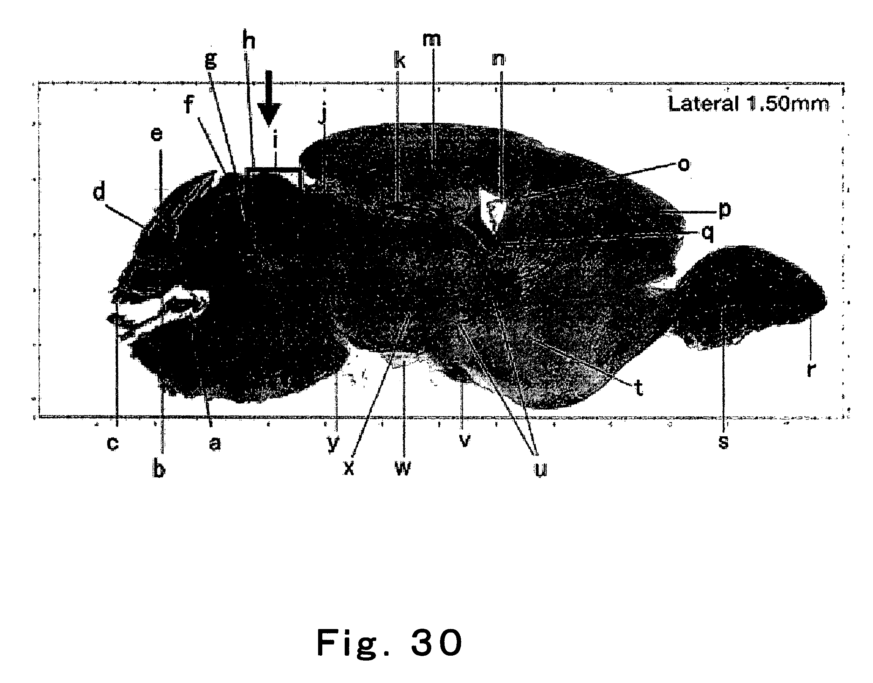

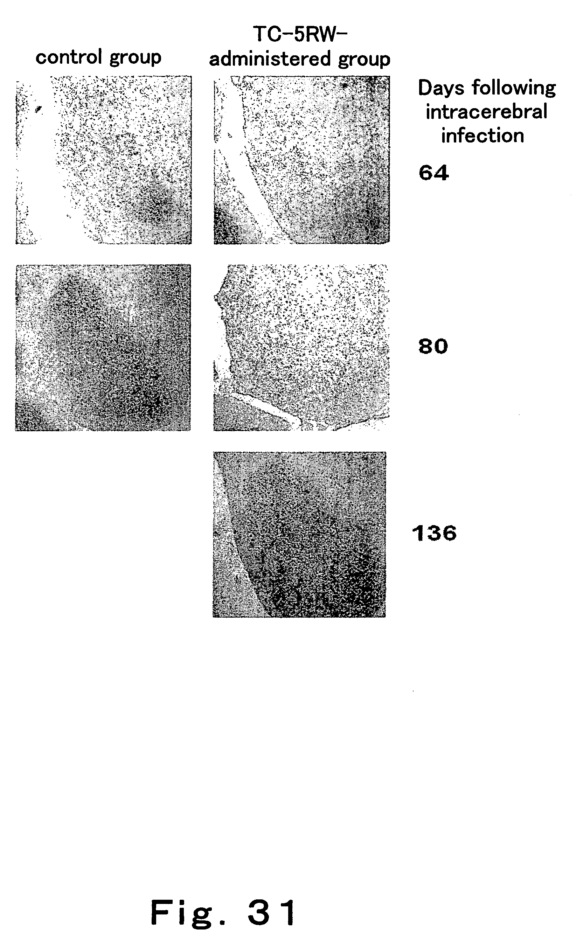

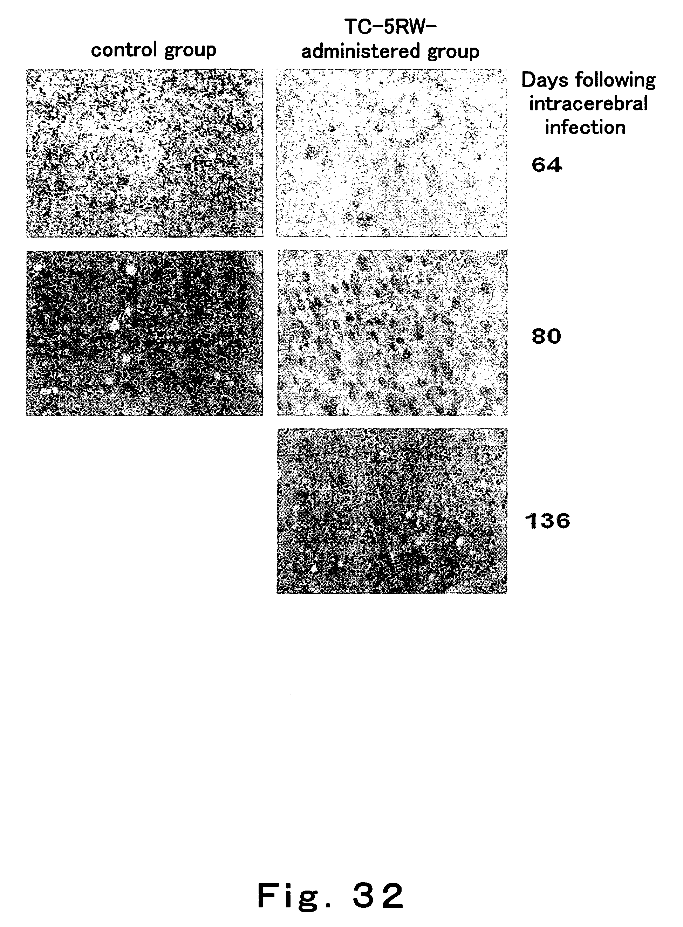

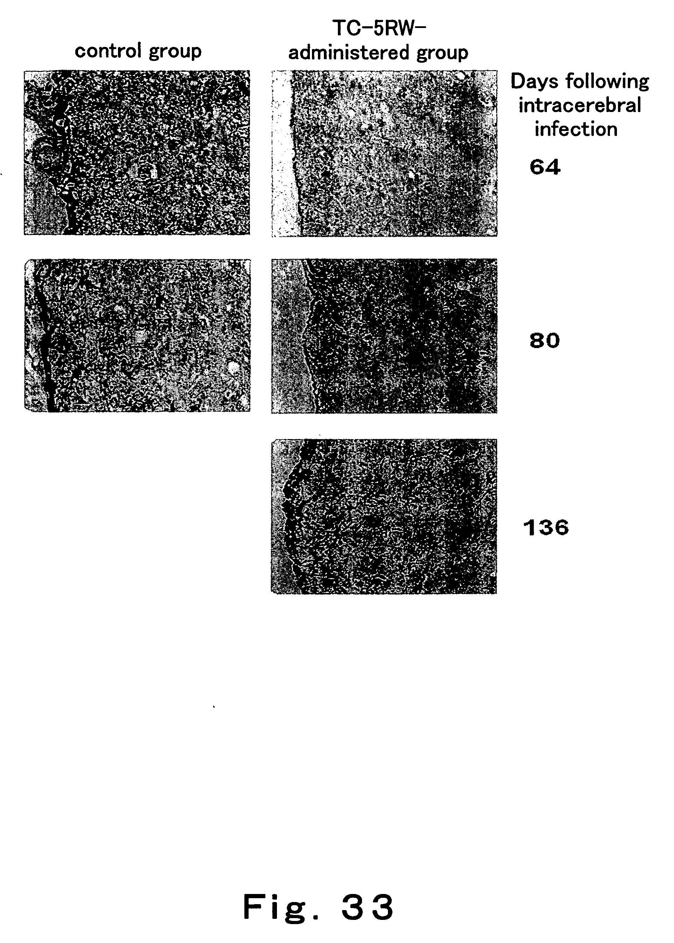

In the control group, among the sites where accumulation of abnormal prion proteins and neurodegeneration were remarkable, photomicrography demonstrating the results of anatomical localization in the vicinity of hippocampus and inferior colliculus (modified enlarged view of sagittal section specimen in "Brain Experiment Manual Viewed in Mouse Color Atlas and Photograph" Mamoru Kurokawa ed., Yodosha Co., Ltd., 2005, p. 52) are shown in Fig. 25 and Fig. 30, respectively. In the figures, the reference signs a to y denote the sites in the vicinity of the tip of each lead line to which each reference sign was added, and the name of the site denoted by each reference sign is as in the following.
a; Fourth ventricle
b; Cerebellum VIII lobula
c; Cerebellum IX lobula
d; Cerebellar corpus medullare
e; Cerebellum VII lobula
f; Cerebellum IV · V lobula
g; Cerebellum III lobula
h; Cerebellum II lobula
i; Inferior colliculus
j; Superior colliculus
k; Dentate gyrus
m; Ammon's horn
n; Dorsum third ventricle
o; Callosum
p; Frontal association area
q; Fimbria
r; External plexiform layer
s; Olfactory bulb granule cell layer
t; Anterior commissure
u; Fasciculus mamillothalamicus
v; Optic tract
w; Corpus mammillare
x; Hypothalamus
y; Pontine nucleus
A photomicrography of a low magnification view revealing accumulation of abnormal prion protein in the vicinity of hippocampus is shown in Fig. 26, and a photomicrography of a high magnification view of the white matter region intervened between the hippocampus and the cerebral cortex positioned at one part of the aforementioned site is shown in Fig. 27. The photographs were taken so as to view from the direction as indicated by the arrow in Fig. 25. In the control group, accumulation of abnormal prion protein was already found in the white matter region intervened between the hippocampus and the cerebral cortex on day 64 following the intracerebral infection, and on day 80 following the intracerebral infection that fell into the terminal stage of the disease, the accumulation of abnormal prion protein in the same region was increased. To the contrary, in the TC-5RW-administered group, accumulation of abnormal prion protein was not found on day 64 and day 80 following the intracerebral infection. Furthermore, in the TC-5RW-administered group, the accumulation of abnormal prion protein was very slightly observed on day 136 following the intracerebral infection that fell into the terminal stage of the disease.
Astroglial cells visualized by GFAP immunostaining are present in the white matter also in normal brain, and when neurodegeneration occurs, the number of GFAP-positive astroglial cells is increased in the gray matter and the white matter at the degenerative site, accompanied by activation. Therefore, GFAP immunostaining is a simple process used for evaluation of neurodegeneration. A photomicrography of GFAP immunostaining at the same site as that observed in Fig. 26 is shown in Fig. 28. Also, a photomicrography of a high magnification view of a field including the pyramidal cell layer of hippocampus that is a part of the same site is shown in Fig. 29. In the control group, extensive proliferation of astroglial cells was already observed in the area from the white matter region intervened between the hippocampus and the cerebral cortex over the hippocampus on day 64 following the intracerebral infection, and on day 80 following the intracerebral infection that fell into the terminal stage of the disease, when degenerative changes were highly exacerbated with massive neuronal cell loss in the pyramidal cell layer. To the contrary, in the TC-5RW-administered group, proliferation of astroglial cells was not found on day 64 and day 80 following the intracerebral infection. Furthermore, in the TC-5RW-administered group, the proliferation of astroglial cells was very slightly observed in the area from the white matter region over the hippocampus on day 136 following the intracerebral infection that fell into the terminal stage of the disease.
The findings as described above were almost similar also in the inferior colliculus. A photomicrography of a low magnification view revealing accumulation of abnormal prion protein in the inferior colliculus is shown in Fig. 31, and a photomicrography of a high magnification view is shown in Fig. 32. The photographs were taken so as to view from the direction as indicated by the arrow in Fig. 30. In the control group, accumulation of abnormal prion protein was already found in the inferior colliculus on day 64 following the intracerebral infection, and on day 80 following the intracerebral infection that fell into the terminal stage of the disease, it was somewhat increased. To the contrary, in the TC-5RW-administered group, accumulation of abnormal prion protein was not found on day 64 and day 80 following the intracerebral infection. Furthermore, in the TC-5RW-administered group, the accumulation of abnormal prion protein was observed on day 136 following the intracerebral infection that fell into the terminal stage of the disease, but it was somewhat less in amount as compared to that of the terminal stage of the disease in the control group. A photomicrography of a high magnification view presenting GFAP immunostaining at the same site as observed in Fig. 31 is shown in Fig. 33. In the control group, proliferation of astroglial cells was already observed in the inferior colliculus on day 64 following the intracerebral infection. By contrast, in the TC-5RW-administered group, proliferation of astroglial cells was not found on day 64 and day 80 following the intracerebral infection, while in the TC-5RW-administered group, proliferation of astroglial cells was observed on day 136 following the intracerebral infection that fell into the terminal stage of the disease.
From the results in the foregoing, it was verified that HPMC exerts the therapeutic effect by suppressing accumulation of abnormal prion protein and neurodegeneration in the brain that is the main site of the lesion in prion diseases.

### Example 25

From the results of Example 9 and Example 10, it is supposed that subcutaneously administered HPMC is absorbed into a blood vessel, transferred from the blood to cerebral parenchyma, and exerts the therapeutic effect for prion diseases. Furthermore, it is presumed from the results of Example 3 and Example 5 that singly administered HPMC sufficiently remains effective, whereby a long period of time may be necessary for degradation and excretion of HPMC. In order to verify these possibilities, HPMC was subcutaneously administered, and anti-prion activity in the blood was determined.

### Cellulose Ethers Used in Evaluation

- Hydroxypropyl methylcellulose (HPMC (TC-5RW)); described in Example 1.

### Evaluation Method

Fifteen 5- to 6-weeks old female Syrian hamsters (body weight: approximately 90 g/animal) were subcutaneously administered with 400 mg of HPMC (TC-5RW) in the dorsal region (administration was carried out by wrapping in a wafer sheet, and embedding subcutaneously in a similar manner to Example 2). As a control, six animals not administered with HPMC (wafer sheet alone) were provided. After a lapse of 1.5 weeks, after a lapse of 3 weeks, and after a lapse of 4. 5 weeks following the administration of HPMC, respectively, whole blood was collected from 5 animals in the HPMC-administered group, and 2 animals in the nonadministered group, followed by separation of the serum. The presence of anti-prion activity in the resulting serum was studied by an in vitro evaluation method described in Example 1. ScN2a cells were seeded in a cell number corresponding to 10% of confluence in a 24-well cell culture dish with 1 mL of the culture medium, to which 50 µL of the collected hamster serum was added, and the cells were cultured. When the cells reached to confluence, abnormal prion protein (prion) was prepared from the cell lysate according to the method of Example 1, and analyzed by Western blotting.

### Results

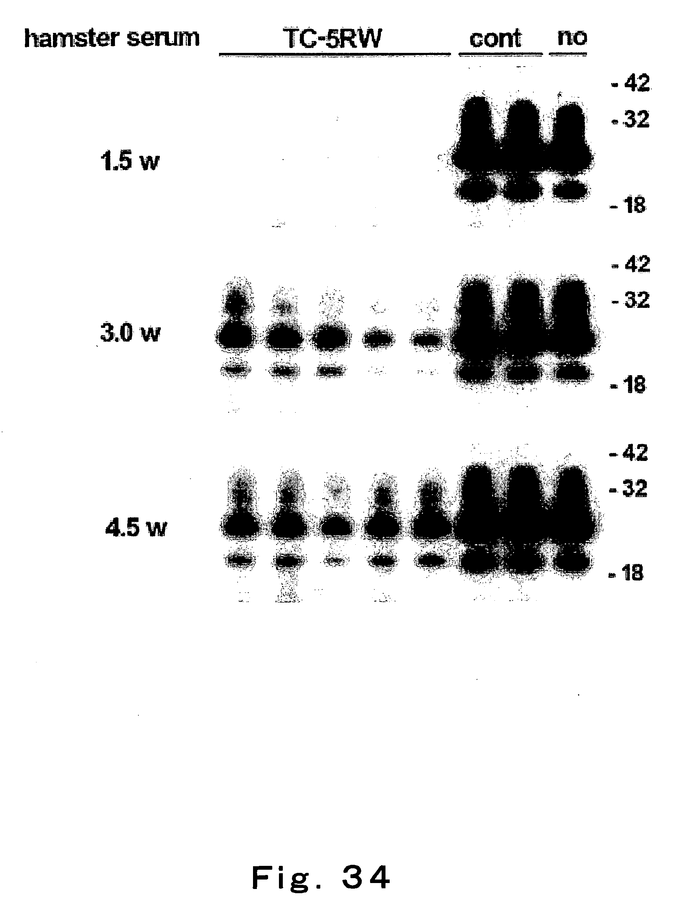

The results are shown in Fig. 34. In the figure, abnormal prion protein signals derived from the ScN2a cells treated with individual hamster sera are indicated, "TC-5RW" for the HPMC (TC-5RW)-administered group, and "cont" for the nonadministered group. The denotation "no" is for the control group without treatment with a hamster serum.
In the study, the hamster serum after a lapse of 1.5 weeks following the administration of HPMC (TC-5RW) exhibited the most potent anti-prion activity. Although the anti-prion activity is decreased after a lapse of 3 weeks, almost the same or just a little lower level of anti-prion activity was present in the serum also after a lapse of 4.5 weeks. This result suggests that the anti-prion activity derived from HPMC is present in the serum for a long period of time even in the case of single administration.

### Example 26

Sensitivity to temperature was studied on anti-prion active factors in the serum.

### Evaluation Method

The hamster serum after a lapse of 1.5 weeks following the administration of HPMC (TC-5RW) in Example 25 (sample on the first lane of "TC-5RW" in Fig. 34), and the control hamster serum collected at the same time point (sample on the first lane of "cont" in Fig. 34) were used.
Each 10µL ot the HPMC (TC-5RW)-administered hamster serum, and the HPMC (TC-5RW)-added control serum (control hamster serum added with HPMC (TC-5RW) to give a final concentration of 4.2 mg/mL) was treated at 35ºC, 50ºC, 65ºC, 80ºC, and 95ºC for 10 min, and the supernatant was obtained by centrifugation (13,000 x g, 5 min) after cooling. ScN2a cells were seeded in a cell number corresponding to 10% of confluence in a 24-well cell culture dish with 1 mL of the culture medium, to which the obtained supernatant was added, and the cells were cultured. When the cells reached to confluence, abnormal prion protein (prion) was prepared from the cell lysate according to the method of Example 1, and analyzed by Western blotting.
In addition, a Montage Albumin Deplete Kit (manufactured by Millipore Corporation) was used, and influences of the treatment with temperatures on the samples of the hamster serum from which albumin was eliminated were similarly studied.

### Results

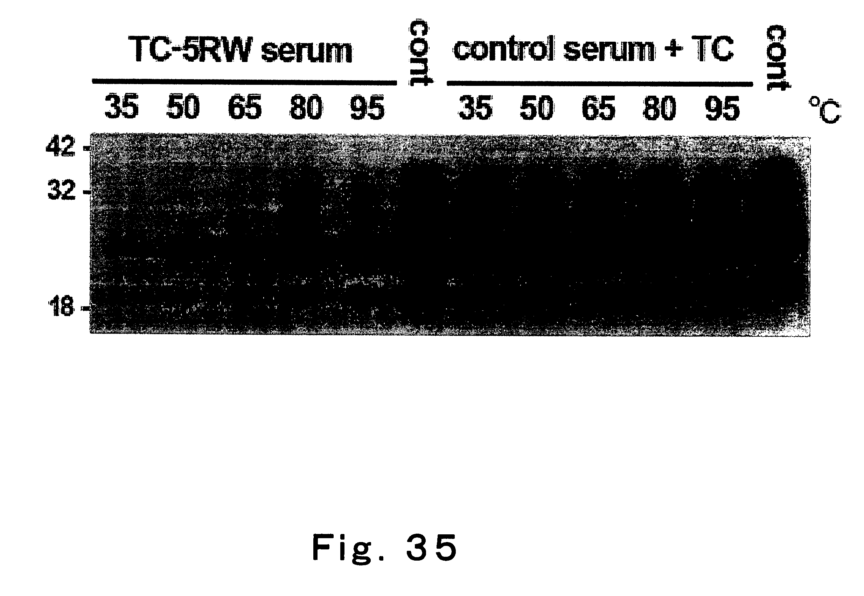

The results when albumin was not eliminated are shown in Fig. 35. The denotation "cont" represents the abnormal prion protein in ScN2a cells to which nothing is added. Although the anti-prion activity of the HPMC (TC-5RW)-administered hamster serum was decreased to some extent by the treatment at no lower than 80ºC, similar alteration was not revealed in the case of the HPMC (TC-5RW)-added control hamster serum. Since HPMC (TC-5RW) is resistant to degradative modification by the treatment at such a high temperature as 80ºC or 95ºC, this result may suggest that the anti-prion active factor in the HPMC (TC-5RW)-administered hamster serum is distinct from HPMC (TC-5RW) and HPMC (TC-5RW) like material. However, a large quantity of proteins such as albumin included in the serum may be thermally denatured by the treatment at no lower than 80ºC to form giant precipitates, whereby it is presumed that a part of the anti-prion active factor may be trapped in the precipitates to lead to lowering of the activity in the supernatant.

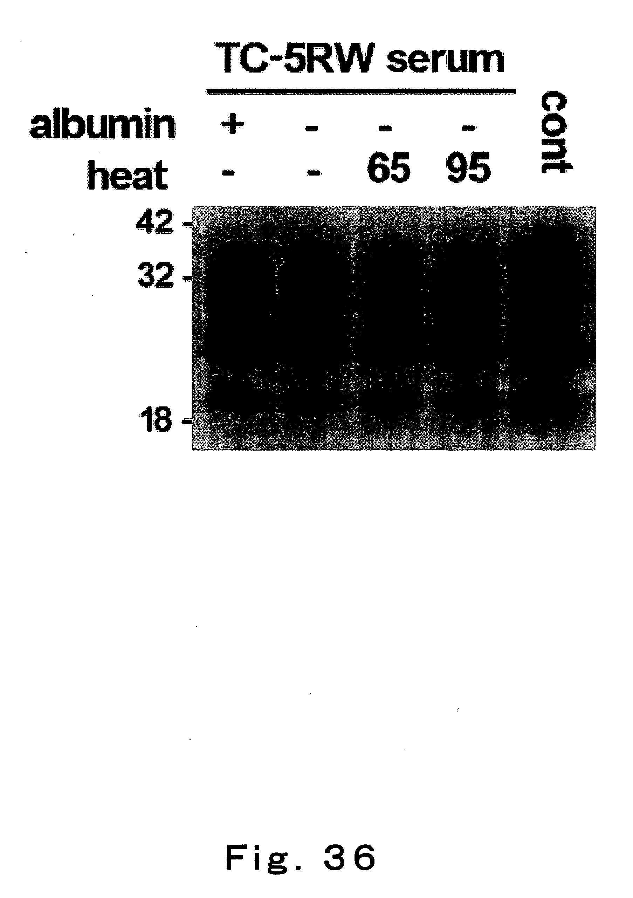

The results when albumin was eliminated are shown in Fig. 36. In the figure, the denotation "cont" represents the abnormal prion protein in ScN2a cells to which nothing is added; "albumin +" represents the case in which the HPMC (TC-5RW) -administered hamster serum without elimination of albumin was used; and "albumin -" represents the case in which the HPMC (TC-5RW) -administered hamster serum from which albumin was eliminated was used. Notable difference was not found among the anti-prion activities for: untreated with temperature ("heat -"), treated at 65ºC ("heat 65"), treated at 95ºC ("heat 95"). From this result, it is appreciated that the anti-prion activity in the HPMC (TC-5RW)-administered hamster serum is heat-resistant, similarly to HPMC (TC-5RW).

### Example 27

Next, molecule size (molecular weight) of the anti-prion active factor in the TC-5RW-administered hamster serum was estimated using a filter (ultrafiltration membrane) having a molecular sieve effect.

### Evaluation Method

Each 10 µL of the HPMC (TC-5RW)-administered hamster serum and the HPMC (TC-5RW)-added control serum (control hamster serum added with HPMC (TC-5RW) to give a final concentration of 20 mg/mL) was subjected to filtration, respectively, with a BIOMAX-30 filter (BIOMAX-30 Filter Unit) (manufactured by Millipore Corporation, "UFC 3BTK00" (model number), cut-off: molecular weight of about 30,000 Da), or BIOMAX-100 filter (BIOMAX-100 Filter Unit) (manufactured by Millipore Corporation, "UFC 3BHK00" (model number), cut-off: molecular weight of about 100,000 Da), and the filtrate ( "filtrate" ) and the retentate which could not be filtered off ("retentate") were fractionated. ScN2a cells were seeded in a cell number corresponding to 10% of confluence in a 24-well cell culture dish with 1 mL of the culture medium, to which the fractionated filtrate or retentate was added, and the cells were cultured. When the cells reached to confluence, abnormal prion protein (prion) was prepared from the cell lysate according to the method of Example 1, and analyzed by Western blotting.

### Results

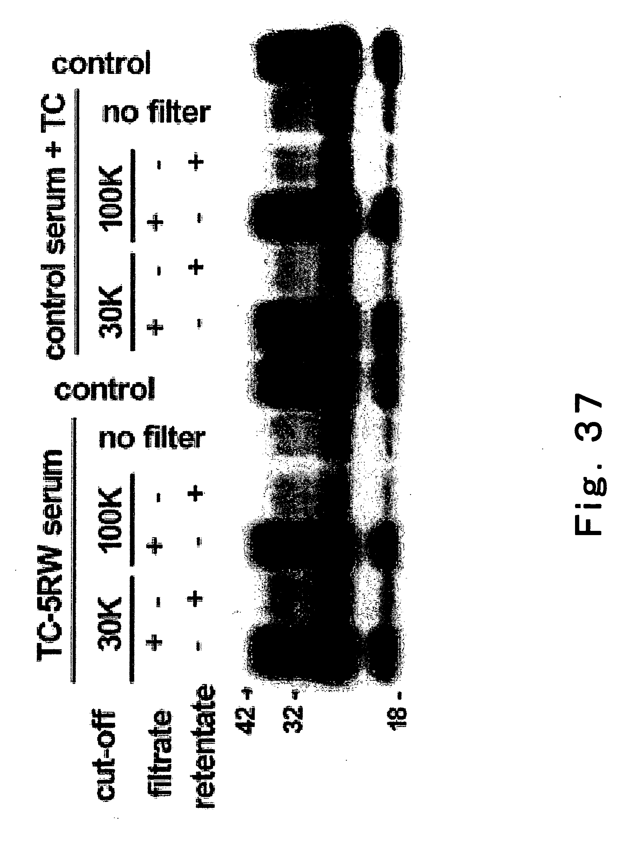

The results are shown in Fig. 37. In the figure, "control" represents the abnormal prion protein in untreated ScN2a cells; "no filter" represents the case in which HPMC (TC-5RW)-administered hamster serum prior to filtration, or HPMC (TC-5RW) -added control serum was used; "30K" represents the case of treatment with the BIOMAX-30 filter; "100K" represents the case of treatment with the BIOMAX-100 filter; "filtrate +" represents the case in which the filtrate was used; and "retentate +" represents the case in which the retentate which could not be filtered off was used. The anti-prion active factor in the HPMC (TC-5RW)-administered hamster serum exhibited a behavior similar to that of the control hamster serum plus HPMC (TC-5RW). It was revealed that the factor did not pass through the filter with cut-off of the molecular weight being about 100,000 Da.

### Example 28

Sensitivity to a treatment with degradative enzymes was studied on anti-prion active factors in the HPMC-administered hamster serum.

### Evaluation Method

After each of 10 µL of the HPMC (TC-5RW)-administered hamster serum, and 10 µL of the HPMC (TC-5RW)-added control serum (control hamster serum added with HPMC (TC-5RW) to give a final concentration of 20 mg/mL) was subjected to a treatment of eliminating albumin with a Montage Albumin Deplete Kit (manufactured by Millipore Corporation), thereto were added α-amylase (manufactured by Sigma Corporation, "A3176" (item number)) to give a final concentration of 0.1 mg/mL, amyloglucosidase (manufactured by Sigma Corporation, "A9228" (item number)) to give a final concentration of 0.1 mg/mL, lipase (manufactured by Wako Pure Chemical Industries, Ltd., "548-00212" (item number)) to give a final concentration of 0.1 mg/mL, deoxyribonuclease I (manufactured by Sigma Corporation, "DN25" (item number)) to give a final concentration of 0.1 mg/mL, and ribonuclease A (manufactured by Sigma Corporation, "R4875" (item number)) to give a final concentration of 0.05 mg/mL, and the reaction was allowed at 37ºC for 1 hour. Thereafter, proteinase K was added to the mixture to give a final concentration of 0.1 mg/mL, and allowed to further react at 37ºC for 1 hour. After treating at 95ºC for 5 min, the reaction mixture was cooled, and centrifuged (13, 000 x g, 5 min) to obtain a supernatant. ScN2a cells were seeded in a cell number corresponding to 10% of confluence in a 24-well cell culture dish with 1 mL of the culture medium, to which the obtained supernatant was added, and the cells were cultured. When the cells reached to confluence, abnormal prion protein (prion) was prepared from the cell lysate according to the method of Example 1, and analyzed by Western blotting.

### Results

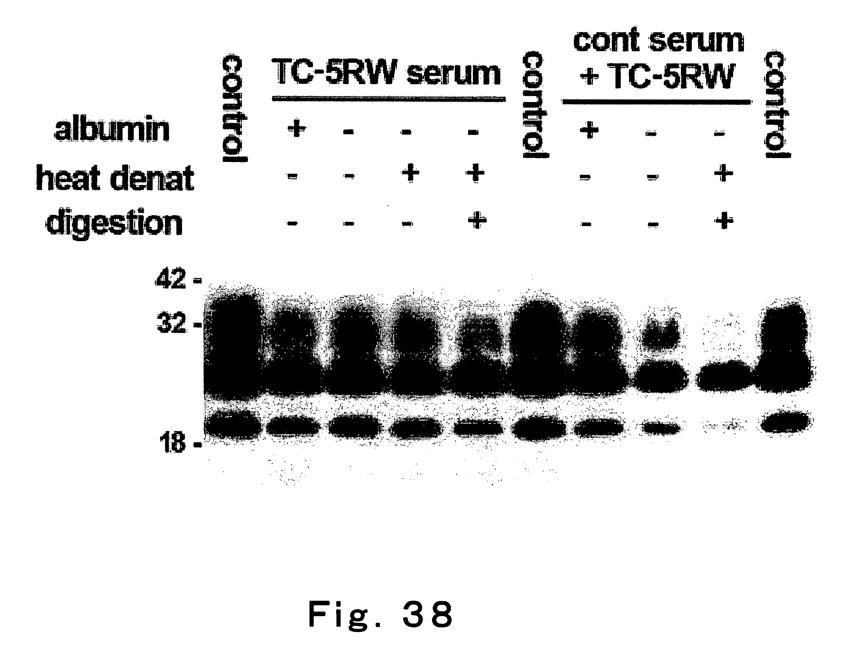

The results are shown in Fig. 38. In the figure, "control" represents the abnormal prion protein in untreated ScN2a cells; "albumin +" represents the case in which the HPMC (TC-5RW)-administered hamster serum or the HPMC (TC-5RW)-added control serum was used without elimination of albumin; and "albumin -" represents the case in which the HPMC (TC-5RW)-administered hamster serum or the HPMC (TC-5RW)-added control serum from which albumin was eliminated was used. Further, "heat denate +" represents the case treated at 95ºC for 5 min; "heat denate -" represents the case not treated at 95ºC for 5 min; "digestion +" represents the case treated with six kinds of enzymes; and "digestion- represents the case not treated with the enzymes. The anti-prion activity in the HPMC (TC-5RW)-administered hamster serum exhibited a behavior similar to that of the anti-prion activity by HPMC (TC-5RW) which had been added to the control hamster serum. The treatment with the aforementioned six kinds of enzymes did not affect the activity.
The results of any of the foregoing Examples 25 to 28 suggest that the anti-prion active factor in the HPMC (TC-5RW)-administered hamster serum has the same characters as those of HPMC (TC-5RW). Therefore, it is probable that the anti-prion active factor may be HPMC (TC-5RW) itself, or partially modified (degraded) HPMC (TC-5RW).

### Example 29

To verify the aforementioned idea, tissue distributionon of HPMC (TC-5RW) when peripherally administered to an animal (mouse) was studied in detail using a radioactive isotope (carbon 14)-labeled HPMC. First, distribution into tissues of the mouse received single administration of HPMC (TC-5RW) from the tail vein was studied.

### Cellulose Ethers Used in Evaluation

- Labeled HPMC; After making an alkali salt of HPMC (TC-5RW) (described in Example 1), it was sealed in a tube together with [¹⁴C]methyl iodide, and hexane (solvent). The tube was kept at 40 to 45ºC for 20 hrs. Unreacted methyl iodide and hexane were evaporated and removed, and thereafter, the residue was dissolved in water for purification by passing through an ion exchange resin (mixed type of H⁺ and OH⁻) column. The eluate was concentrated and dried to give labeled HPMC (specific activity; 74 kBq/mg) incorporating a ¹⁴carbon methyl group.

### Evaluation Method

Animals (Tg7 mice) which received from the tail vein single administration of the labeled HPMC in a dose described in Table 2 were killed after collecting blood from inferior vena cava under anesthesia with ether at a predetermined time. Specified tissues were extirpated, and radioactivity density and distribution rate in the tissue were determined to examine the radioactivity distribution to, and time dependent alteration in each tissue.

### Test Conditions

**Table 2**

| Test group | Administration route | Amount of administration (based on HPMC) | Measurement time | Number of measured samples | Number of animals used |
|---|---|---|---|---|---|
| first group | intravenous | 2.5 mg/body | 5 min after administration | each one sample/ tissue | 3 animals per group (total 12 animals) |
| second group | | | 2 hrs after administration | | |
| third group | | | 24 hrs after administration | | |
| fourth group | | | 72 hrs after administration | | |

### Evaluated Tissue

Samples were collected from plasma, blood, cerebrum, cerebellum*, pituitary gland**, eyeball*, Harderian gland*, thyroid gland**, mandibular gland*, thymus*, heart*, lung*, liver*, kidney*, adrenal gland**, spleen*, pancreas*, fat, skeletal muscle*, skin, bone marrow**, aorta**, testis*, epididymis*, prostate gland**, urinary bladder*, stomach*, small intestine*, large intestine* in Tg7 mouse (genetically modified mouse overexpressing a hamster type prion protein).
* : distribution rate determined. With respect to the blood and skeletal muscle, measurement was carried out provided that total amount of blood accounts for 7.78% of the body weight (reference document: Yoshio Tajima ed., Special Experimental Zoology (Zikken Doubutsugaku Kakuron), Tokyo, Asakura Publishing Co., Ltd., 1972, p. 12), and the skeletal muscle accounts for 45% of the body weight (reference document: Gerlowski LE, Jain RK, Journal of Pharmaceutical Sciences, 1983, Vol. 72, No. 10, pp. 1103-1127).
* *: determination made with three examples combined.

### Process for Collecting and Preparing Samples

With respect to the tissues other than blood and skeletal muscle, total weight of each tissue was measured for determining the distribution rate. Total weight of the liver was measured after removing the gallbladder. Total weight of the bone marrow was measured after collecting from the femur. The fat was collected from the vicinity of kidney, the skin was collected from the axillary part, and the skeletal muscle was collected from the femoral region. In weighing, the urinary bladder and the gastrointestinal tract were sufficiently washed with physiological saline after removing the contents. The small intestine was collected by recovering from the duodenum to ileum, while the large intestine was collected by recovering from the cecum to the colon.
a) Blood; The blood was collected from the inferior vena cava using a syringe treated with heparin. The blood in a volume of 100 µL was placed in a vial, and thereto was added 2 mL of a tissue dissolving agent "SOLUENE-350" (trade name) (manufactured by Perkin Elmer, Inc.) to permit dissolution. Thereafter, the solution was decolorized by adding 0. 4 mL of a benzoyl peroxide saturated benzene solution.
b) Plasma; The remaining blood other than the sample for measuring the radioactivity was subjected to centrifugal separation (8000 x g, 4ºC, 5 min), and thus obtained plasma was placed in a vial in a volume of 100 µL. Thereto was added 2 mL of a tissue dissolving agent "SOLUENE-350" (trade name) to permit dissolution.
c) Cerebrum and kidney; The cerebrum was divided into two portions, and one was placed in a vial. After measuring its weight, thereto was added 2 mL of a tissue dissolving agent "SOLUENE-350", followed by warming to permit dissolution. One of the kidneys was placed in a vial, and its weight was measured. Then, thereto was added 2 mL of a tissue dissolving agent "SOLUENE-350" (trade name), followed by warming to permit dissolution.
d) Liver; The liver was placed in a plastic vessel, and thereto was added in about two times its total weight of physiological saline. The weight was measured again, and the homogenate was prepared after mincing into small pieces. The homogenate thus prepared in a volume of 0.5 mL was placed in a vial, and the weight was measured, Thereafter, 2 mL of a tissue dissolving agent "SOLUENE-350" (trade name) was added, and warmed to permit dissolution.
e) Stomach, small intestine, large intestine; To whole of the collected tissue was added 3 mL of an aqueous 0.5 mol/L sodium hydroxide solution, followed by warming to permit dissolution. The solution was diluted by adding 5 mL of water, and 1 mL of each solution was placed in a vial.
f) Fat; About 50 mg of fat was placed in a vial, and 2 mL of a tissue dissolving agent "SOLUENE-350" (trade name) was added thereto, followed by warming to permit dissolution.
g) Eyeball and skin; Both eyeballs, and skin in an amount of about 150 mg were weighed on a cup filter paper for combustion.
h) Other tissue; The tissues in an amount of about 150 mg (the entirety when the total weight is less than about 150 mg) were placed into vials, respectively, and 2 mL of a tissue dissolving agent "SOLUENE-350" (tirade name) was added thereto, followed by warming to permit dissolution.

### Method of Radioactivity Determination

a) Eyeball and skin (combustion method); After the samples placed on the cup filter paper for combustion were dried in an incubator at 40ºC for no shorter than 24 hrs, combustion in an automated combustion apparatus was allowed. Thus generated ¹⁴CO₂ was absorbed to 6 mL of a CO₂ absorbing agent "CARBO-SORB E" (trade name) (manufactured by Perkin Elmer, Inc.). Furthermore, 12 mL of a scintillator "PERMAFLUOR E+" (trade name) (manufactured by Perkin Elmer, Inc.) was added thereto, and the radioactivity was measured using a liquid scintillation counter (LSC) (manufactured by Perkin Elmer, Inc., "2700TR" (trade name)). In the measurement by the combustion method, radioactivity density in the tissue and the distribution rate were derived after correcting with the recovery rate in combustion determined by the following formula using 0.2 mL of a ¹⁴C standard sample for sample oxidizer (manufactured by Perkin Elmer, Inc., "SPEC-CHEC ¹⁴C" (trade name)).
   Recovery rate (%)= [radioactivity (dpm) recovered following combustion of standard sample]/[radioactivity (dpm) of standard samples subjected to the combustion] x 100
b) Tissues other than eyeball and skin (dissolution method); A scintillator "HIONIC-FLUOR" (trade name) (manufactured by Perkin Elmer, Inc.) in a volume of 10 mL was added to each sample following the preparation, and the radioactivity was measured using LSC. The radioactivity density in the tissue and the distribution rate were determined from thus obtained values.

### Results

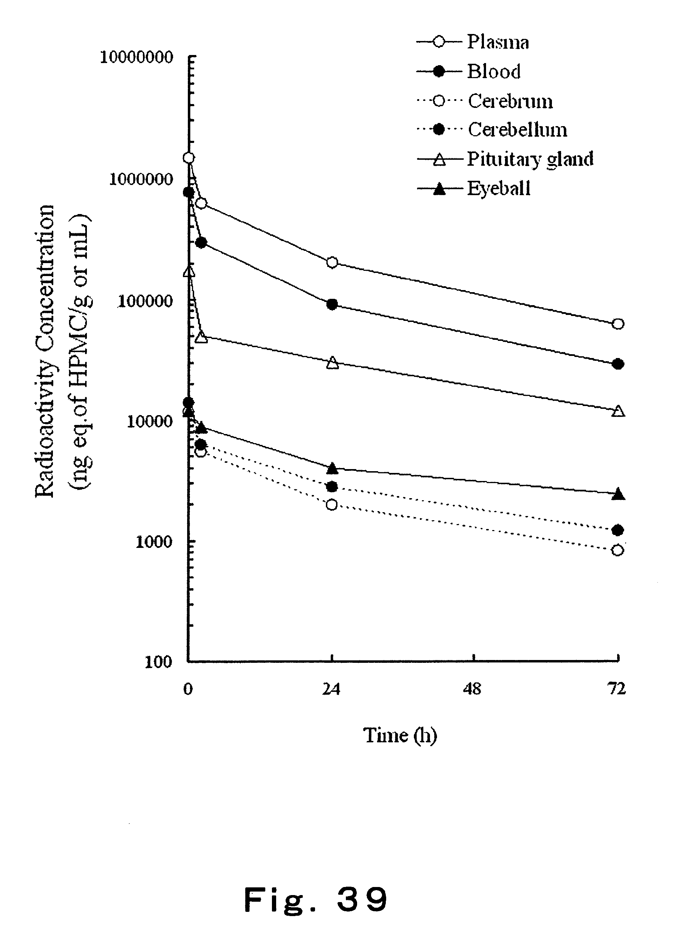

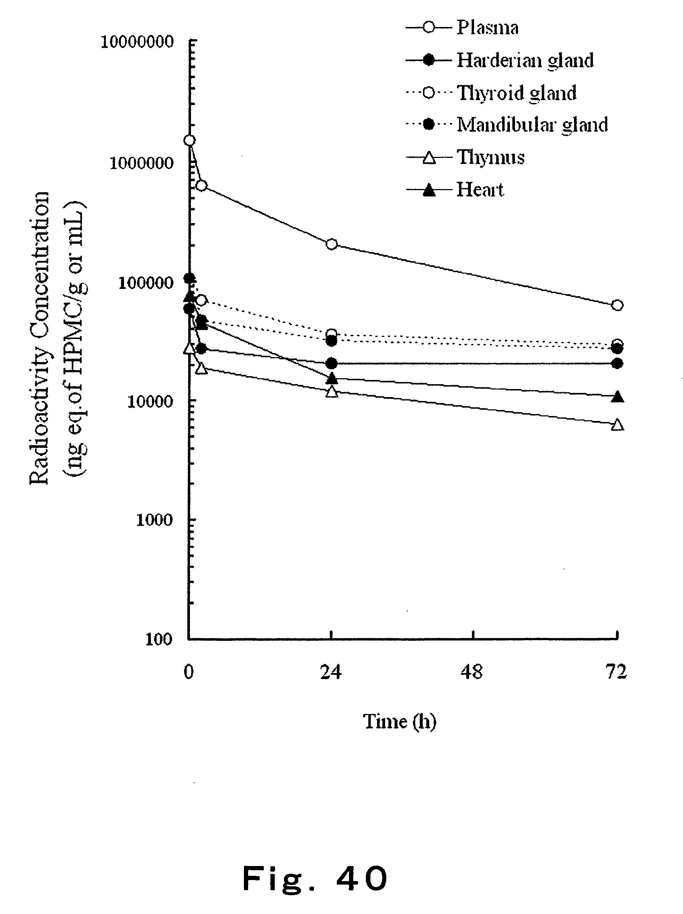

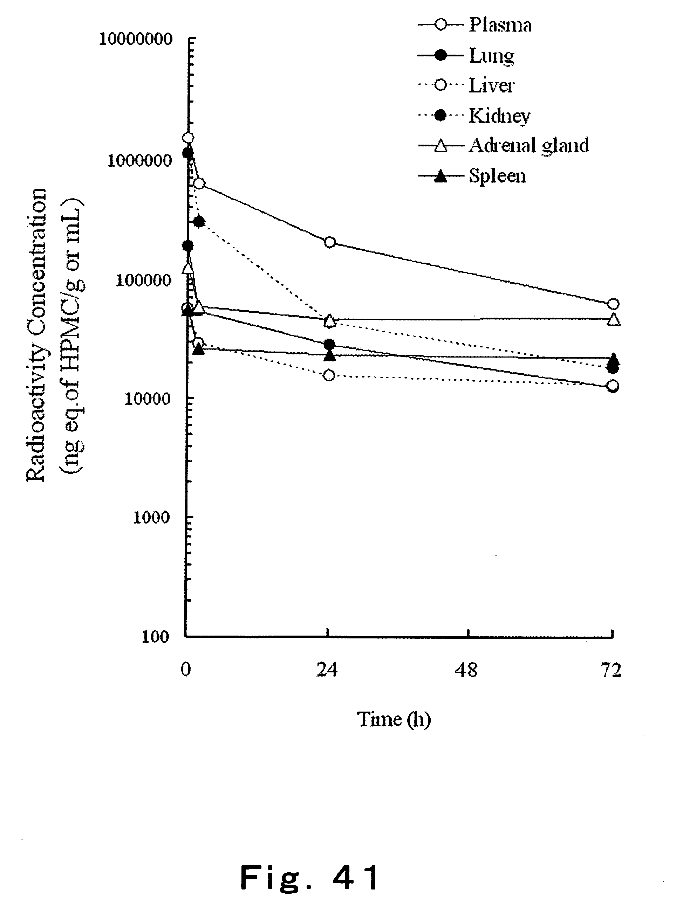

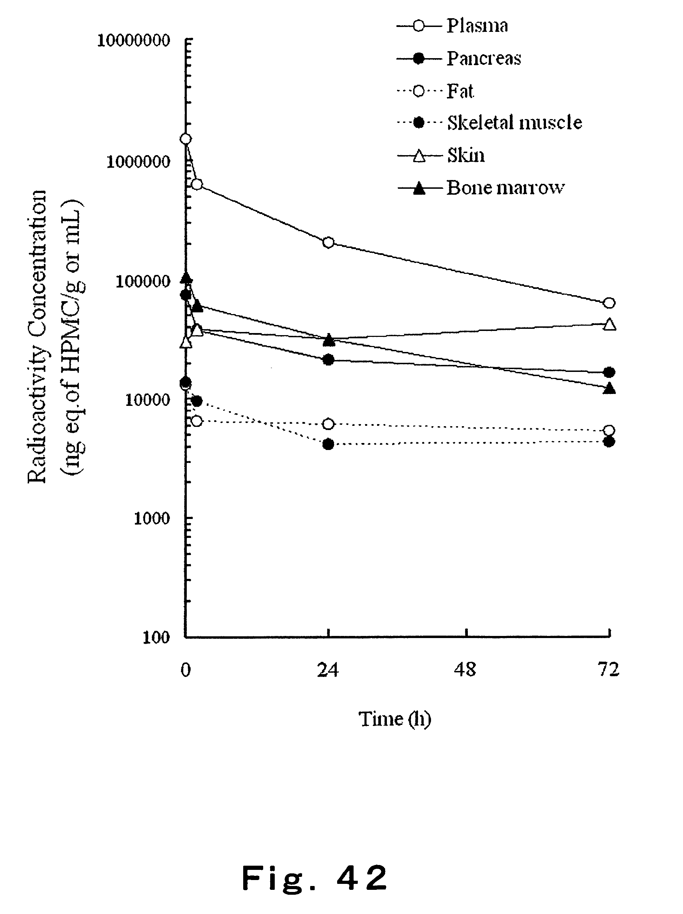

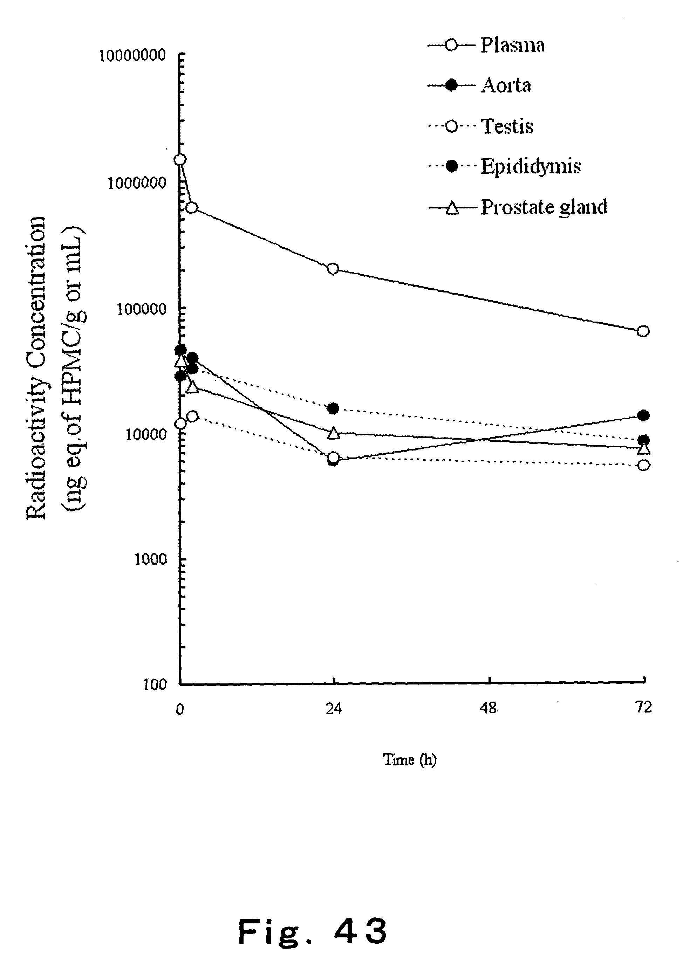

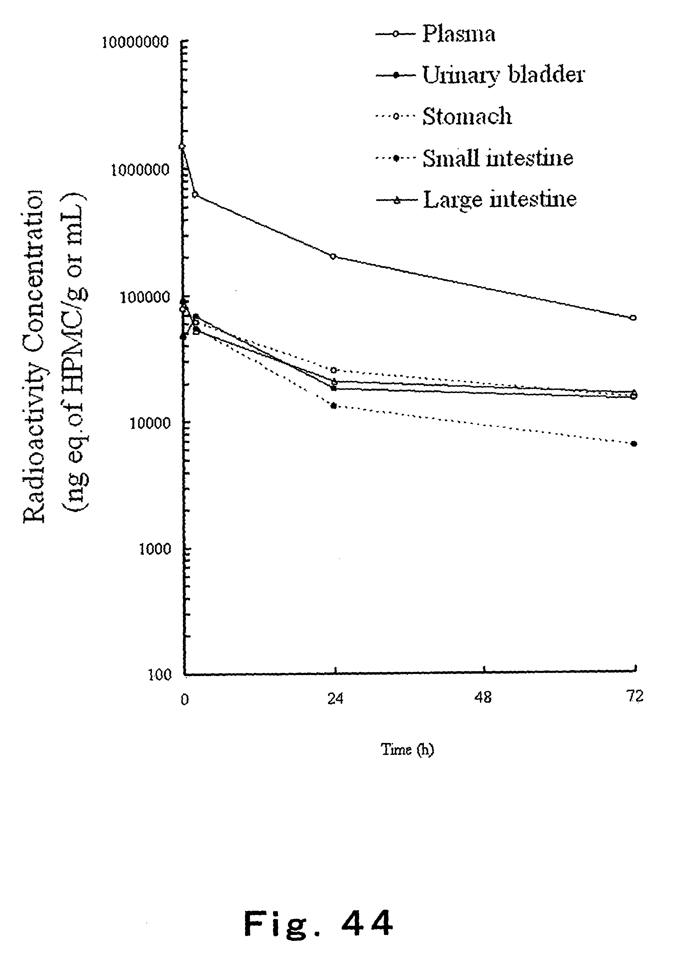

The results are shown in Figs. 39 to 44. The radioactivity density in the tissue exhibited the maximum value at 2 hours following the administration in the aorta, testis and urinary bladder; at 72 hours following the administration in the skin; and at 5 minutes following the administration (the first measurement time) in other tissues, respectively. At any of the measurement time, low radioactivity density was observed in the cerebrum and cerebellum. At 5 minutes following the administration, the highest radioactivity density (1489031 ng eq. /mL) was found in the plasma, followed by 0.75 times and 0.51 times radioactivity density of that in the plasma in the kidney (1121869 ng eq./g) and the blood (763832 ng eq./mL), respectively. The radioactivity density in other tissues was no greater than 0.13 times the radioactivity density in the plasma. At 24 hours following the administration, 75% of the maximum density was found in the skin. The radioactivity density in the testis, fat, thymus, spleen, adrenal gland, eyeball, Harderian gland, thyroid gland, epididymis, stomach, mandibular gland, skeletal muscle, bone marrow, pancreas, liver, prostate gland, urinary bladder and large intestine fell in the range of 47% to 22% of each of the maximum density, while reduction to no greater than 20% of each maximum density was found in other tissues. At 72 hours following the administration, the highest density (42338 ng eq./g) was found in the skin. The radioactivity density in the fat, testis, spleen, adrenal gland, Harderian gland, aorta, skeletal muscle, thyroid gland, mandibular gland, liver, thymus, pancreas, urinary bladder, eyeball, epididymis, prostate gland, stomach, large intestine, heart and bone marrow fell in the range of 41% to 11% of each of the maximum density, while reduction to no greater than 9% of each maximum density was found in other tissues. The radioactivity density in the cerebrum and cerebellum was the lowest among the tested tissues, and time dependent distribution pattern in these tissues almost agreed to those in the blood and the plasma.

### Example 30

Tissue distribution of labeled HPMC in mouse received single subcutaneous administration to the dorsal region was studied.

### Evaluation Method

Animals (Tg7 mice) which received single administration of the labeled HPMC (specific activity; 74 kBq/mg) prepared in a similar manner to Example 29 in a dose shown in Table 3 were killed after collecting blood from inferior vena cava under anesthesia with ether at a predetermined time. Specified tissues were extirpated, and radioactivity density and distribution rate in the tissue were determined to examine the radioactivity distribution to, and time dependent alteration in each tissue.

### Test Conditions

**Table 3**

| Test group | Administration route | Amount of administration (based on HPMC) | Measurement time | Number of measured samples | Number of animals used |
|---|---|---|---|---|---|
| fifth group | subcutaneous | 2.5 mg/body | 2 hrs after administration | each one sample/ tissue | 3 animals per group (total 9 animals) |
| sixth group | | | 24 hrs after administration | | |
| seventh group | | | 336 hrs after administration | | |

### Results

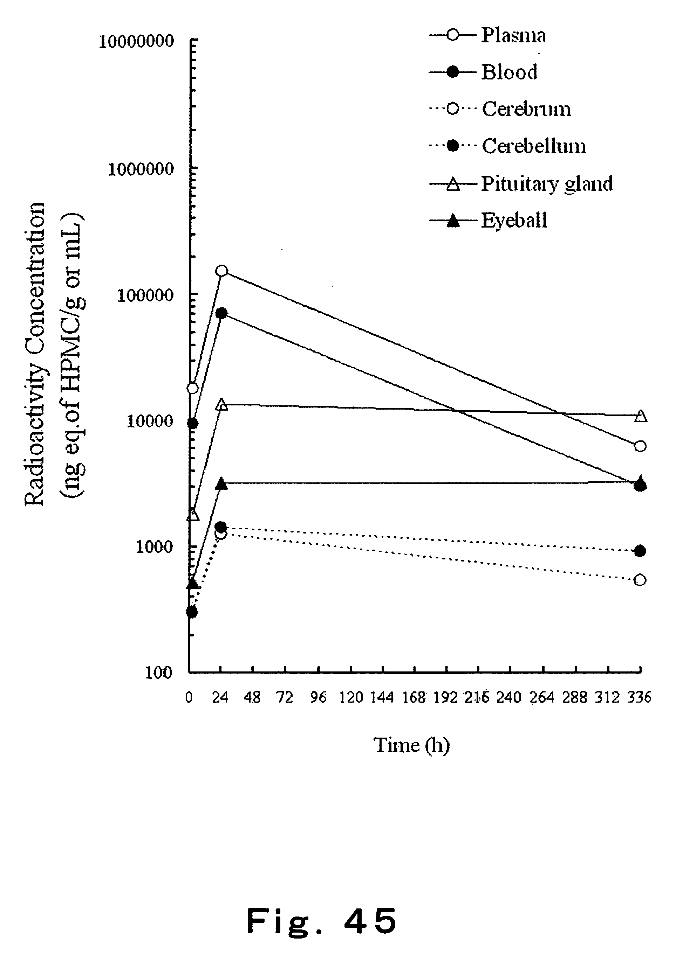

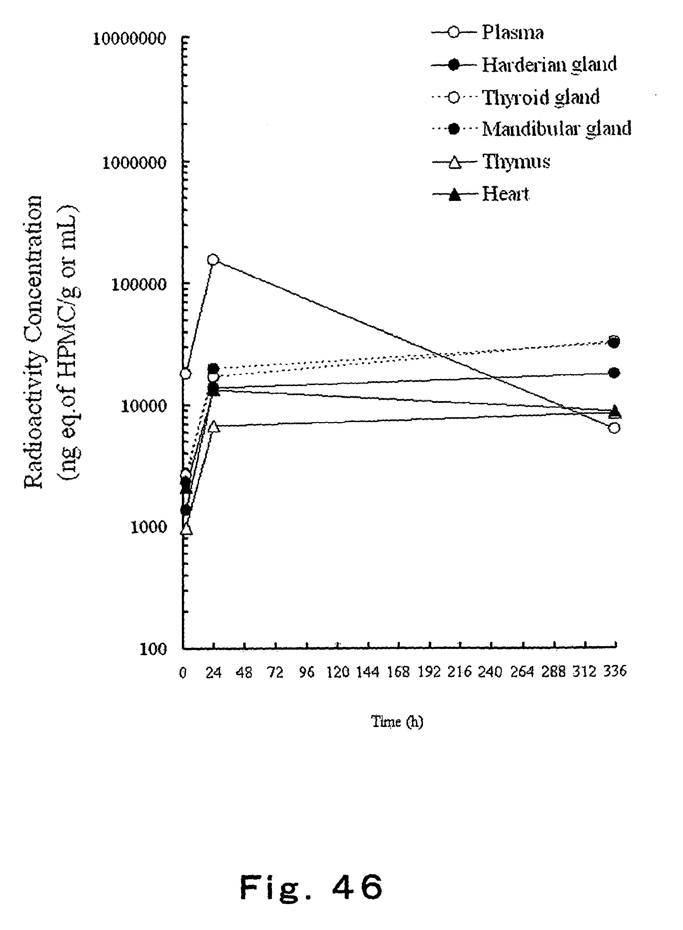

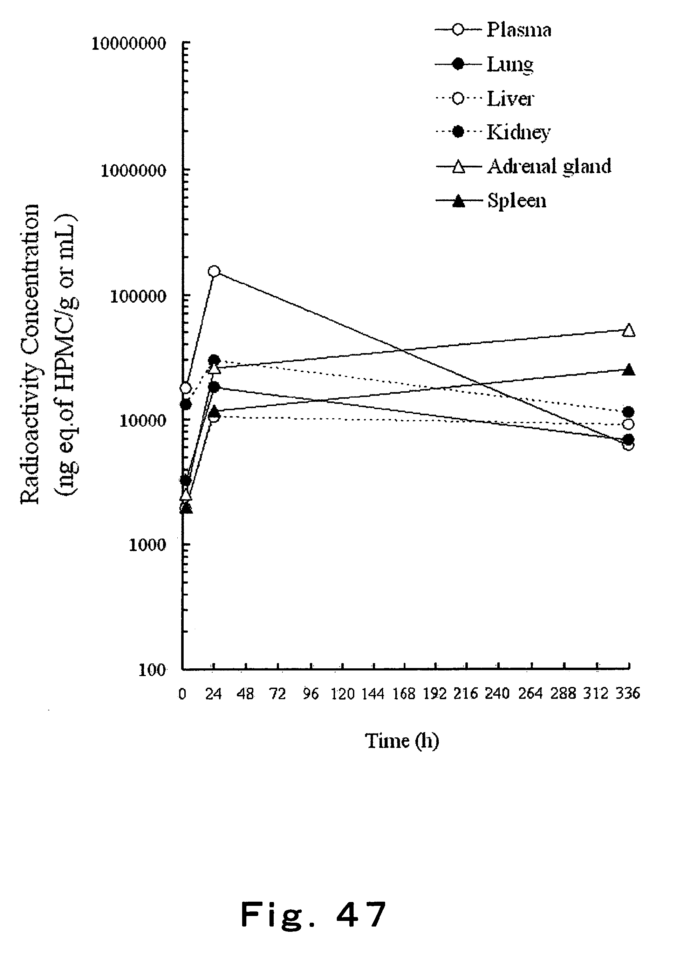

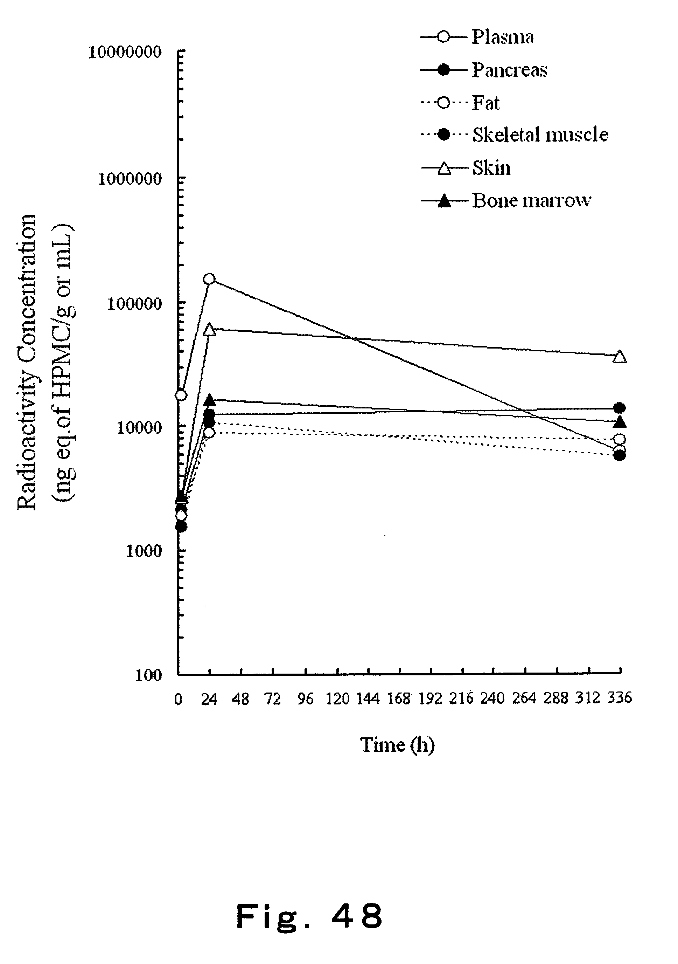

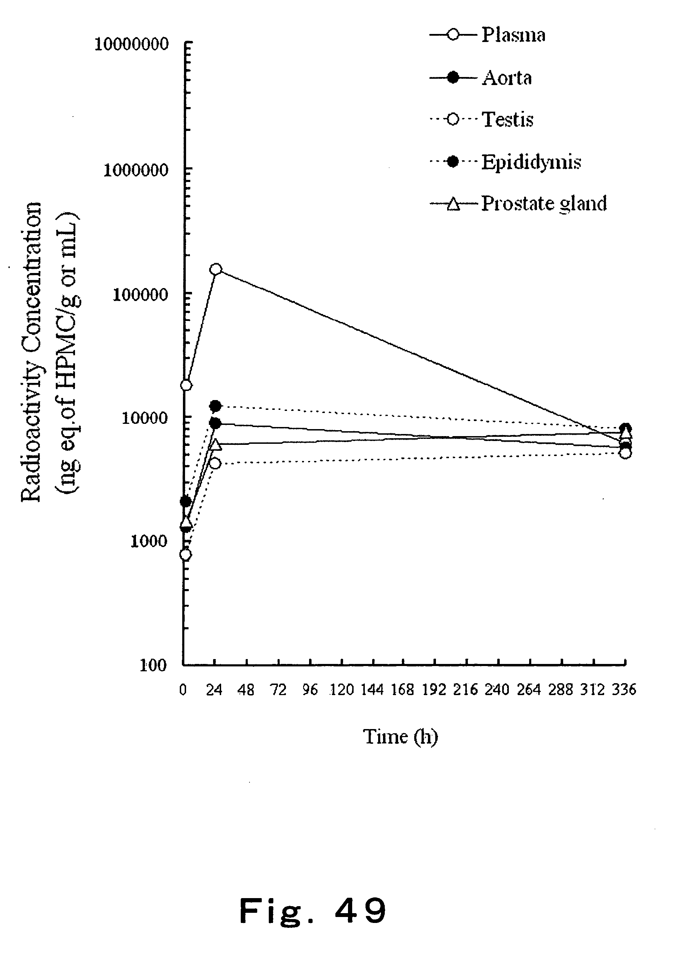

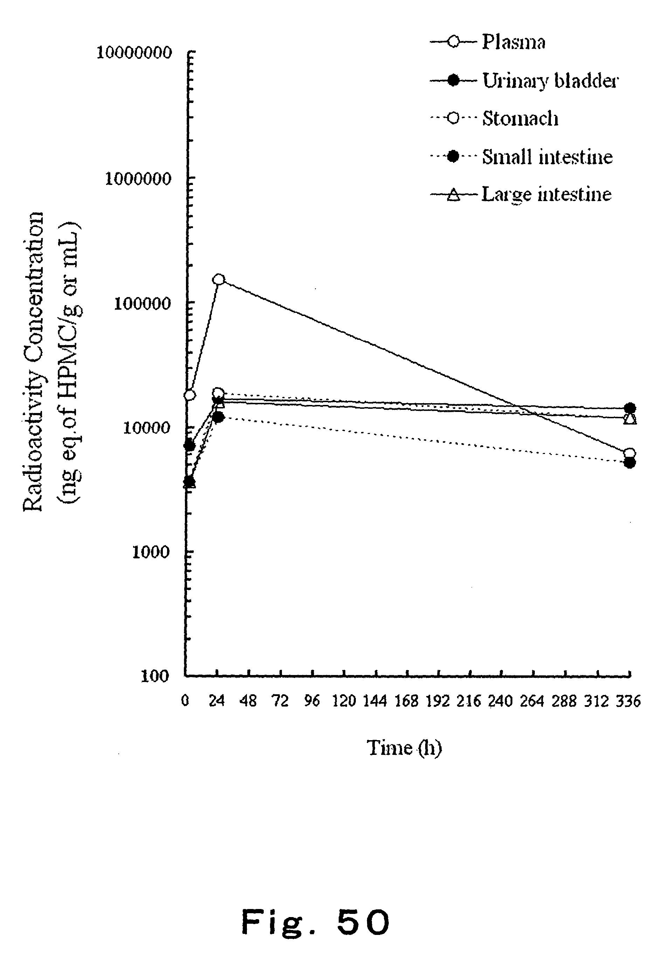

The results are shown in Figs. 45 to 50. The radioactivity density in the tissues of male Tg7 mice received subcutaneous single administration of the labeled HPMC in a dose of 2.5 mg/body exhibited the maximum value at 24 hours following the administration in the plasma, blood, cerebrum, cerebellum, pituitary gland, heart, lung, liver, kidney, fat, skeletal muscle, skin, bone marrow, aorta, epididymis, urinary bladder, stomach, small intestine and large intestine, and at 336 hours following the administration in the other tissues. At any of the measurement time, low radioactivity density was observed in the cerebrum and cerebellum. At 2 hours following the administration, the highest radioactivity density (17822 ng eq./mL) was found in the plasma, followed by 0.74 times and 0.52 times radioactivity density of that in the plasma in the kidney (13106 ng eq./g) and the blood (9294 ng eq./mL), respectively. The radioactivity density in other tissues was no greater than 0.39 times the radioactivity density in the plasma. At 24 hours following the administration, the highest radioactivity density (154071 ng eq./mL) was found in the plasma, followed by 0.45 times and 0.39 times radioactivity density of that in the plasma in the blood (69708 ng eq./mL) and the skin (299291 ng eq./g), respectively. The radioactivity density in other tissues was no greater than 0.19 times the radioactivity density in the plasma. At 336 hours following the administration, the maximum radioactivity density was exhibited in the eyeball, Harderian gland, thyroid gland, mandibular gland, thymus, adrenal gland, spleen, pancreas, testis and prostate gland, suggesting that the radioactivity derived from the labeled HPMC accumulates in these tissues. Furthermore, also in the tissues other than the blood and plasma, 87% to 37% of each maximum density, thereby revealing retarded disappearance from each tissue. These results suggest that HPMC (TC-5RW) tends to be gradually absorbed into the blood from the subeutaneous administration site, distributed to each tissue, and is accumulated in a part of the tissues, and thus a substantially long time period might be required until the labeled HPMC disappears from such tissues.

### Example 31

In addition, distribution of the radioactivity derived from the labeled HPMC in the mouse body received single intravenous administration was visualized, whereby detailed localization was studied.

### Evaluation Method

An animal (Tg7 mouse) which received single administration of the labeled HPMC (specific activity; 74 kBq/mg) in a dose shown in Table 4 was killed by anesthesia with ether at a predetermined time. Thereafter, systemic autoradiogram was produced, and distribution of the radioactivity to each tissue was studied.

### Test Conditions

**Table 4**

| Test group | Administrat ion route | Amount of administration (based on HPMC) | Production time | Number of animals used |
|---|---|---|---|---|
| eighth group | intraveno us | 2.5 mg/body | 2 hrs after administra tion | 1 animal per group (total 1 animal) |

### Process for Production

The animal was killed by anesthesia with ether at a predetermined time, and the hair coat was quickly shaved. The nasal cavity and anus were then plugged with 4% CMC-Na. Subsequently, the cadaver was frozen in dry ice-acetone, and anterior and posterior limbs, and tail were resected from thus obtained frozen cadaver, followed by embedding with 4% CMC-Na, freezing in dry ice-acetone, and then fixation on a cryomicrotome. An adhesive tape (manufactured by Sumitomo 3M Limited, "No. 810" (model number)) was attached on the produced frozen block, and a frozen section having a thickness of 30 µm was produced by the cryomicrotome, followed by freeze-drying. After the dried section was covered with a protection film (manufactured by Mitsubishi Polyester Film GmbH, "Diafoil(R)" (trade name), thickness: 4 µm), an imaging plate (manufactured by Fuji Photo Film Co. , Ltd. , "TYPE BAS SR2040" (trade name)) was brought into close contact, thereby allowing for exposure at room temperature in a lead shield box for a specified time period. Following the exposure, radioactive image on the imaging plate was read by BAS (Bio-imaging Analyzer System, manufactured by Fuji Photo Film Co., Ltd., "FUFIX-BAS2500" (trade name)). Accordingly, the radioluminogram was produced.

### Results

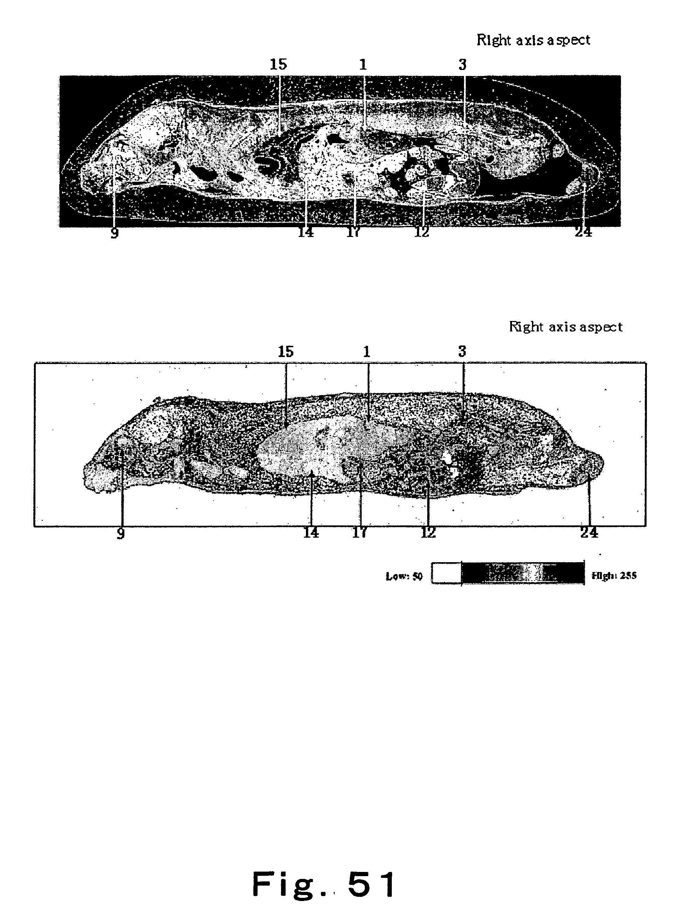

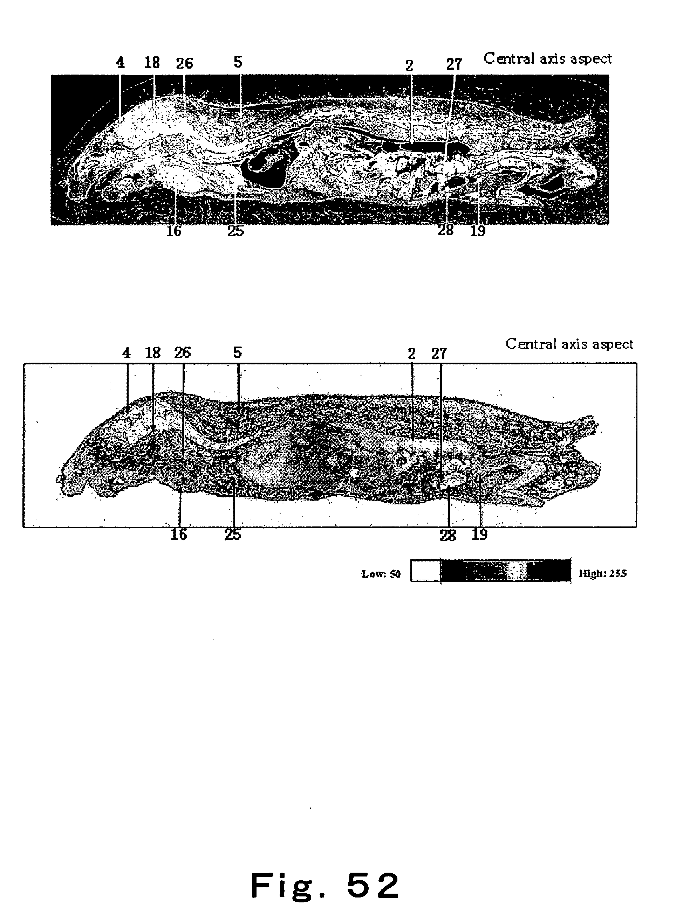

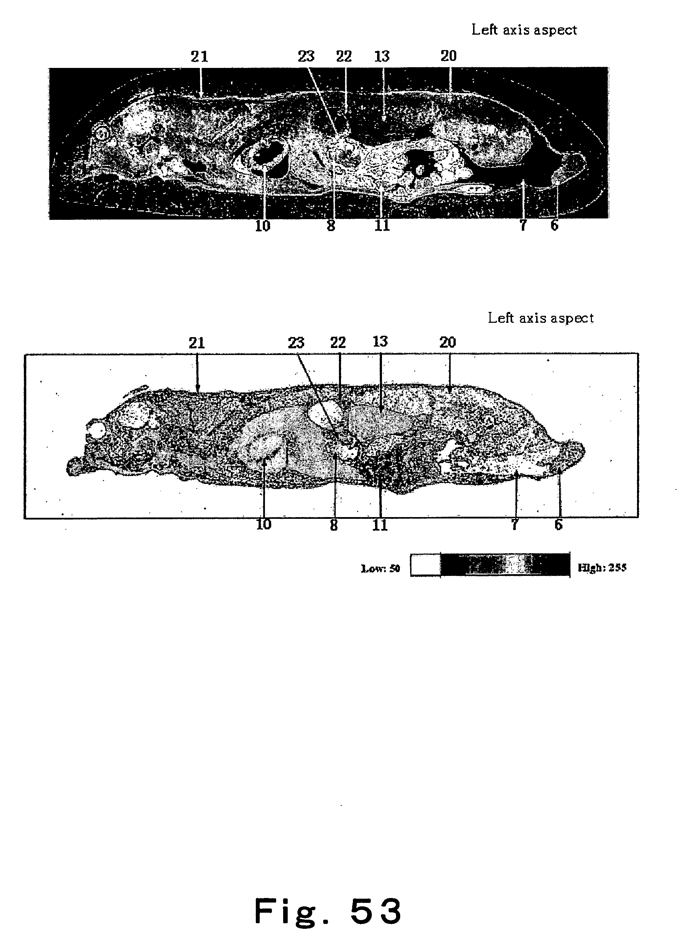

The results are shown in Figs. 51 to 53. In the figures, the reference signs 1 to 28 denote the tissues in the vicinity of the arrow head of each lead line to which each reference sign was added, and the name of the tissue denoted by each reference sign is as in the following.
1; Adrenal gland
2; Blood
3; Bone marrow
4; Brain
5; Brown fat
6; Epididymis
7; Fat
8; Gastric contents
9; Harderian gland
10; Heart
11; Intestinal contents
12; Intestine
13; Kidney
14; Liver
15; Lung
16; Mandibular gland
17; Pancreas
18; Pituitary gland
19; Prostate gland
20; Skeletal muscle
21; Skin
22; Spleen
23; Stomach
24; Testis
25; Thymus
26; Thyroid gland
27; Urinary bladder
28; Urine in bladder
When distribution into tissues at 2 hours following single intravenous administration of the labeled HPMC in a dose of 2.5 mg/body to male Tg7 mouse was studies by systemic autoradiogram, the highest radioactivity was found in the urine in bladder, and next high radioactivity was found in the blood. The radioactivity lower than that in the blood was found in the lung, adrenal gland, liver, kidney, intestine, pituitary gland, thyroid gland, epididymis, mandibular gland, urinary bladder, stomach, heart, brown fat, bone marrow, pancreas, Harderian gland, skin, prostate gland, intestinal contents, spleen, thymus and testis, and still lower radioactivity was found in the gastric contents, skeletal muscle and brain. The radioactivity in the fat was the lowest. The distribution of the radioactivity in the brain was not uniform, and it was suggested that the radioactivity signal agreed with the localization of blood vessels in the brain.
Taking into consideration the results of Examples 29 to 31, the following three points are suggested.
(1) In the case of the subcutaneously administered HPMC, HPMC itself, or its degradative modification product is gradually absorbed in the blood, distributed to each tissue in the body, and concomitantly excreted into urine.
(2) HPMC or its degradative modification product distributed into each tissue is extremely gradually degraded or released outside the tissue.
(3) It is very unlikely that HPMC or its degradative modification product directly migrates into the cerebral parenchyma across the brain-blood barrier.

### Example 32

In Alzheimer's disease that is one of typical conformational diseases, major causes of the disease are suggested to be aggregation and deposition of AB protein in the brain, and nerve cell damage resulting from the aggregated AB. Thus, effect of inhibiting aggregation of the Aβ protein by cellulose ethers was studied *in vitro.*

### Cellulose Ethers Used in Evaluation

- Hydroxypropyl methylcellulose (HPMC (TC-5RW)); described in Example 1.
- Methyl cellulose (MC (SM-4)); described in Example 8.
- Hydroxypropyl cellulose (HPCL); described in Example 8.
- HPMC hydrolyzed with 0.3% hydrochloric acid; described in Example 22.
- HPMC hydrolyzed with 40% hydrochloric acid; described in Example 22.

### Evaluation Method

A 1 mM solution of monomer type Aβ (1-40) protein(manufactured by Peptide Institute, Inc., "4379-v" (Cat. No.)) dissolved with distilled water just before use in a volume of 0.6 µL was mixed with a solution of cellulose ethers in PBS (pH 7.5). Total volume of the mixture was adjusted to 40 µL with PBS, and the reaction was allowed at 37ºC for 24 hours. Final concentration of the AB protein was 15 µM, and final concentration of each cellulose ethers was adjusted to 0.4, 2, 10, and 50 pg/mL. The extent of amyloidation of the AB protein was determined according to Thioflavin T method by Naiki, and Nakakuki (Naiki H, Nakakuki K., Laboratory Investigation 1996, Vol. 74, No. 2, pp. 374-383). Thioflavin T was blended to give a final concentration of 5 µM into the mixed reaction solution of the AB protein and the compound, and the fluorescence was measured with a fluorescence extinction microplate reader (manufactured by Molecular Dynamics Inc., "Biolumin-960" (model number), excitation wavelength: 450 nm/fluorescence intensity measurement wavelength: 485 nm). Before thioflavin T is blended, fluorescence of the mixed reaction solution of the compound and the Aβ protein was measured, and the value was subtracted from the fluorophotometric value of the mixture after blending with thioflavin T. The obtained value was converted into a relative value (%), provided that the value of the control (no compound) group was 100%.

### Results

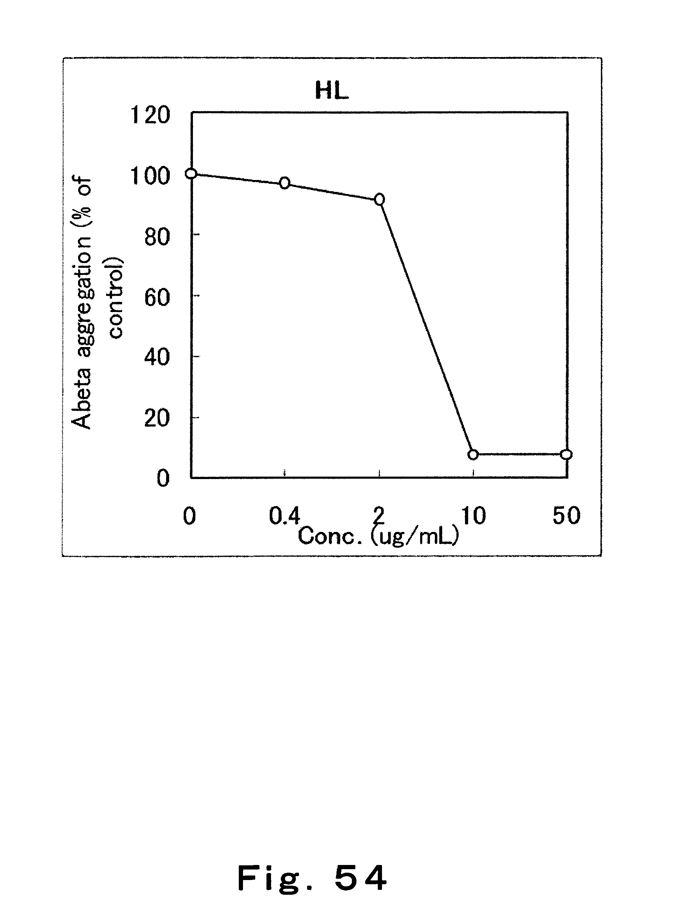

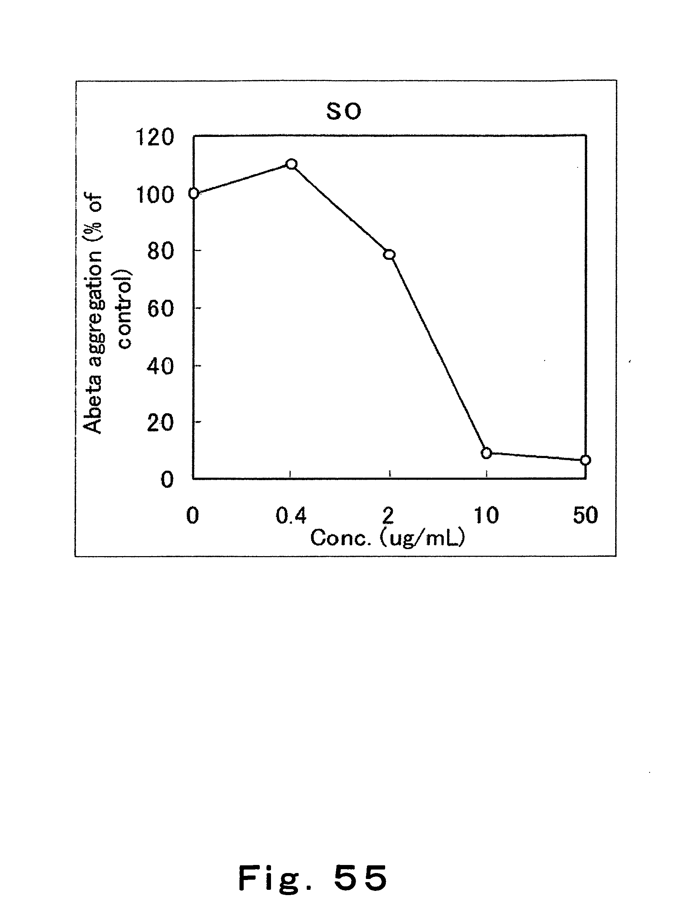

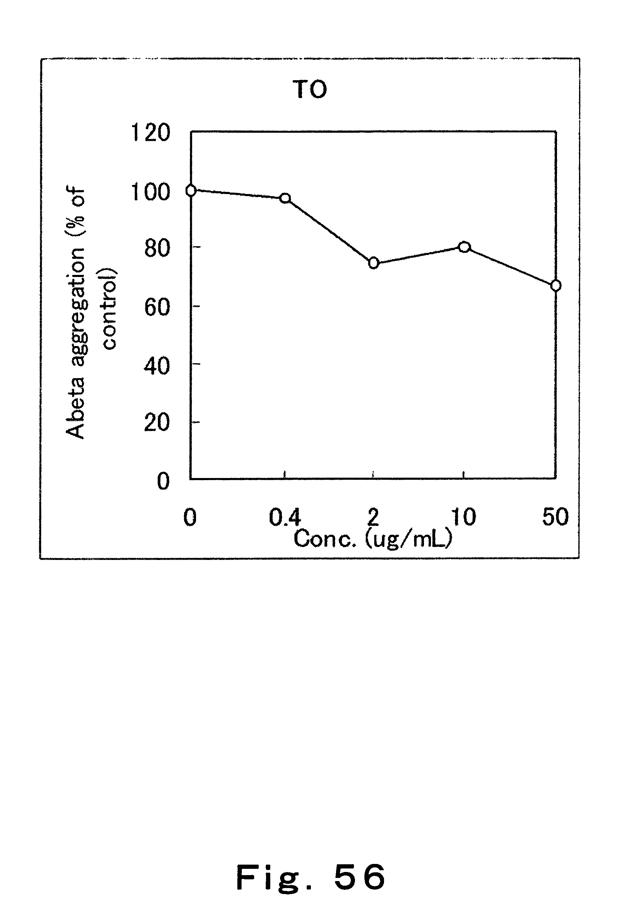

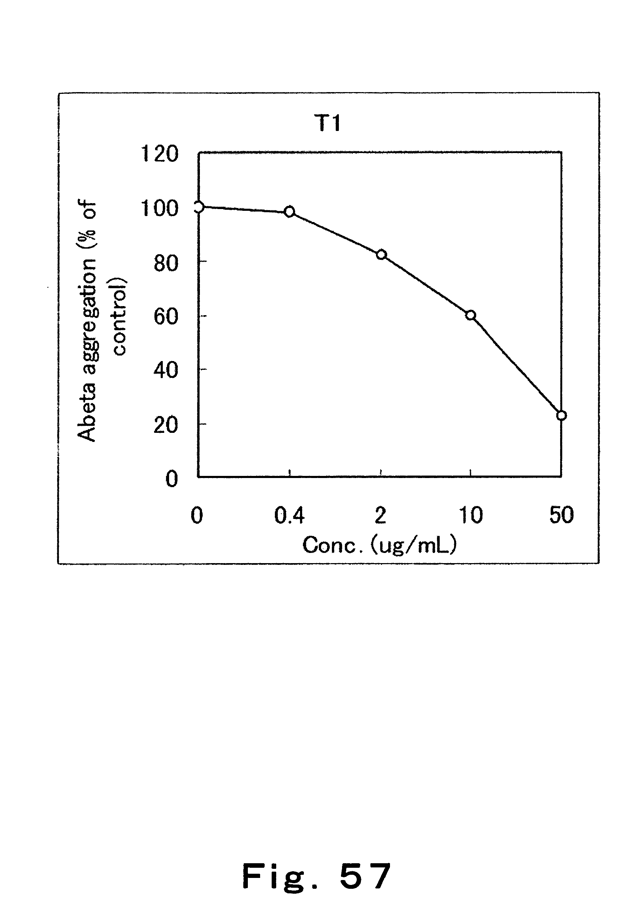

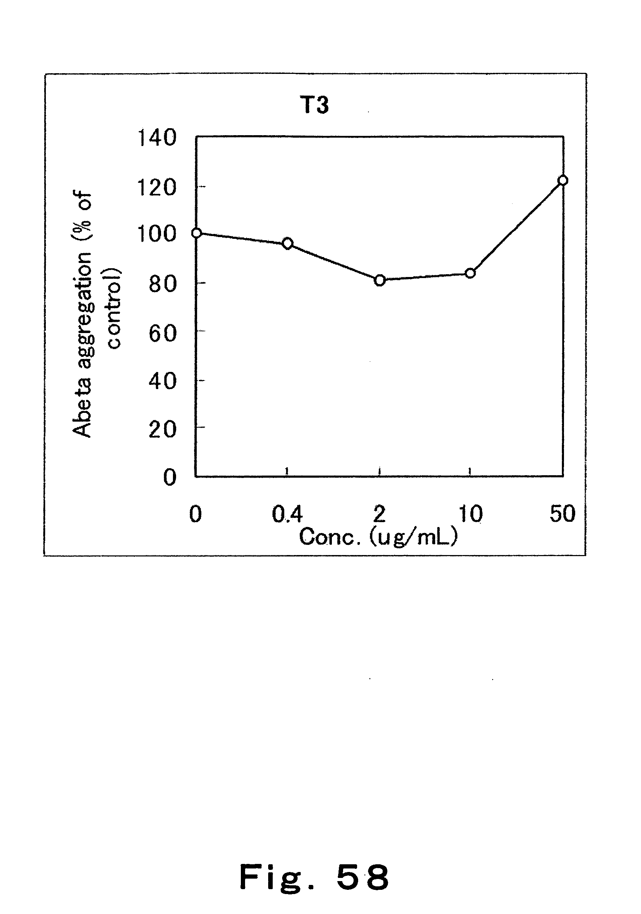

The results are shown in Figs. 54 to 58. In the figure, "HL", "SO", "TO", "T1", and "T3" respectively represent HPCL, MC (SM-4), HPMC (TC-5RW), HPMC (TC-5RW) hydrolyzed with 0.3% hydrochloric acid, and HPMC (TC-5RW) hydrolyzed with 40% hydrochloric acid. HPCL (IC₅₀ =5.1 µg/mL) and MC (SM-4) (IC₅₀ = 5.3 µg/mL) exhibited almost the same level of AB protein aggregation-inhibitory activity. HPMC (TC-5RW) hydrolyzed with 0.3% hydrochloric acid (IC₅₀ = 13.8 µg/mL) exhibited a slightly lower activity than HPCL and MC (SM-4). Meanwhile, HPMC (TC-5RW) not subjected to a hydrolyzing treatment, and HPMC (TC-5RW) hydrolyzed with 40% hydrochloric acid had an extremely weak inhibitory effect.

### Example 33

With respect to cellulose ethers, protective effect for nerve cell damage induced by the aggregated AB was studied in a mixed culture system of nerve cells and glial cells.

### Evaluation Method

Aggregated AB 1-40 (35 µM) and a PBS solution (8, 40, 200 µg/mL) of the cellulose ethers studied in Example 32 were added to a Wistar rat embryo-derived neuron and glia mixed culture system which had been cultured in a 16-well slide chamber, and incubated for 2 days (each n=3). Thereafter, the cells were fixed, and immunostained for a microtubule-associated protein (MAP2) that is present in the soma and dendrites of nerve cells. The number of MAP2 positive cells of the specimen was counted under a light microscope mounting a grated ocular lens (objective lens: X 20, and ocular lens: X 10). One site in the central part of each well was decided as the site for counting. In the Wistar rat embryo derived neuron and glia mixed culture, nerve cells and astroglial cells were prepared using a female Wistar rat embryo on day 18-19 of gestation, according to the method described in "Hiroshi Hatanaka, Hiroko Tsukui, Experimental Medicine, Extra number: Manual for Neurobiochemistry (1990) p129-135 Culture of nerve cells from several central nerve nuclei ". The nerve cells diluted to a density of 10⁶/mL with a 10% FBS/Neurobasal medium (GIBCO, Cat. No. 21103-049) to which an antibiotics mixed solution (Sigma Corporation, Cat. No. A9909) had been added was dispensed in a 16-well slide chamber (100 µL/well) in which astroglial cells had already been proliferated to reach to confluence, and cultured in a CO₂ incubator. On day 3 following initiation of the mixing culture, the medium was replaced with a B27 supplement (Invitrogen, Cat. No. 17504-044)-added Neurobasal medium, and then subjected to the experiment. The aggregated AB was produced by dissolving Aβ1-40 protein (manufactured by Peptide Institute, Inc., 4379-v (Cat. No.)) in Dulbecco's Modified Eagle Medium (DMEM) (manufactured by Invitrogen Corporation, 11885-084 (item number)) to have a concentration of 1 mM, incubating for 2 weeks in a CO₂ incubator, and diluting with DMEM for use in the experiment. The immunostaining for MAP2 was carried out as follows.
After the cells were washed with PBS, they were immersed in 5% acetic acid-added 95% ethanol cooled to -30ºC for 10 min to fix the cells. After extensively washing with PBS, a treatment of blocking endogenous peroxidase was conducted. After allowed in 1% bovine serum albumin-added PBS for 15 min, the cells were washed three times with PBS, and an anti-MAP2 antibody (manufactured by Sigma Corporation, "M-1406" (item number), diluted x 400 with 1% bovine serum albumin-added PBS) was allowed to react at 4ºC overnight. After washing five times with PBS, a horseradish peroxidase-conjugated anti-mouse immunoglobulin antibody (manufactured by Amersham plc, "NA93IV" (item number), diluted x 100 with 1% bovine serum albumin-added PBS) was allowed to react at room temperature for 1 hour. After washing five times with PBS, diaminobenzidine (DAB) was used to visualize an antigen-antibody complex by way of peroxidase reaction.

### Results

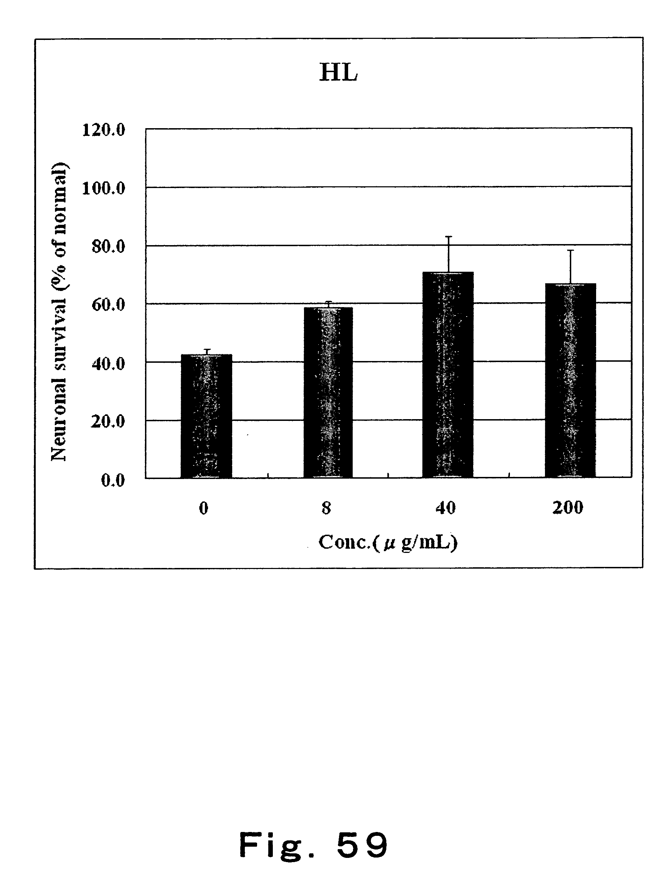

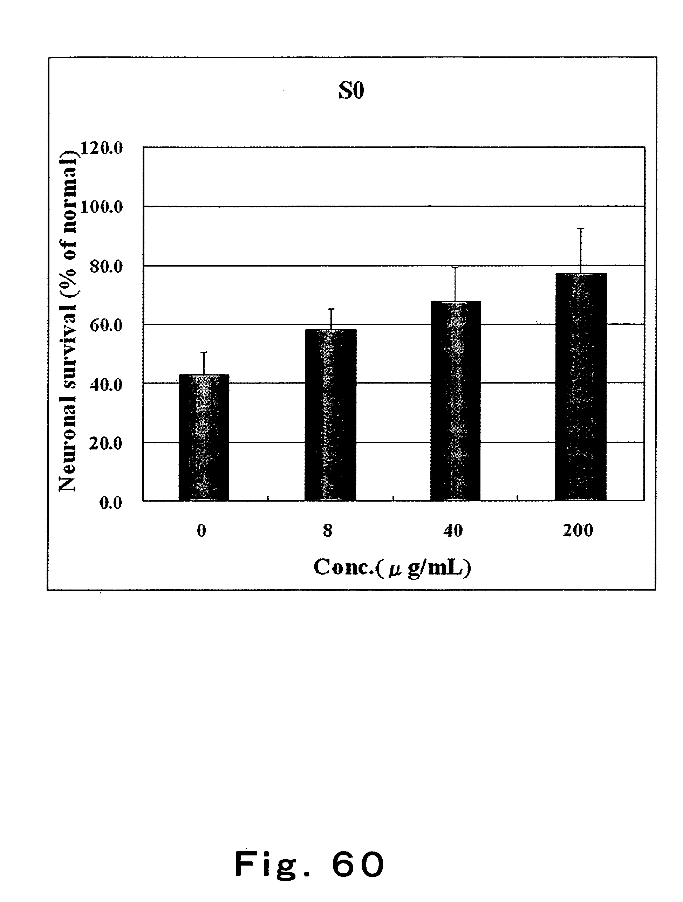

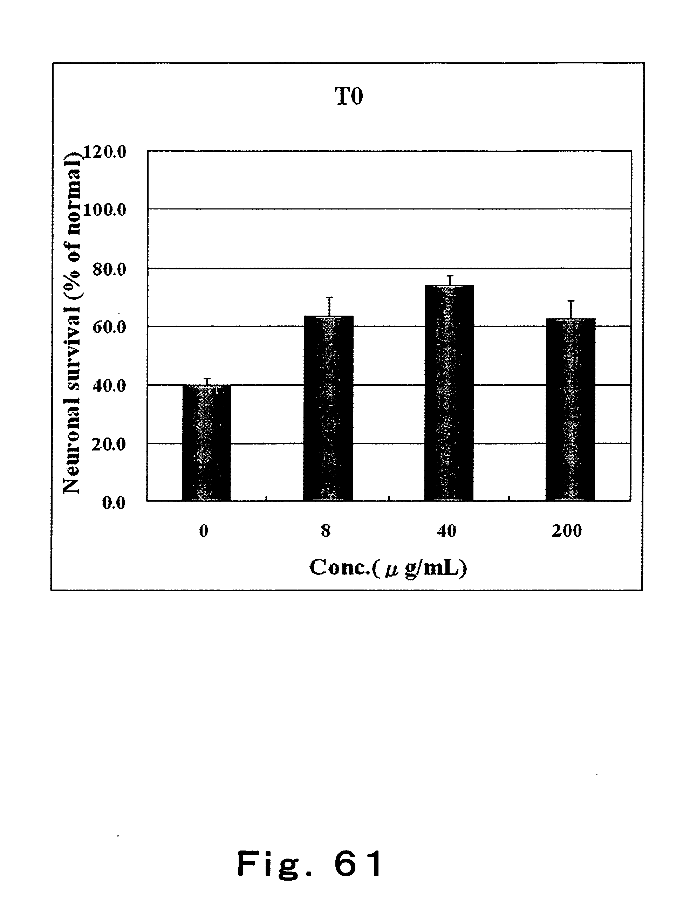

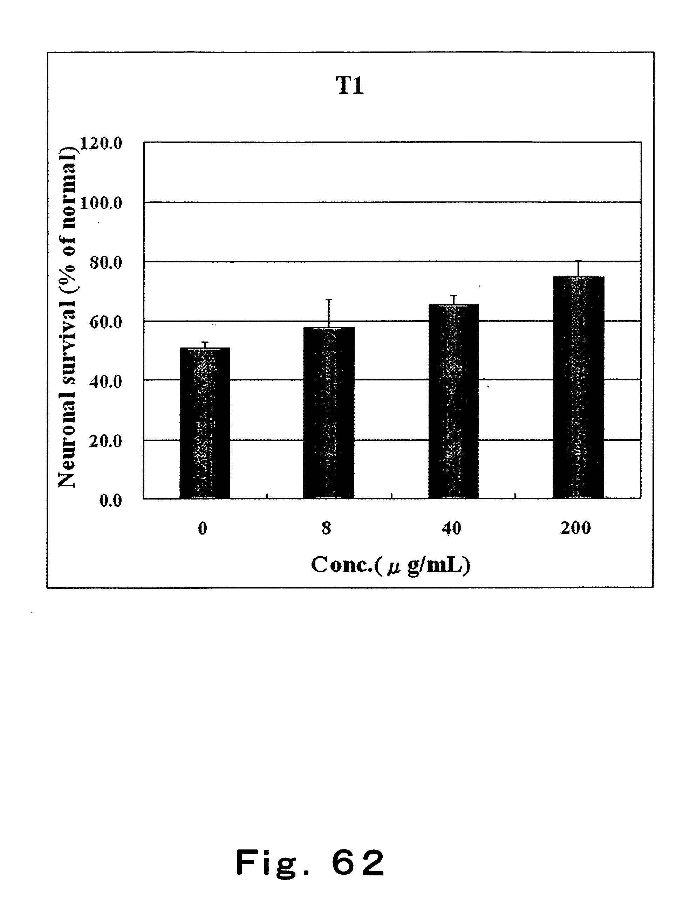

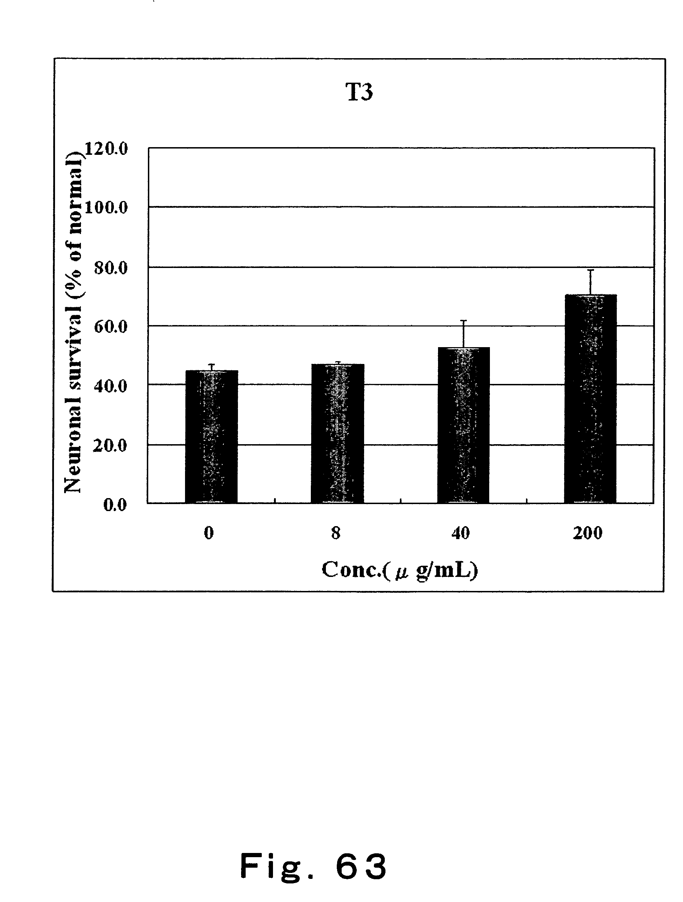

The results are shown in Figs. 59 to 63. In the figure, "HL", "SO, "TO, "T1, and "T3" represent HPCL, MC (SM-4), HPMC (TC-5RW), HPMC (TC-5RW) hydrolyzed with 0.3% hydrochloric acid, and HPMC (TC-5RW) hydrolyzed with 40% hydrochloric acid, respectively. When the cellulose ethers were added at a concentration of 8, 40, and 200 µg/mL, any of the cellulose ethers at a concentration of up to 40 pg/mL exhibited a protective effect for nerve cells against aggregated AB in a concentration dependent manner. The HPMC (TC-5RW) hydrolyzed with 40% hydrochloric acid exhibited a slightly poor effect. HPCL, MC (SM-4) and HPMC (TC-5RW) exhibited almost the same level of the protective effect for nerve cells at a concentration of 40 pg/mL. At a concentration of 200 pg/mL, further improvement of the effect was found with MC (SM-4) and hydrolyzed HPMC (TC-5RW), but in the case of HPCL and HPMC (TC-5RW), the effect was likely to be somewhat inferior to that at a concentration of 40 pg/mL. These results were almost consistent to those of the inhibitory effect on aggregation of Aβ protein.

### Example 34

From Example 32 and Example 33, therapeutic effect for Alzheimer's disease was expected, therefore, the effect of the cellulose ethers was studied using a disease model animal (mouse) of Alzheimer's disease.

### Cellulose Ethers Used in Evaluation

- Hydroxypropyl methylcellulose (HPMC (60SH-50)); described in Example 6.
- Hydroxypropyl methylcellulose(HPMC (60SH-400)); described in Example 11.

### Evaluation Method

As a disease model mouse, APP/Tau transgenic mouse (manufactured by Taconic, 600902 (item number)) was used in which a mutated form of amyloid precursor protein and a mutated form of tau protein are expressed. One group was constituted with the same aged (weeks old) five female animals, and the cellulose ethers were prepared to form a PBS solution having a concentration of 25 mg/mL, while PBS alone was used as a control. In a similar manner to Example 10, each solution was filled in an osmotic pump, and when the animal became 15 weeks old, intracerebroventricular administration for 4 weeks was started. In addition, from 20 weeks old until 30 weeks old, 0.25 mL of each solution of the cellulose ethers having a concentration of 25 mg/mL was kept intraperitoneally administered to each mouse at a rate of once per every two weeks. From 39 weeks old, a glucose liquid consumption test was initiated. In addition, a burrowing test was initiated from 40 weeks old. Both tests were performed according to the method of Boche *et al.,* (D. Boche, C. Cunningham, et al., Neurobiology of Disease, 2006, Vol. 22, pp. 638-650), once per week from 9 o'clock in the morning until 9 o'clock in the morning on the next day. In the burrowing test, 190 g of feed pellets was placed in a pipe (vinyl chloride pipe having a diameter of 76 mm, a length of 200 mm, and a height of the inlet from the floor of 30 mm), and the weight of the feed remaining in the pipe was measured on the next day, whereby the amount of transfer was determined. In a glucose liquid consumption test, a 5% glucose solution was prepared by dissolving D(+) glucose (manufactured by Wako Pure Chemical Industries, Ltd., "041-00595" (Cat. No.) in distilled water, filled in a water supply bottle, and allowed the mouse to take ad libitum. On the following day, the residual quantity was measured to determine the consumed amount of the glucose solution.

### Results

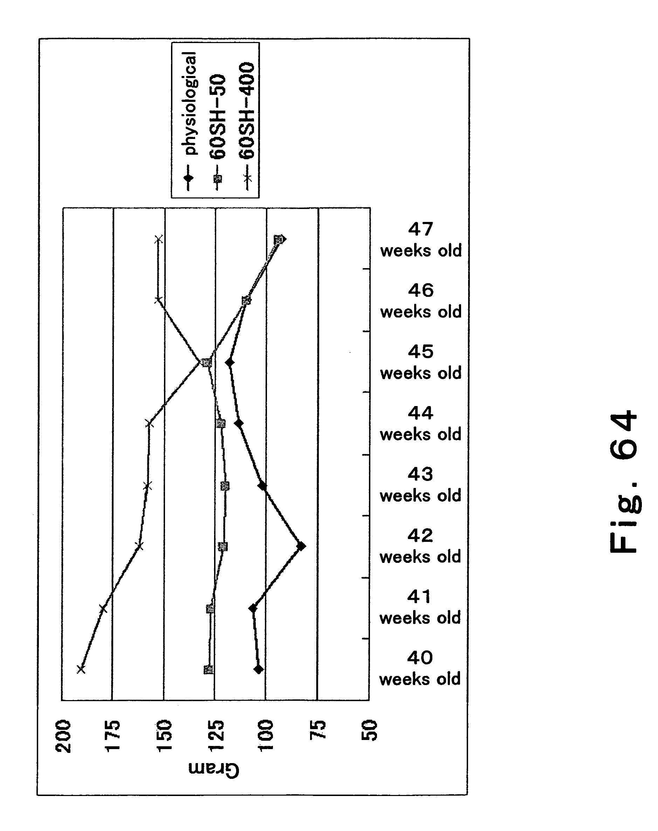

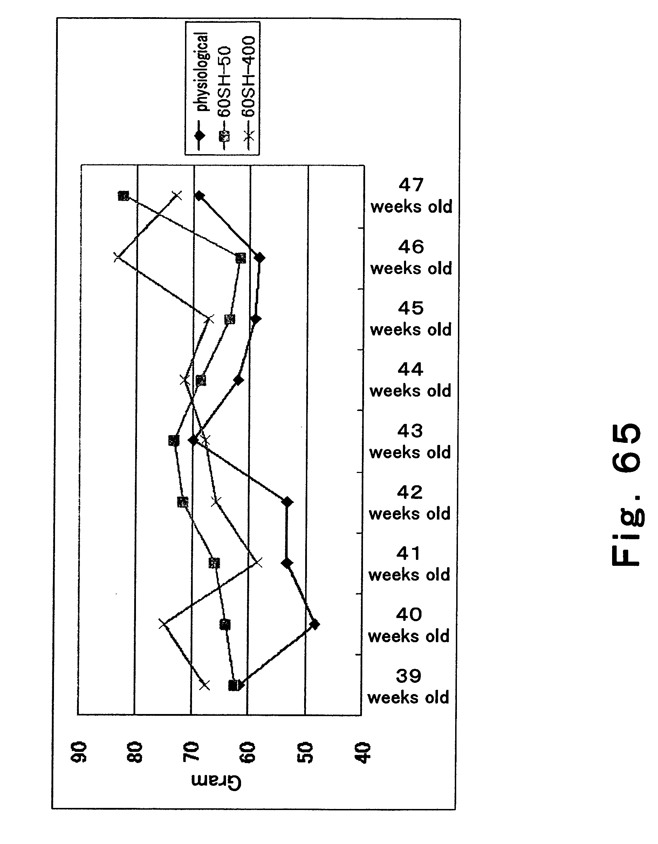

The results of the burrowing test are shown in Fig. 64. Both the HPMC (60SH-50)-administered group and the HPMC (60SH-400)-administered group achieved significantly good results (p<0.05, and p < 0.01, respectively) as compared with the control group. Further, the results of the glucose liquid consumption test are shown in Fig. 65. Both the HPMC (60SH-50)-administered group and the HPMC (60SH-400)-administered group achieved significantly good results (both p < 0.05) as compared with the control group. The foregoing results suggest that the cellulose ethers can exert the therapeutic effect for Alzheimer's disease also *in vivo.*

### Example 35

Amyloidoses also form a group of diseases typical of conformational diseases. The effect of suppressing amyloid deposition by the cellulose ethers was studied in vivo in an animal disease model of AA amyloidosis where AA amyloid is accumulated in various peripheral organs in association with chronic inflammatory diseases such as rheumatism, Crohn's disease and the like. The outcome achieved in this animal disease model has been known to be capable of being adapted not only to AA amyloidosis, but also to other amyloidosis, Alzheimer's disease, and the like (Nonpatent Document 5, 7 and 8).

### Evaluation Method

In this model, an inflammatory agent is administered to a mouse thereby increasing the blood level of serum amyloid A protein (SAA) that is an inflammatory reactive protein, similarly to the disease state in human, and then amyloid A protein (AA) that is an amyloid-forming protein cleaved from SAA aggregates to accumulate in various peripheral organs (most frequently invaded organs: spleen and liver). With inflammation only, one month or longer period is required until amyloid is accumulated in the mouse, but it is known that the time period required for amyloid accumulation can be shortened to several days when an AA amyloid-accumulated tissue homogenate (amyloid enhancing factor; AEF) is administered concomitant with or prior to the administration of the inflammatory agent.

To the dorsal region of six months or older female ICR mouse (available from CLEA Japan, Inc., inbred Jcl: ICR mouse, body weight: about 30 g/animal) 50 mg, 75 mg or 100 mg of HPMC (TC-5RW), MC (SM-4), or HPCL was subcutaneously administered (administration was carried out by wrapping in a wafer sheet, and embedding subcutaneously in a similar manner to Example 2). A compound-nonadministered group (wafer sheet alone) was provided as a control. In each group, one animal was used. AEF and an inflammatory agent were administered to allow amyloidosis to be developed 1.5 months or 4 months following the administration of the cellulose ether. AEF was prepared by adding three times weight of PBS to a mixture of spleen and liver of an AA amyloid mouse in which amyloid had already accumulated enough, and homogenizing the mixture using a Polytron homogenizer. The homogenate in a volume of 0.5 mL diluted two times with PBS was intraperitoneally administered to the mouse. The inflammatory agent was prepared by dissolving azocasein (manufactured by Sigma Corporation) in sterile water to produce a 10% solution, adding thereto an equal amount of Freund's Complete Adjuvant (manufactured by GIFCO Inc.), and homogenizing the mixture with a Polytron homogenizer while cooling to give an emulsion. The emulsion was subcutaneously administered to the dorsal region in a volume of 0.5 mL per animal. One week later, the liver and the spleen were removed from the mouse, and immersed in buffered formalin for 1 week to permit fixation, followed by embedding in paraffin to produce a 0.4 µm tissue section. The tissue section was subjected to Congo red staining to detect amyloid that produces green birefringence under a polarizing microscope. In addition, serial tissue sections were immunostained with an antibody against SAA, whereby deposition of AA (amyloid) was detected. The immunostaining was carried out as follows.
The tissue section subjected to a treatment for blocking endogenous peroxidase was treated with 98% formic acid at room temperature for 5 min. After the tissue section was washed with 50 mM Tris hydrochloric acid (pH 7.5) twice, and with distilled water once, a treatment with 0.1% sodium N-dodecyl sarcosinate (Sarkosyl), 25 mM NaOH, and 2% NaCl solution was allowed at room temperature for 10 min. After sufficiently washing with running water, distilled water, and 50 mM Tris hydrochloric acid, an anti-SAA antibody (manufactured by Santa Cruz Biotechnology, Inc., sc-20651 (item number), diluted x 500 with 0.1% bovine serum albumin-added PBS) was allowed to react at room temperature for 1 hour. After sufficiently washing with 50 mM Tris hydrochloric acid, HISTOFINE (manufactured by Nichirei Bioscience Co. Ltd.) was added onto the tissue section dropwise, and the reaction was allowed at room temperature for 30 min. After sufficiently washing with 50 mM Tris hydrochloric acid, the antigen-antibody complex was visualized by way of a peroxidase reaction using diaminobenzidine (DAB).

### Results

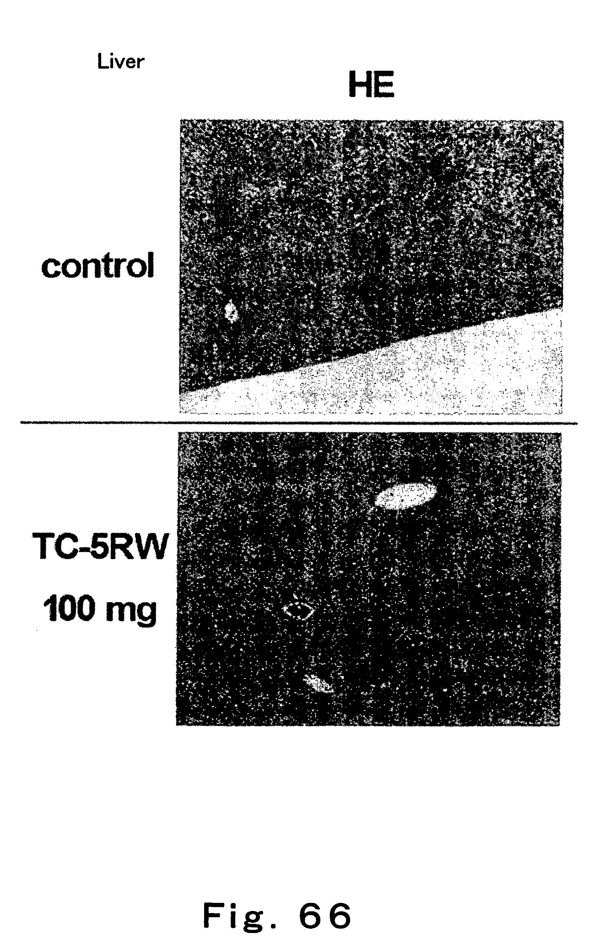

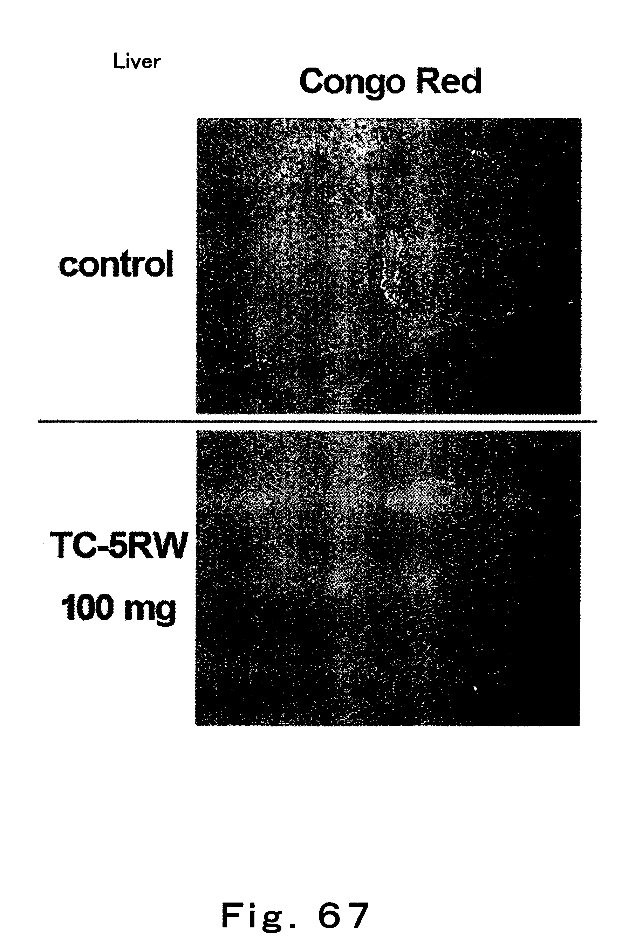

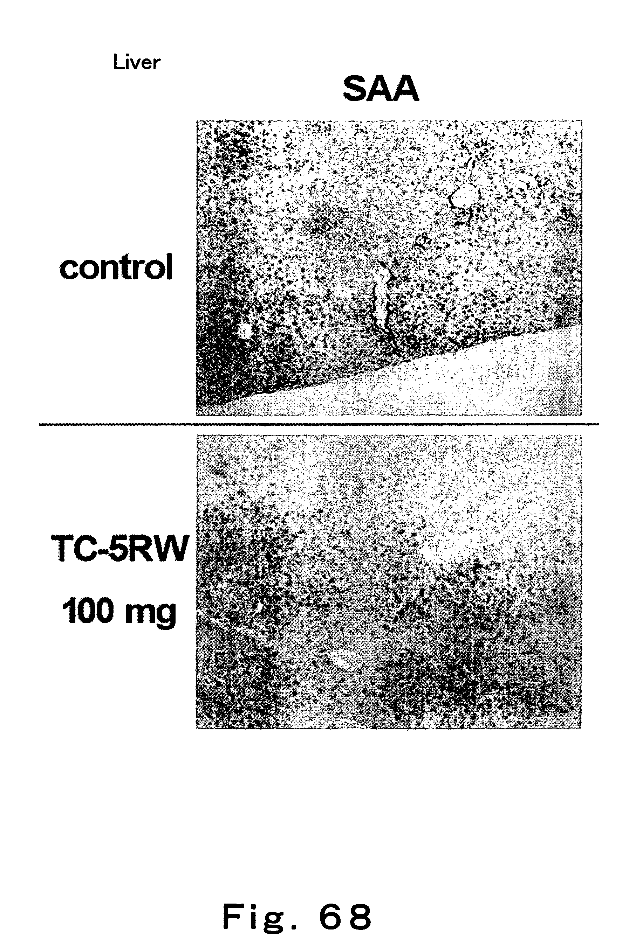

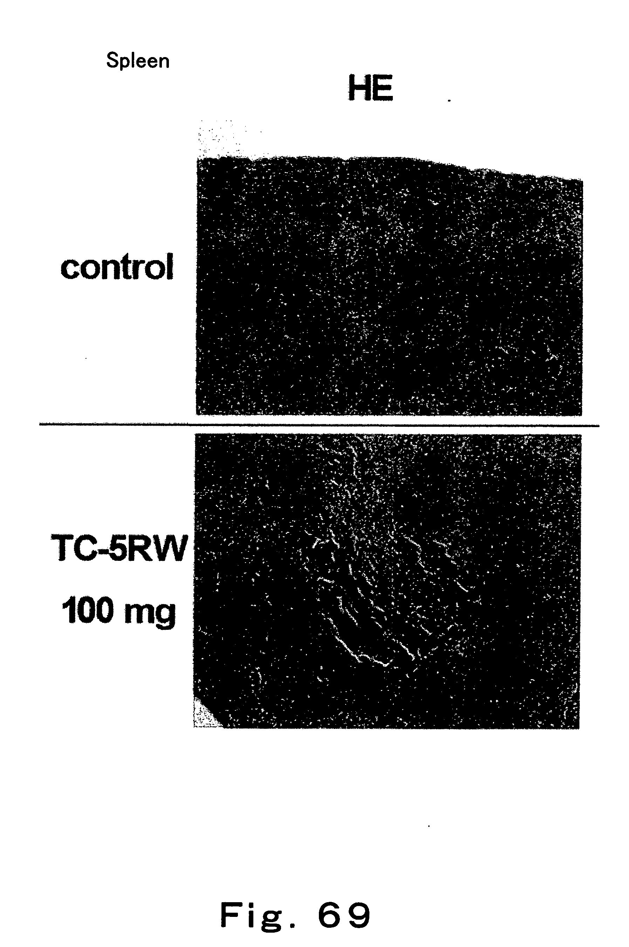

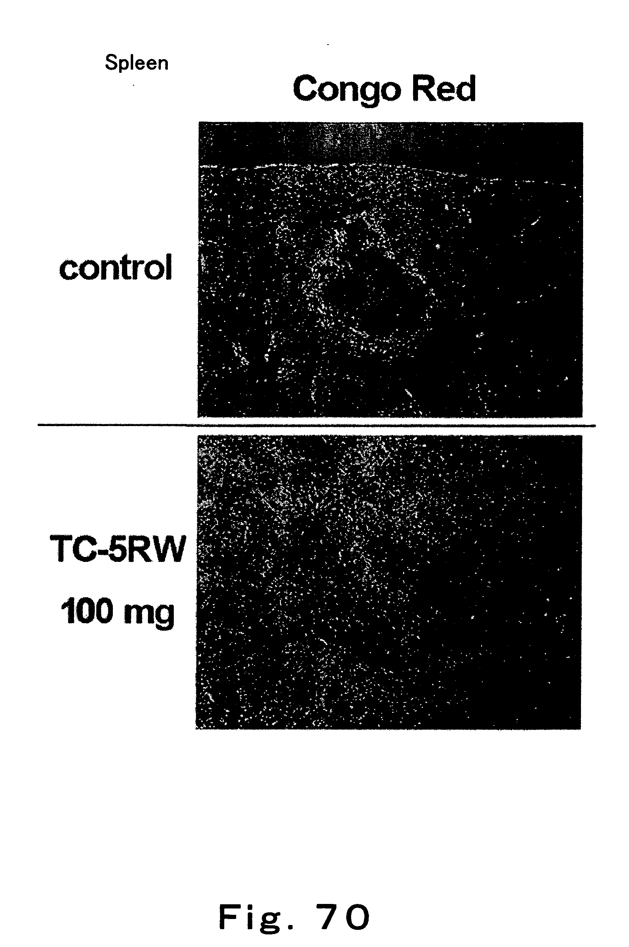

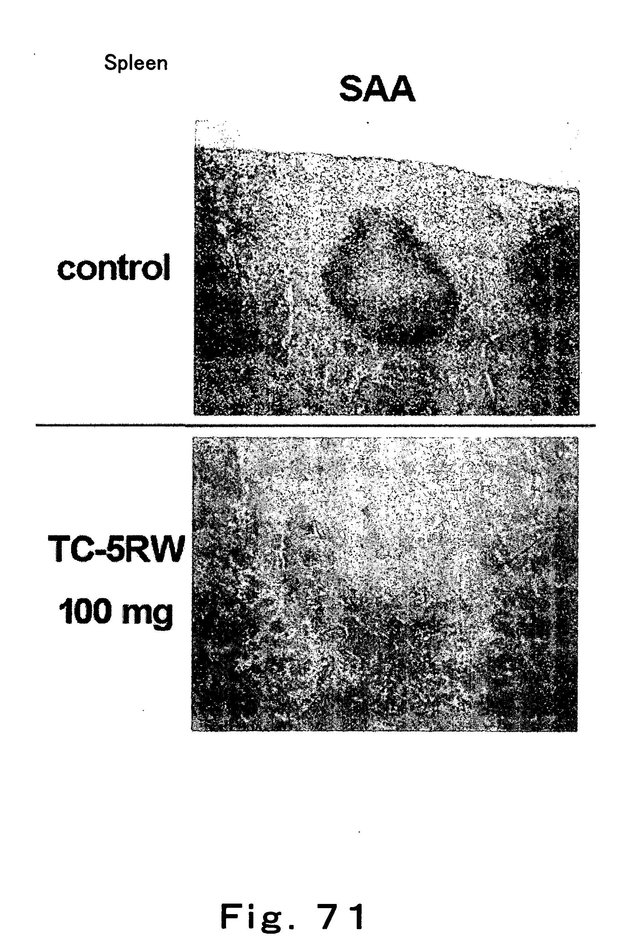

The results are shown in Table 5 and Figs. 66 to 71. In Table, "-" indicates that accumulation of amyloid or AA was not found; "±"indicates that accumulation of amyloid or AA was found just in part; and "+" indicates that accumulation of amyloid or AA was widely found.

**Table 5**

| Compound administered 1.5 months before producing amyloid | | | | |
|---|---|---|---|---|
| | Congo red staining | | SAA immunostaining | |
| | liver | Spleen | liver | Spleen |
| Control | + | ± | + | + |
| SM-4 50 mg | ± | ± | + | + |
| SM-4 75 mg | ± | ± | + | ± |
| TC-5RW 50 mg | - | - | ± | ± |
| TC-5RW 75 mg | ± | ± | ± | ± |
| HPCL 50 mg | - | - | ± | ± |
| HPCL 75 mg | - | - | - | - |

| Compound administered 4 months before producing amyloid | | | | |
|---|---|---|---|---|
| | Congo red staining | | SAA immunostaining | |
| | liver | Spleen | liver | Spleen |
| Control | + | + | + | + |
| SM-4 75 mg | ± | ± | + | + |
| SM-4 100 mg | ± | ± | + | + |
| TC-5RW 75 mg | ± | ± | + | + |
| TC-5RW 100 mg | - | - | - | - |
| HPCL 75 mg | - | - | ± | + |
| HPCL 100 mg | - | - | - | ± |

Figs. 66 to 71 show a mouse specimen to which 100 mg of HPMC (TC-5RW) was administered 4 months before producing amyloid, and a control mouse specimen to which no compound was administered. "HE" represents hematoxylin eosin staining; "Congo Red" represents Congo red staining; and "SAA" represents immnostaining with an anti-SAA antibody. Although MC (SM-4) hardly suppressed accumulation of amyloid or AA, HPMC (TC-5RW) and HPCL markedly suppressed the accumulation of amyloid or AA. This suggests that HPMC (TC-5RW) and HPCL have therapeutic and prophylactic effect for amyloidosis. Industrial Applicability

The present invention provides prophylactic and therapeutic drugs for conformational diseases which have been referred to as intractable diseases, using familiar and safe compounds, and greatly contributes for human beings to improving welfare and derating health care costs, by preventing onset of these diseases and ameliorating survival period and life quality following the onset. In addition, for animals, particularly for livestocks, their safe quality can be ensured without loosing the commercial values by preventing onset of these diseases.

## Claims

1. A pharmaceutical composition for a conformational disease comprising as an active ingredient one or more cellulose ethers selected from a cellulose ether having a structural unit represented by the general formula (1): 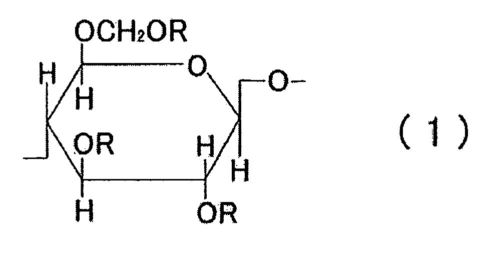 wherein, at least one R is selected from the group consisting of CH₃ , C₂H₅ , CH₂CH₂OH, CH₂CH (OH) CH₃, and CH₂COOM; while other R is H; and M is selected from the group consisting of H, Na, K and Ca,
an alkali metal salt of the cellulose ether, an aliphatic acid ester of the cellulose ether, and a mixture thereof.

2. The pharmaceutical composition for a conformational disease according to claim 1, wherein the cellulose ether is one or more selected from the group consisting of hydroxypropyl methylcellulose, hydroxypropyl cellulose and methyl cellulose.

3. The pharmaceutical composition for a conformational disease according to claim 1 or 2, wherein the pharmaceutical composition is formulated to be administrable to a mammal including human.

4. The pharmaceutical composition for a conformational disease according to any one of claims 1 to 3, wherein the pharmaceutical composition is formulated in a dosage form selected from injections, tablets, granules, powdered formulations, capsules, pills, lozenges, liquid formulations, suspensions, emulsions, syrups, spirits, elixirs, lemonade, ointments, plasters, cataplasms, tinctures, lotions, liniments, aerosols, and suppositories.

5. The pharmaceutical composition for a conformational disease according to any one of claims 1 to 4, which is a prophylactic drug for a conformational disease.

6. The pharmaceutical composition for a conformational disease according to any one of claims 1 to 4, which is a therapeutic drug for a conformational disease.

7. The pharmaceutical composition for a conformational disease according to any one of claims 1 to 6, wherein the conformational disease is a prion disease of any one of Creutzfeldt-Jakob disease (CJD) in human, Gerstmann-Straussler-Scheinker syndrome in human, fatal familial insomnia in human, bovine spongiform encephalopathy (BSE) in cattle, scrapie in sheep, and chronic wasting disease in deer.

8. The pharmaceutical composition for a conformational disease according to any one of claims 1 to 6, wherein the conformational disease is Alzheimer's disease.

9. The pharmaceutical composition for a conformational disease according to any one of claims 1 to 6, wherein the conformational disease is amyloidosis.
